# EUROPEAN PATENT SPECIFICATION

(11) **EP 1 165 136 B1**
(45) Date of publication and mention of the grant of the patent: **10.09.2003**
(21) Application number: 00912174.0
(22) Date of filing: 03.03.2000
(51) Int. Cl.: A61K 45/06, A61K 38/55, A61P 9/00

(54) **COMBINATION THERAPY OF ANGIOTENSIN CONVERTING ENZYME INHIBITOR AND EPOXY-STEROIDAL ALDOSTERONE ANTAGONIST FOR TREATMENT OF CARDIOVASCULAR DISEASE**
KOMBINATIONSTHERAPIE VON ANGIOTENSIN-KONVERTIERENDEM ENZYM HEMMER UND EPOXY-STEROIDALEM ALDOSTERON ANTAGONIST ZUR BEHANDLUNG VON KARDIOVASKULÄREN ERKRANKUNGEN
COMBINAISON THERAPEUTIQUE D'UN INHIBITEUR D'ENZYME DE CONVERSION DE L'ANGIOTENSINE ET D'UN ANTAGONISTE D'ALDOSTERONE EPOXY-STEROIDIQUE POUR LE TRAITEMENT DES MALADIES CARDIO-VASCULAIRES

(30) Priority: 05.03.1999 US 122977 P
(43) Date of publication of application: 02.01.2002
(73) Proprietor: G.D. Searle LLC, Chicago, Illinois 60680-5110 (US)
(72) Inventor: ALEXANDER, John, C., Winnetka, IL 60093 (US); RONIKER, Barbara, Chicago, IL 60610 (US); DESAI, Subhash, Wilmette, IL 60091 (US)
(74) Representative: Albrecht, Thomas, Dr.
(86) International application number: US0005633
(87) International publication number: WO00051642

(56) References cited:
- WO-A-96/40255
- WO-A-96/40257
- W.J.REMME: "Modulation of the renin-angiotensin-aldosterone system-pivotal in heart failure treatment" ZEITSCHRIFT FÜR KARDIOLOGIE, vol. 88, no. 3, 1999, pages 12-17, XP002136875
- B.PITT, B.RONIKER: "Eplerenone, a novel selective aldosterone receptor antagonist (SARA): dose finding study in patients with heart failure" JOURNAL OF THE AMERICAN COLLEGE OF CARDIOLOGY, vol. 33, no. 2/A, 1999, page 1188A XP002136876

## Description

This application claims priority of U.S. provisional application Ser. No. 60/122,997 filed March 05, 1999 and U.S. provisional application Ser. No. 60/122,998 filed March 05, 1999.

### Field of the Invention

Combinations of an angiotensin converting enzyme inhibitor and an epoxy-steroidal aldosterone receptor antagonist are described for use in treatment of circulatory disorders, including cardiovascular diseases such as heart failure, hypertension and congestive heart failure. Of particular interest are therapies using epoxy-steroidal-type aldosterone receptor antagonist compound eplerenone in combination with an angiotensin converting enzyme inhibitor.

### Background of the Invention

Myocardial (or cardiac) failure, whether a consequence of previous myocardial infarction(s), heart disease associated with hypertension, or primary cardiomyopathy, is a major health problem of worldwide proportions. The incidence of symptomatic heart failure has risen steadily over the past several decades.

In clinical terms, decompensated cardiac failure consists of a constellation of signs and symptoms that arise from congested organs and hypoperfused tissues to form the congestive heart failure (CHF) syndrome. Congestion is caused largely by increased venous pressure and by inadequate sodium (Na⁺) excretion, relative to dietary Na⁺ intake, and is importantly related to circulating levels of aldosterone (ALDO). An abnormal retention of Na⁺ occurs via tubular epithelial cells throughout the nephron, including the later portion of the distal tubule and cortical collecting ducts, where ALDO receptor sites are present.

ALDO is the body's most potent mineralocorticoid hormone. As connoted by the term mineralocorticoid, this steroid hormone has mineral-regulating activity. It promotes Na+ reabsorption not only in the kidney, but also from the lower gastrointestinal tract and salivary and sweat glands, each of which represents classic ALDO-responsive tissues. ALDO regulates Na+ and water resorption at the expense of potassium (K⁺) and magnesium (Mg²⁺) excretion.

Aldosterone plays an important role in the pathophysiology of heart failure (HF) (Dzau VJ, Colucci WS, Hollenberg NK, Williams GH. Relation of the renin-angiotensin-aldosterone system to clinical state in congestive heart failure. Circulation 1981; 63 (3):645-651). Aldosterone promotes sodium retention, magnesium and potassium loss (which contributes to arrhythmias and sudden cardiac death), sympathetic activation and parasympathetic inhibition, myocardial and vascular fibrosis, baroreceptor dysfunction, impaired arterial compliance, and vascular damage. (Wang W. Chronic administration of aldosterone depresses baroreceptor reflex function in the dog. Hypertension 1994;24(5):571-575;). Chronic elevations in circulating aldosterone levels result in fibrous tissue formation in the heart and vessels, which contributes to progressive HF. (Weber KT, et al. Collagen in the hypertrophied pressure-overloaded myocardium. Cir 1987;75:140-147). An increase in myocardial collagen production and subsequent left ventricular (LV) hypertrophy leads to myocardial stiffness, reduced ventricular and vascular compliance, impaired diastolic filling, diastolic and systolic dysfunction, ischemia, and ultimately HF. Myocardial fibrosis can also lead to arrhythmias and sudden death. Aldosterone blocks myocardial norepinephrine uptake, increases plasma norepinephrine, and promotes ventricular ectopic activity. (Struthers AD. Aldosterone escape during ACE inhibitor therapy in chronic heart failure. Eur Heart J 1995;16(Suppl N) :103-106; Struthers AD. Aldosterone escape during angiotensin-converting enzyme inhibitor therapy in chronic heart failure. J Cardiac Failure 1996; 2(1):47-54). Aldosterone affects baroreceptor function and causes cerebro- and renal vascular damage as well as endothelial dysfunction. (Struthers AD. Aldosterone escape during ACE inhibitor therapy in chronic heart failure. Eur Heart J 1995;16(Suppl N):103-106). Aldosterone also has been shown to increase plasminogen activator inhibitor levels and thereby may impede fibrinolysis.

ALDO can also provoke responses in nonepithelial cells. Elicited by a chronic elevation in plasma ALDO level that is inappropriate relative to dietary Na⁺ intake, these responses can have adverse consequences on the structure of the cardiovascular system. Hence, ALDO can contribute to the progressive nature of myocardial failure for multiple reasons.

Multiple factors regulate ALDO synthesis and metabolism, many of which are operative in the patient with myocardial failure. These include renin as well as non-renin-dependent factors (such as K⁺, ACTH) that promote ALDO synthesis. Hepatic blood flow, by regulating the clearance of circulating ALDO, helps determine its plasma concentration, an important factor in heart failure characterized by reduction in cardiac output and hepatic blood flow.

The renin-angiotensin-aldosterone system ("RAAS") is one of the hormonal mechanisms involved in regulating pressure/volume homeostasis and also in the development of hypertension, a precursor conditon implicated in the progression of more serious cardiovascular diseases such as congestive heart failure. Activation of the renin-angiotensin-aldosterone system begins with secretion of the enzyme renin from the juxtaglomerular cells in the kidney. The enzyme renin acts on a naturally-occurring substrate, angiotensinogen, to release a decapeptide, Angiotensin I. This decapeptide is cleaved by angiotensin converting enzyme ("ACE") to provide an octapeptide, Angiotensin II, the primary active species of this system. This octapeptide, angiotensin II, is a potent vasoconstrictor and also produces other physiological effects such as stimulating aldosterone secretion, promoting sodium and fluid retention, inhibiting renin secretion, increasing sympathetic nervous system activity, stimulating vasopressin secretion, causing positive cardiac inotropic effect and modulating other hormonal systems.

Emphasis has been placed on minimizing hyperaldosteronism as a basis for optimizing patient treatment. Many clinicians have assumed that the inhibition of the renin angiotensin aldosterone system (RAAS) by an angiotensin-converting enzyme inhibitor (ACE-I) will prevent aldosterone formation. However, increasing evidence suggests that ACE-I only transiently suppresses aldosterone levels. (Struthers AD.

Aldosterone escape during angiotensin-converting enzyme inhibitor therapy in chronic heart failure. J Cardiac Failure 1996; 2(1):47-54). Plasma aldosterone levels decrease initially with ACE-I treatment, but return to pretreatment levels after three to six months of ACE-I therapy, despite good compliance with continued drug administration. (Staessen J, Lijnen P, Fagard R, Verschueren LJ, Amery A. Rise in plasma concentration of aldosterone during long-term angiotensin II suppression. J Endocr 1981;91:457-465) This phenomenon is known as aldosterone "escape," since there are other important determinants of aldosterone release, such as serum potassium. (Pitt B. "Escape" of aldosterone production in patients with left ventricular dysfunction treated with an angiotensin converting enzyme inhibitor: implications for therapy. Cardiovascular Drugs and Therapy 1995;9:145-149).

Attempts to reduce ALDO-receptor antagonism both in patients treated with conventional diuretic programs and in patients treated with angiotensin-converting enzyme (ACE) inhibitors, who are often constrained to small doses of ACE inhibitor because of orthostatic hypotension. Such patients may demonstrate a recurrence of heart failure symptoms likely related to elevations in plasma ALDO levels.

Blockade of aldosterone with ACE-I has been shown to have a beneficial effect on survival and hospitalization in patients with HF. In the CONSENSUS (Cooperative North Scandinavian Enalapril Survival Study) trial, mortality at one year was reduced by 31% in patients with severe HF (NYHA Class IV) treated with enalapril (an ACE-I) plus diuretics compared to placebo plus diuretics. In CONSENSUS, patients with high baseline plasma aldosterone levels had a higher mortality than patients with low baseline levels. In the group treated with enalapril, mortality was reduced only in the group with baseline aldosterone plasma levels above the median. In the group whose baseline aldosterone plasma levels were below the median, no difference from placebo in mortality was observed. (Swedberg K, Eneroth P, Kjekshus J, Wilhelmsen L. Hormones regulating cardiovascular function in patients with severe congestive heart failure and their relation to mortality. CONSENSUS Trial Study Group. Circulation 1990; 82(5) :1730-1736).

Aldosterone receptor antagonists act directly by blocking aldosterone's site of action. Aldosterone receptor blocking agents have not been widely used in conjunction with ACE-I because of the potential for serious hyperkalemia. However, in the Randomized ALdactone Evaluation Study (RALES), the addition of spironolactone 25 mg daily, a dose which is neither diuretic nor hemodynamic, to standard therapy (ACE-I and loop diuretic, with or without digoxin) in the treatment of patients with severe HF (NYHA Class III or IV) resulted in a 30 percent risk reduction for all cause mortality compared to patients treated with standard therapy plus placebo (p<0.001). Patients treated with spironolactone also had a 35 percent lower frequency of hospitalization for worsening HF than placebo patients. (Pitt B, Zannad F, Remme WJ, Cody R, Castaigne A, Perez A, Palensky J, Wittes J for the Randomized Aldactone Evaluation Study Investigators. The effect of spironolactone on morbidity and mortality in patients with severe heart failure. N Engl J Med 1999; 341(10):709-717) It should be emphasized that these improvements in mortality and morbidity occurred when spironolactone was added to standard therapy, which included ACE-I. Patients treated with spironolactone had a median increase in serum potassium concentration that was statistically but not clinically significant compared to patients treated with placebo.

Patients who experience acute myocardial infarction (AMI) often go on to develop HF and subsequent death. Blockade of the RAAS by ACE-I has been shown to reduce all cause mortality in such patients. In the Acute Infarction Ramipril Efficacy Study (AIRE), administration of an ACE-I to patients with clinical evidence of HF at any time after an AMI resulted in a risk reduction of 27% in all cause mortality compared to placebo (p=0.002). (The Acute Infarction Ramipril Efficacy (AIRE) Study Investigators. Effect of ramipril on mortality and morbidity of survivors of acute myocardial infarction with clinical evidence of heart failure. Lancet 1993;342:821-828) Moreover, there was a 23% reduction in the risk of developing severe resistant HF in treated patients compared to placebo (p=0.017). (Cleland JGF, Erhardt L, Murray F, Hall AS, Ball SG. Effect of ramipril on morbidity and mode of death among survivors of acute myocardial infarction with clinical evidence of heart failure: a report from the AIRE Study Investigtors. Eur Heart J 1997;18:41-51) In a long-term (three year) follow-up of the AIRE patients (AIREX), Hall et al. found that patients treated with ACE-I had a risk reduction in all cause mortality of 36% compared to placebo (p=0.002), suggesting that inhibition of the RAAS after AMI has not only a significant impact on survival, but also a long term one. (Hall AS, Murray GD, Ball SG on behalf of the AIREX Study Investigators. Follow-up study of patients randomly allocated ramipril or placebo for heart failure after acute myocardial infarction: AIRE Extension (AIREX) Study. Lancet 1997;349(9064):1493-1497) In another post-MI study (Trandolapril Cardiac Evaluation study, TRACE), patients with LV dysfunction 2 to 6 days following MI treated with ACE-I had a relative reduction in the risk of death of 18% compared to placebo (p=0.001), and a reduction of 29% compared to placebo in the risk of progression to severe HF (p=0.003). (Køber L, Torp-Pedersen C, Carlsen JE, Bagger H, Eliasen P, Lyngborg K, Videbæk J, Cole DS, Auclert L, Pauly NC, Aliot E, Persson S, Camm AJ for the Trandolapril Cardiac Evaluation (TRACE) Study Group. N Engl J Med 1995;333:1670-1676; Torp-Pedersen C, Køber L, Carlsen J on behalf of the TRACE Study Group. Angiotensin-converting enzyme inhibition after myocardial infarction: the trandolapril cardiac evaluation study. Am Heart J 1996;132:235-243) In a study of ramipril versus spironolactone, Rodriguez et al (found that both drugs prevented ventricular dilatation and further decline of systolic dysfunction in patients with impaired ejection fraction post AMI. The results of these studies suggest that the addition of an aldosterone receptor antagonist to an ACE-I in patients following an AMI will further reduce mortality. (Rodriguez JA, Godoy I, Castro P, Quintana JC, Chavez E, Corbalan R. A double-blind randomized placebo controlled study of ramipril vs. spironolactone on left ventricular remodeling after acute myocardial infarction. JACC 1997;abstract 947-9:133A)

Many aldosterone receptor blocking drugs and their effects in humans are known. For example, spironolactone is a drug which acts at the mineralocorticoid receptor level by competitively inhibiting aldosterone binding. This steroidal compound has been used for blocking aldosterone-dependent sodium transport in the distal tubule of the kidney in order to reduce edema and to treat essential hypertension and primary hyperaldosteronism [F. Mantero et al, Clin. Sci. Mol. Med., 45 (Suppl 1), 219s-224s (1973)]. Spironolactone is also used commonly in the treatment of other hyperaldosterone-related diseases such as liver cirrhosis and congestive heart failure [F.J. Saunders et al, Aldactone; Spironolactone: A Comprehensive Review, Searle, New York (1978)]. Progressively-increasing doses of spironolactone from 1 mg to 400 mg per day [i.e., 1 mg/day, 5 mg/day, 20 mg/day] was administered to a spironolactone-intolerant patient to treat cirrhosis-related ascites [P.A. Greenberger et al, N. Eng. Reg. Allergy Proc., 7(4), 343-345 (Jul-Aug, 1986)]. It has been recognized that development of myocardial fibrosis is sensitive to circulating levels of both Angiotensin II and aldosterone, and that the aldosterone antagonist spironolactone prevents myocardial fibrosis in animal models, thereby linking aldosterone to excessive collagen deposition [D. Klug et al, Am. J. Cardiol., 71 (3), 46A-54A (1993)]. Spironolactone has been shown to prevent fibrosis in animal models irrespective of the development of left ventricular hypertrophy and the presence of hypertension [C.G. Brilla et al, J. Mol. Cell. Cardiol., 25 (5), 563-575 (1993)]. Spironolactone at a dosage ranging from 25 mg to 100 mg daily is used to treat diuretic-induced hypokalemia, when orally-administered potassium supplements or other potassium-sparing regimens are considered inappropriate [Physicians' Desk Reference, 46th Edn., p. 2153, Medical Economics Company Inc., Montvale, N.J. (1992)].

Previous studies have shown that inhibiting ACE inhibits the renin-angiotensin system by substantially complete blockade of the formation of Angiotensin II. Many ACE inhibitors have been used clinically to control hypertension. While ACE inhibitors may effectively control hypertension, side effects are common including chronic cough, skin rash, loss of taste sense, proteinuria and neutropenia.

Moreover, although ACE inhibitors effectively block the formation of Angiotensin II, aldosterone levels are not well controlled in certain patients having cardiovascular diseases. For example, despite continued ACE inhibition in hypertensive patients receiving captopril, there has been observed a gradual return of plasma aldosterone to baseline levels [J. Staessen et al, J. Endocrinol., 91, 457-465 (1981)]. A similar effect has been observed for patients with myocardial infarction receiving zofenopril [C. Borghi et al, J. Clin. Pharmacol., 33, 40-45 (1993)]. Patients suffering from cardiovascular diseases are also often put on low sodium diets. This regimen can induce an increase in aldosterone production and an increase in angiotensin receptors which stimulate aldosterone synthesis. Thus, a patient on a low sodium diet may induce a condition of hyperaldosteronism even in the presence of an ACE inhibitor. This phenomenon has been termed "aldosterone escape". In a side-by-side treatment of two cohorts of rats, one cohort treated with spironolactone subcutaneously and the other cohort treated with captopril, spironolactone was found to prevent fibrosis in the hypertensive-rat cohort [C.G. Brilla et al, J. Mol. Cell. Cardiol., 25, 563-575 (1993)].

Another series of steroidal-type aldosterone receptor antagonists is exemplified by epoxy-containing spironolactone derivatives. For example, U.S. Patent No. 4,559,332 issued to Grob et al describes 9a,11a-epoxy-containing spironolactone derivatives as aldosterone antagonists useful as diuretics. These 9a,11a-epoxy steroids have been evaluated for endocrine effects in comparison to spironolactone [M. de Gasparo et al, J. Pharm. Exp. Ther., 240(2), 650-656 (1987)].

Combinations of an aldosterone antagonist and an ACE inhibitor have been investigated for treatment of heart failure. It is known that mortality is higher in patients with elevated levels of plasma aldosterone and that aldosterone levels increase as CHF progresses from RAAS activation. Routine use of a diuretic may further elevate aldosterone levels. ACE inhibitors consistently inhibit angiotensin II production but exert only a mild and transient antialdosterone effect.

Combining an ACE inhibitor and spironolactone has been suggested to provide substantial inhibition of the entire RAAS. For example, a combination of enalapril and spironolactone has been administered to ambulatory patients with monitoring of blood pressure [P. Poncelet et al, Am. J. Cardiol., 65(2), 33K-35K (1990)]. In a 90-patient study, a combination of captopril and spironolactone was administered and found effective to control refractory CHF without serious incidents of hyperkalemia. [U. Dahlstrom et al, Am. J. Cardiol., 71, 29A-33A (21 Jan 1993)]. Spironolactone coadministered with an ACE inhibitor was reported to be highly effective in 13 of 16 patients afflicted with congestive heart failure [A.A. van Vliet et al, Am. J. Cardiol., 71, 21A-28A (21 Jan 1993)]. Clinical improvements have been reported for patients receiving a co-therapy of spironolactone and the ACE inhibitor enalapril, although this report mentions that controlled trials are needed to determine the lowest effective doses and to identify which patients would benefit most from combined therapy [F. Zannad, Am. J. Cardiol., 71(3), 34A-39A (1993)]. PCT Application Ser. No. US 96/01969 published 15 August 1996 describes a combination therapy of an ACE Inhibitor and a side-effect reduced amount of an aldosterone antagonist, namely, spironolactone, to treat congestive heart failure. PCT Application Ser. No. US96/01764 published 15 August 1996 describes a combination therapy of an ACE Inhibitor and a side-effect reduced amount of an aldosterone antagonist, namely, spironolactone, and a diuretic, such as a loop diuretic, to treat congestive heart failure.

Combinations of an angiotensin II receptor antagonist and aldosterone receptor antagonist are known. For example, PCT Application No. US91/09362 published 25 June 1992 describes treatment of hypertension using a combination of an imidazole-containing angiotensin II antagonist compound and a diuretic such as spironolactone. PCT Application Ser. No. US96/09342 published 19 December 1996 describes treatment of congestive heart failure with a combination of an angiotensin II antagonist and the aldosterone receptor antagonist spironolactone. PCT Application Ser. No. US96/08823 published 19 December 1996 describes treatment of myocardial fibrosis with a combination of an angiotensin II antagonist and the aldosterone receptor antagonist spironolactone. PCT Application Ser. No. US96/09335 published 19 December 1996 describes treatment of congestive heart failure with a combination of an angiotension II antagonist and the epoxy-steroidal aldosterone antagonist epoxymexrenone. PCT Application Ser. No. US96/08709 published 19 December 1996 describes treatment of cardiofibrosis with a combination of an angiotensin II antagonist and the epoxy-steroidal aldosterone receptor antagonist epoxymexrenone.

### Summary of Drawing Figures

Fig. 1-A shows X-ray powder diffraction patterns of Form H eplerenone.

Fig. 1-B shows X-ray powder diffraction patterns of Form L eplerenone.

Fig. 1-C shows X-ray powder diffraction patterns of the methyl ethyl ketone solvate of eplerenone.

Fig. 2-A shows a differential scanning calorimetry (DSC) thermogram of non-milled Form L directly crystallized from methyl ethyl ketone.

Fig. 2-B shows a differential scanning calorimetry (DSC) thermogram of non-milled Form L prepared by desolvation of a solvate obtained by crystallization of a high purity eplerenone from methyl ethyl ketone.

Fig. 2-C shows a differential scanning calorimetry (DSC) thermogram of Form L prepared by crystallizing a solvate from a solution of high purity eplerenone in methyl ethyl ketone, desolvating the solvate to yield Form L, and milling the resulting Form L.

Fig. 2-D shows a differential scanning calorimetry (DSC) thermogram of non-milled Form H prepared by desolvation of a solvate obtained by digestion of low purity eplerenone from appropriate solvents.

Fig. 3-A shows the infrared spectra (diffuse reflectance, DRIFTS) of Form H eplerenone.

Fig. 3-B shows the infrared spectra (diffuse reflectance, DRIFTS) of Form L eplerenone.

Fig. 3-C shows the infrared spectra (diffuse reflectance, DRIFTS) of the methyl ethyl ketone solvate of eplerenone.

Fig. 3-D shows the infrared spectra (diffuse reflectance, DRIFTS) of eplerenone in chloroform solution.

Fig. 4 shows ¹³C NMR spectra for Form H of eplerenone.

Fig. 5 shows ¹³C NMR spectra for Form L of eplerenone.

Fig. 6-A shows the thermogravimetry analysis profile for the methyl ethyl ketone solvate.

Fig. 7 shows an X-ray powder diffraction pattern of a crystalline form of 7-methyl hydrogen 4α,5α:9α,11α-diepoxy-17-hydroxy-3-oxo-17α-pregnane-7α,21-dicarboxylate, γ-lactone isolated from methyl ethyl ketone.

Fig. 8 shows an X-ray powder diffraction pattern of the crystalline form of 7-methyl hydrogen 11α,12α-epoxy-17-hydroxy-3-oxo-17α-pregn-4-ene-7α,21-dicarboxylate, γ-lactone isolated from isopropanol.

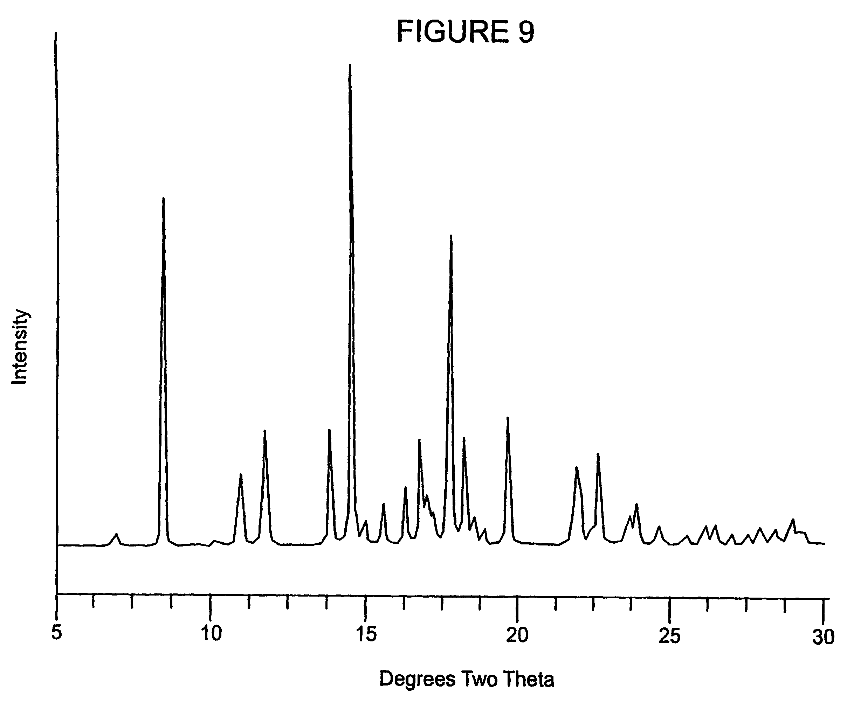

Fig. 9 shows an X-ray powder diffraction pattern of the crystalline form of 7-methyl hydrogen 17-hydroxy-3-oxo-17a-pregna-4,9(11)-diene-7a,21-dicarboxylate, γ-lactone isolated from n-butanol.

Fig. 10 shows the X-ray powder diffraction patterns for the wet cake (methyl ethyl ketone solvate) obtained from (a) 0%, (b) 1%, (c) 3%, and (d) 5% diepoxide-doped methyl ethyl ketone crystallizations.

Fig. 11 shows the X-ray powder diffraction patterns for the dried solids obtained from (a) 0%, (b) 1%, (c) 3%, and (d) 5% diepoxide-doped methyl ethyl ketone crystallizations.

Fig. 12 shows the X-ray powder diffraction patterns for the dried solids from the methyl ethyl ketone crystallization with 3% doping of diepoxide (a) without grinding of the solvate prior to drying, and (b) with grinding of the solvate prior to drying.

Fig. 13 shows the X-ray powder diffraction patterns for the wet cake (methyl ethyl ketone solvate) obtained from (a) 0%, (b) 1%, (c) 5%, and (d) 10% 11,12-epoxide-doped methyl ethyl ketone crystallizations.

Fig. 14 shows the X-ray powder diffraction patterns for the dried solids obtained from (a) 0%, (b) 1%, (c) 5%, and (d) 10% 11,12-epoxide-doped methyl ethyl ketone crystallizations.

Fig. 15 shows a cube plot of product purity, starting material purity, cooling rate and endpoint temperature based on the data reported in Table 7A.

Fig. 16 shows a half normal plot prepared using the cube plot of Fig. 15 to determine those variables having a statistically significant effect on the purity of the final material.

Fig. 17 is an interaction graph based on the results reported in Table 7A showing the interaction between starting material purity and cooling rate on final material purity.

Fig. 18 shows a cube plot of Form H weight fraction, starting material purity, cooling rate and endpoint temperature based on the data reported in Table 7A.

Fig. 19 shows a half normal plot prepared using the cube plot of Fig. 18 to determine those variables having a statistically significant effect on the purity of the final material.

Fig. 20 is an interaction graph based on the results reported in Table 7A showing the interaction between starting material purity and endpoint temperature on final material purity.

Fig. 21 shows an X-ray diffraction pattern of amorphous eplerenone.

Fig. 22 shows a DSC thermogram of amorphous eplerenone.

Fig. 23 summarizes a clinical trial plan.

Fig. 24 shows baseline parameters for the different treatment groups.

Fig. 25 shows concurrent medications for the patients in a study involving combination therapy.

Fig. 26 shows changes in diastolic blood pressure for eplerenone (50 mg/day) and spironolactone (25 mg/day) treated groups, as compared to placebo.

Fig. 27 shows urinary aldosterone levels at different rates of eplerenone (25 mg QD, 25 mg B1D, 50 mg QD, 100 mg QD) and spironolactone (25 mg QD) administration, as compared to placebo.

Fig. 28 shows plasma renin activity and aldosterone excretion for groups treated with eplerenone (50 mg/day) and spironolactone (25 mg/day), as compared to placebo.

Fig. 29 shows changes in BNP levels for all patients at different rates of eplerenone and spironolactone administration, as compared to placebo.

Fig. 30 shows changes in BNP levels for patients with high baseline BNP at different rates of eplerenone and spironolactone administration, as compared to placebo.

Fig. 31 shows the study schematic for Biological Evaluation II: multicenter, randomized, double-blind, placebo-controlled, two-arm, parallel group trial.

### Description of the Invention

Treatment of circulatory disorders, including cardiovascular disorders such as heart failure, cirrhosis, hypertension and congestive heart failure is provided by a combination therapy comprising a therapeutically-effective amount of an angiotensin converting enzyme ("ACE") inhibitor along with a therapeutically-effective amount of epoxy-steroidal-type aldosterone receptor antagonist and optionally, a non-aldosterone-receptor-antagonist-type diuretic or digoxin. Preferably, the diuretic is a loop diuretic.

The phrase "angiotensin converting enzyme inhibitor" ("ACE inhibitor") is intended to embrace an agent or compound, or a combination of two or more agents or compounds, having the ability to block, partially or completely, the rapid enzymatic conversion of the physiologically inactive decapeptide form of angiotensin ("Angiotensin I") to the vasoconstrictive octapeptide form of angiotensin ("Angiotensin II"). Blocking the formation of Angiotensin II can quickly affect the regulation of fluid and electrolyte balance, blood pressure and blood volume, by removing the primary actions of Angiotensin II. Included in these primary actions of Angiotensin II are stimulation of the synthesis and secretion of aldosterone by the adrenal cortex and raising blood pressure by direct constriction of the smooth muscle of the arterioles.

The phrase "aldosterone receptor antagonist" embraces an.agent or compound, or a combination of two or more of such agents or compounds, which agent or compound binds to the aldosterone receptor as a competitive inhibitor of the action of aldosterone itself at the receptor site in the renal tubules, so as to modulate the receptor-mediated activity of aldosterone. Typical of such aldosterone receptor antagonists are epoxy-steroidal-type compounds.

The phrase "epoxy-steroidal aldosterone receptor antagonist" is intended to embrace one or more agents or compounds characterized by a steroid-type nucleus and having an epoxy moiety attached to the nucleus and which agent or compound binds to the aldosterone receptor, as a competitive inhibitor of the action of aldosterone itself at the receptor site, so as to modulate the receptor-mediated activity of aldosterone.

The phrase "combination therapy" (or "co-therapy"), in defining use of an ACE inhibitor agent and an aldosterone receptor antagonist agent, and optionally a non-aldosterone-receptor-antagonist-type diuretic or digoxin, is intended to embrace administration of each agent in a sequential manner in a regimen that will provide beneficial effects of the drug combination, and is intended as well to embrace co-administration of these agents in a substantially simultaneous manner, such as in a single capsule having a fixed ratio of these active agents or in multiple, separate capsules for each agent.

The phrase "therapeutically-effective" is intended to qualify the amount of each agent for use in the combination therapy which will achieve the goal of improvement in cardiac sufficiency by reducing or preventing, for example, the progression of congestive heart failure, while avoiding adverse side effects typically associated with each agent.

The phrase "side-effect reducing amount", in characterizing a therapeutically-effective amount of the epoxy-steroidal aldosterone receptor antagonist agent in the combination therapy, is intended to define a quantity of such agent, or a range of quantity of such agent, that is capable of improving cardiac sufficiency while reducing or avoiding one or more aldosterone-antagonist-induced side effects, such as hyperkalemia. A dosage of epoxy-steroidal aldosterone receptor antagonist agent which would accomplish the therapeutic goal of favorably enhancing cardiac sufficiency, while reducing or avoiding side effects, would be a dosage that substantially avoids inducing diuresis, that is, a substantially non-diuresis-effect dosage.

The phrase "non-diuresis-effective" amount, especially as to the amount of epoxy-steroidal aldosterone receptor antagonist agent, is intended to define a quantity of such agent, or a range of quantity of such agent, that does not cause a substantial increase in diuretic effect, i.e., an increase in sodium and water excretion.

A preferred combination therapy would consist essentially of two or three active agents, namely, an ACE inhibitor agent, an epoxy-steroidal aldosterone receptor antagonist agent, and optionally, a non-aldosterone-receptor-antagonist-type diuretic or digoxin. For the ACE inhibitor and an ALDO antagonist combination the agents would be used in combination in a weight ratio range from about 0.5-to-one to about twenty-to-one of the angiotensin converting enzyme agent to the aldosterone receptor antagonist agent. A preferred range of these two agents (ACE inhibitor-to-ALDO antagonist) would be from about one-to-one to about fifteen-to-one, while a more preferred range would be from about one-to-one to about five-to-one, depending ultimately on the selection of the ACE inhibitor and ALDO antagonist. The optional diuretic agent may be present in a range from about 1 mg to about 200 mg, prefeably from 5 mg to 150 mg, depending upon the diuretic selected and the disease state targetted.

Examples of ACE inhibitors which may be used in the combination therapy are shown in the following four categories.

A first group of ACE inhibitors consists of the following compounds: AB-103, ancovenin, benazeprilat, BRL-36378, BW-A575C, CGS-13928C, CL-242817, CV-5975, Equaten, EU-4865, EU-4867, EU-5476, foroxymithine, FPL 66564, FR-900456, Hoe-065, I5B2, indolapril, ketomethylureas, KRI-1177, KRI-1230, L-681176, libenzapril, MCD, MDL-27088, MDL-27467A, moveltipril, MS-41, nicotianamine, pentopril, phenacein, pivopril, rentiapril, RG-5975, RG-6134, RG-6207, RGH-0399, ROO-911, RS-10085-197, RS-2039, RS 5139, RS 86127, RU-44403, S-8308, SA-291, spiraprilat, SQ-26900, SQ-28084, SQ-28370, SQ-28940, SQ-31440, Synecor, utibapril, WF-10129, Wy-44221, Wy-44655, Y-23785, Yissum P-0154, zabicipril and zofenopril.

A second group of ACE inhibitors of interest consists of the following compounds: Asahi Brewery AB-47, alatriopril, BMS 182657, Asahi Chemical C-111, Asahi Chemical C-112, Dainippon DU-1777, mixanpril, Prentyl, zofenoprilat and 1-(-(1-carboxy-6-(4-piperidinyl)hexyl) amino)-1-oxopropyl octahydro-1H-indole-2-carboxylic acid.

A third group of ACE inhibitors of greater interest consists of the following compounds: Bioproject BP1.137, Chiesi CHF 1514, Fisons FPL-66564, idrapril, Marion Merrell Dow MDL-100240, perindoprilat and Servier S-5590.

A fourth group of ACE inhibitors of highest interest consists of the following compounds: alacepril, benazepril, captopril, cilazapril, delapril, enalapril, enalaprilat, fosinopril, fosinoprilat, imidapril, lisinopril, perindopril, quinapril, ramipril, saralasin acetate, temocapril, trandolapril, ceranapril, moexipril, quinaprilat and spirapril.

Many of these ACE inhibitors are commercially available, expecially those listed in the fourth group, above. For example, a highy preferred ACE inhibitor, captopril, is sold by E.R. Squibb & Sons, Inc., Princeton, N.J., under the trademark "CAPOTEN", in tablet dosage form at doses of 12.5 mg, 50 mg and 100 mg per tablet. Enalapril, or Enalapril Maleate, and Lisinopril are two more highly preferred ACE inhibitors sold by Merck & Co, West Point, Pa. Enalapril is sold under the trademark "VASOTEC" in tablet dosage form at doses of 2.5 mg, 5 mg, 10 mg and 20 mg per tablet. Lisinopril is sold under the trademark "PRINIVIL" in tablet dosage form at doses of 5 mg, 10 mg, 20 mg and 40 mg per tablet.

The non-aldosterone-receptor-antagonist-type diuretic agent which is used in the combination of ACE inhibitor and aldosterone receptor antagonist may be selected from several known classes, such as thiazides and related sulfonamides, potassium-sparing diuretics, loop diuretics and organic mercurial diuretics.

Examples of thiazides are bendroflumethiazide, benzthiazide, chlorothiazide, cyclothiazide, hydrochlorothiazide, hydroflumethiazide, methyclothiazide, polythiazide and trichlormethiazide.

Examples of sulfonamides related to thiazides are chlorthalidone, quinethazone and metolazone.

Examples of potassium-sparing diuretics are triameterene and amiloride.

Examples of loop diuretics, i.e., diuretics acting in the ascending limb of the loop of Henle of the kidney, are furosemide and ethynacrylic acid.

Examples of organic mercurial diuretics are mercaptomerin sodium, merethoxylline, procaine and mersalyl with theophylline

Epoxy-steroidal aldosterone receptor antagonist compounds suitable for use in the combination therapy consist of these compounds having a steroidal nucleus substituted with an epoxy-type moiety. The term "epoxy-type" moiety is intended to embrace any moiety characterized in having an oxygen atom as a bridge between two carbon atoms, examples of which include the following moieties: 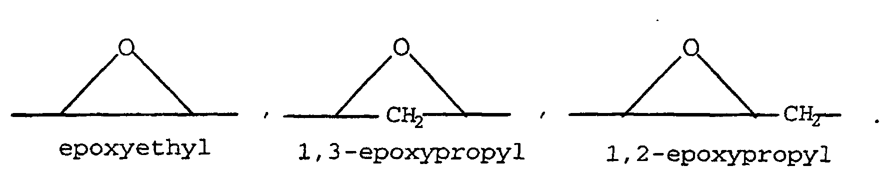 The term "steroidal", as used in the phrase "epoxy-steroidal", denotes a nucleus provided by a cyclopentenophenanthrene moiety, having the conventional "A", "B", "C" and "D" rings. The epoxy-type moiety may be attached to the cyclopentenophenanthrene nucleus at any attachable or substitutable positions, that is, fused to one of the rings of the steroidal nucleus or the moiety may be substituted on a ring member of the ring system. The phrase "epoxy-steroidal" is intended to embrace a steroidal nucleus having one or a plurality of epoxy-type moieties attached thereto.

Epoxy-steroidal aldosterone receptor antagonists suitable for use in combination therapy include a family of compounds having an epoxy moiety fused to the "C" ring of the steroidal nucleus. Especially preferred are 20-spiroxane compounds characterized by the presence of a 9α,11α-substituted epoxy moiety. ^Table 1, below, describes a series of 9α,11α-epoxy-steroidal compounds which may be used in the combination therapy. Especially preferred of these compounds of Table 1 is Compound #1 which is known by the common name epoxymexrenone and also by the USAN designation eplerenone. These epoxy steroids may be prepared by procedures described in U.S. Patent No. 4,559,332 to Grob et al issued 17 December 1985.

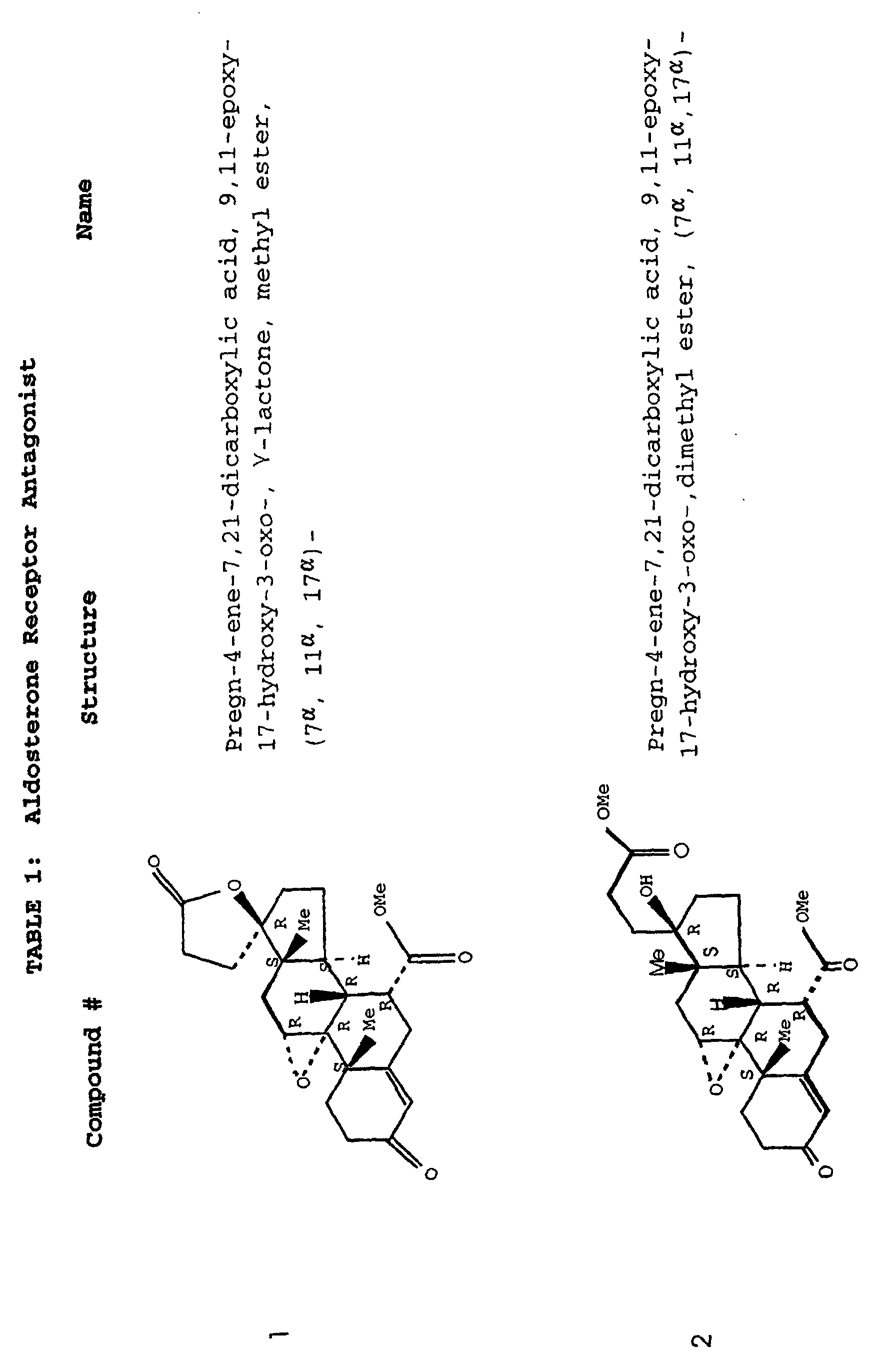

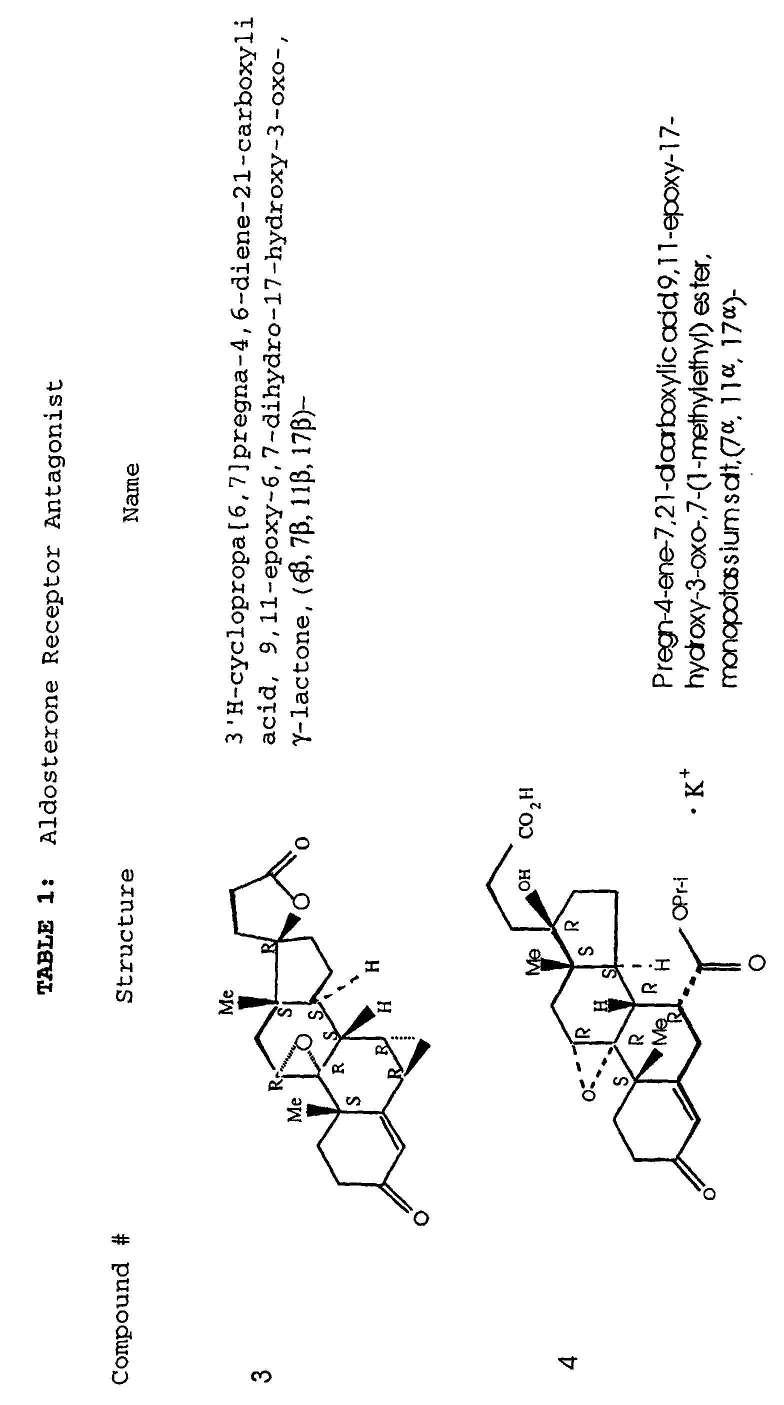

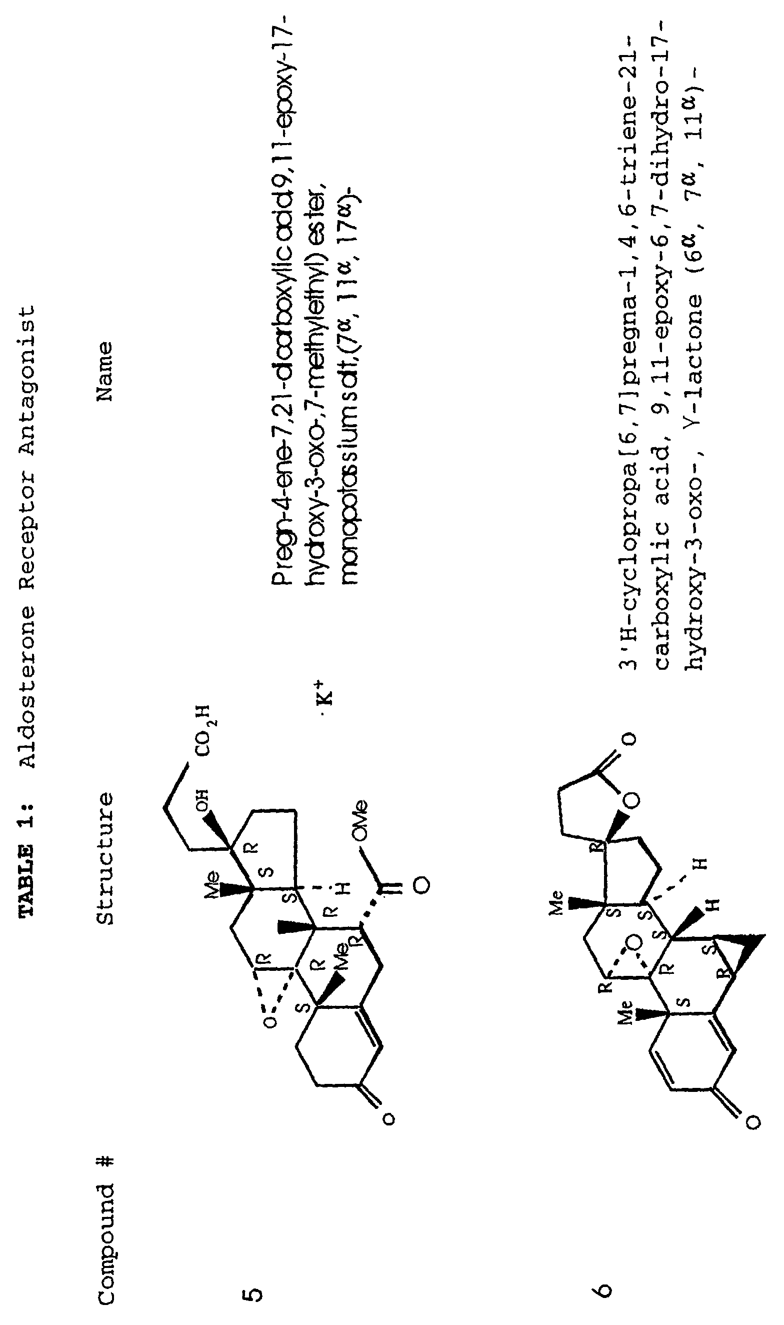

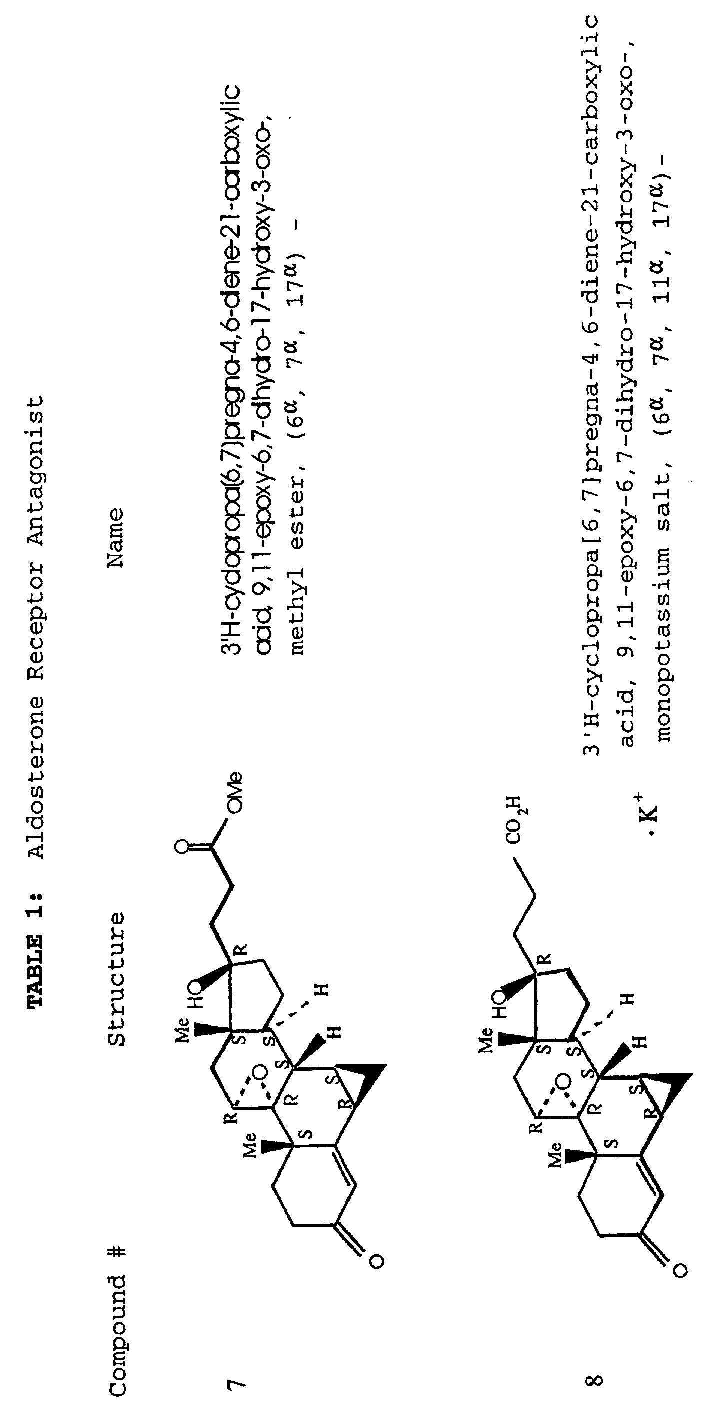

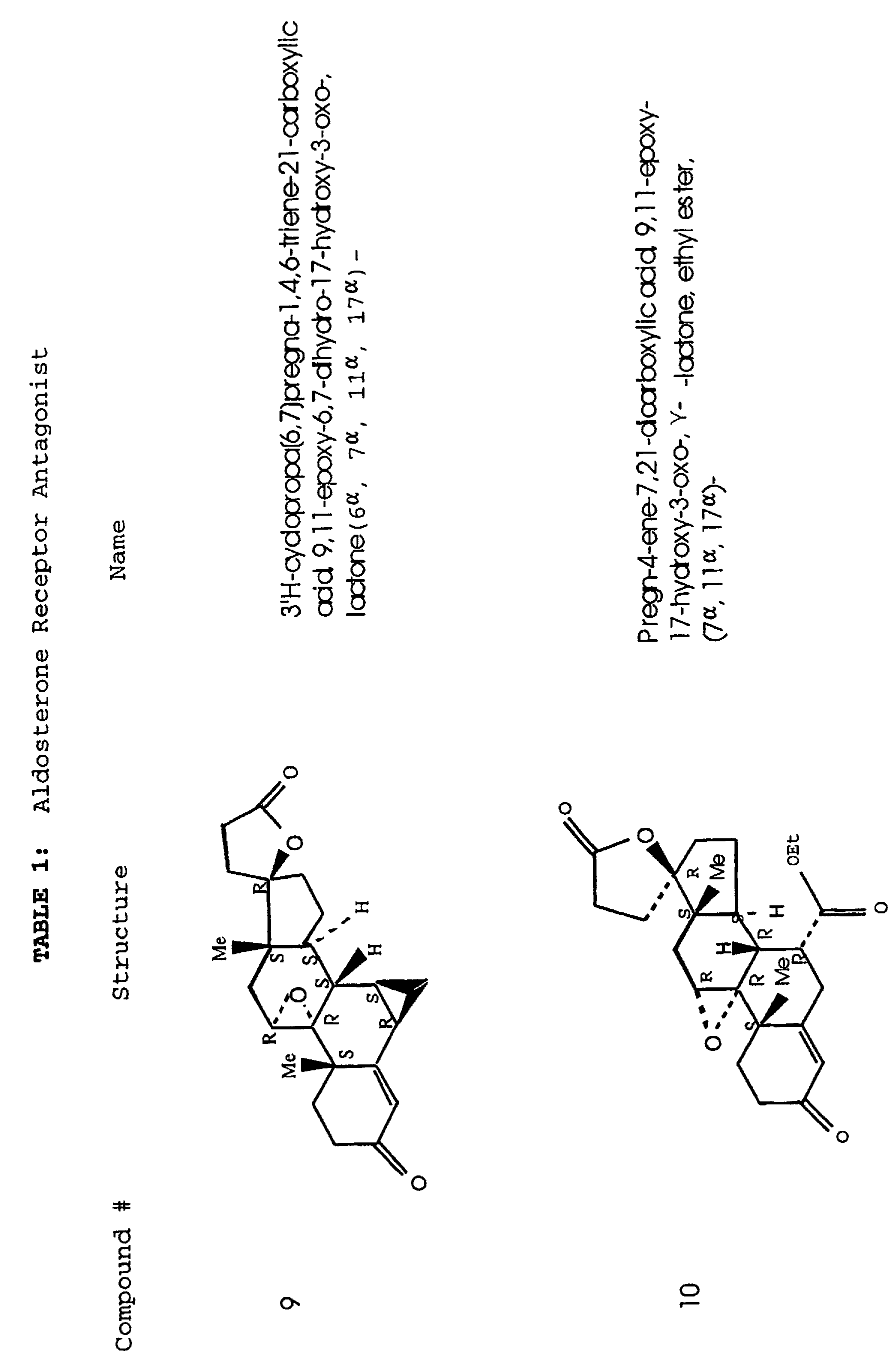

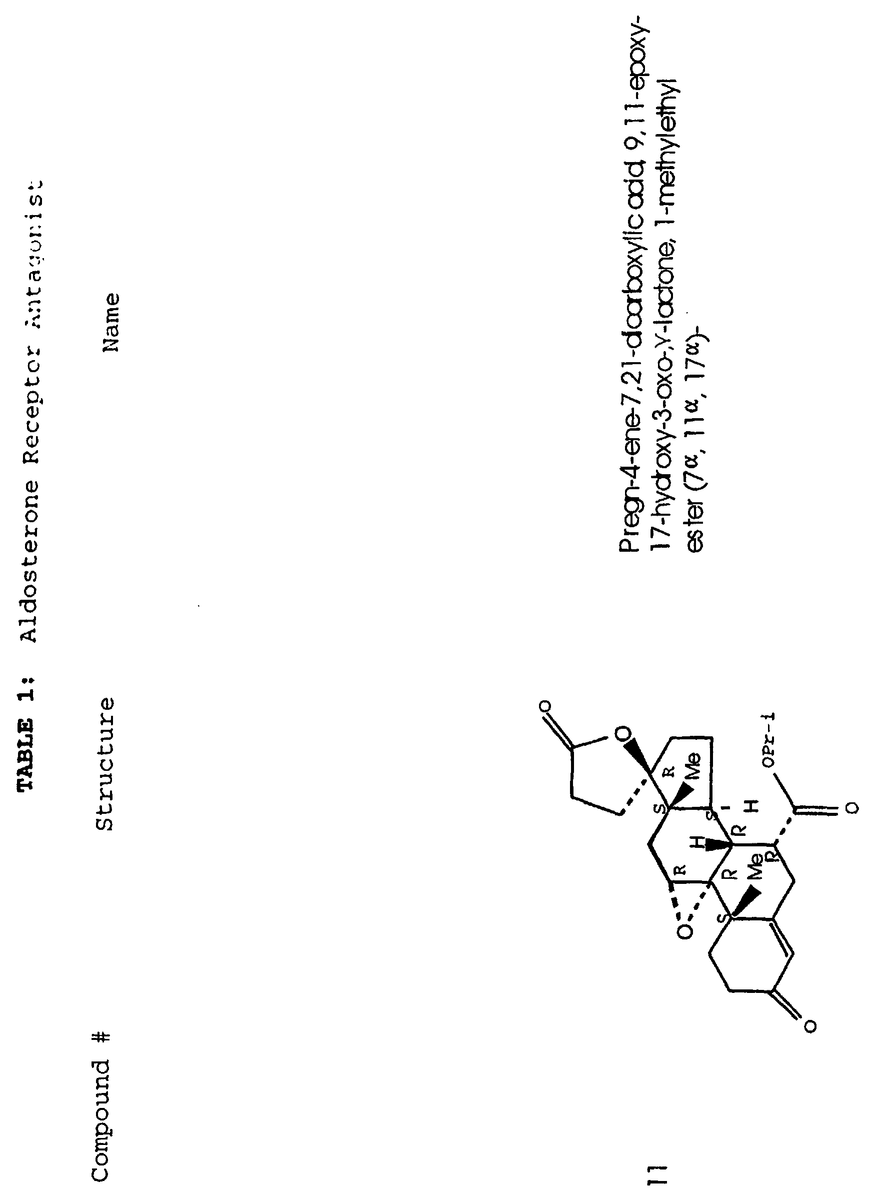

Administration of the ACE inhibitor, the aldosterone receptor antagonist, and optionally, a non-aldosterone-receptor-antagonist-type diuretic or digoxin may take place sequentially in separate formulations, or may be accomplished by simultaneous administration in a single formulation or separate formulations. Administration may be accomplished by oral route, or by intravenous, intramuscular or subcutaneous injections. The formulation may be in the form of a bolus, or in the form of aqueous or non-aqueous isotonic sterile injection solutions or suspensions. These solution and suspensions may be prepared from sterile powders or granules having one or more pharmaceutically-acceptable carriers or diluents, or a binder such as gelatin or hydroxypropylmethyl cellulose, together with one or more of a lubricant, preservative, surface-active or dispersing agent.

For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units are tablets or capsules. The ACE inhibitor may be present in an amount from about 1 to 200 mg, preferably from about 2 to 150 mg, depending upon the specific ACE inhibitor selected. A suitable daily dose for a mammal may vary widely depending on the condition of the patient and other factors. The ALDO antagonist may be present in an amount of from about 1 to 400 mg, preferably from about 2 to 150 mg, depending upon the specific ALDO antagonist compound selected and the specific disease state being targetted for the combination therapy.

For disease states which require prevention, reduction or treatment of a cardiovascular disease state without incidence of hyperkalemia, for example, the ALDO antagonist component, typically eplerenone, will be present in the combination therapy in an amount in a range from about 5 mg to about 200 mg per dose. A preferred range for eplerenone would be from about 25 mg to 50 mg per dose. More preferably would be a range from about 10 mg to 15 mg per dose per day.

The active ingredients may also be administered by injection as a composition wherein, for example, saline, dextrose or water may be used as a suitable carrier.

The dosage regimen for treating a disease condition with the combination therapy of this invention is selected in accordance with a variety of factors, including the type, age, weight, sex and medical condition of the patient, the severity of the disease, the route of administration, and the particular compound employed, and thus may vary widely.

For therapeutic purposes, the active components of this combination therapy invention are ordinarily combined with one or more adjuvants appropriate to the indicated route of administration. If administered per os, the components may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Formulations for parenteral administration may be in the form of aqueous or non-aqueous isotonic sterile injection solutions or suspensions. These solutions and suspensions may be prepared from sterile powders or granules having one or more of the carriers or diluents mentioned for use in the formulations for oral administration. The components may be dissolved in water, polyethylene glycol, propylene glycol, ethanol, corn oil, cottonseed oil, peanut oil, sesame oil, benzyl alcohol, sodium chloride, and/or various buffers. Other adjuvants and modes of administration are well and widely known in the pharmaceutical art.

Pharmaceutical compositions for use in the treatment methods of the invention may be administered in oral form or by intravenous adminstration. Oral administration of the combination therapy is preferred. Dosing for oral administration may be with a regimen calling for single daily dose, or for a single dose every other day, or for multiple, spaced doses throughout the day. The active agents which make up the combination therapy may be administered simultaneously, either in a combined dosage form or in separate dosage forms intended for substantially simultaneous oral administration. The active agents which make up the combinatin therapy may also be administered sequentially, with either active component being administered by a regimen calling for multi-step ingestion. Thus, a regimen may call for sequential administration of the active agents with spaced-apart ingestion of the separate, active agents. The time period between the multiple ingestion steps may range from a few minutes to several hours, depending upon the properties of each active agent such a potency, solubility, bioavailability, plasma half-life and kinetic profile of the agent, as well as depending upon the age and condition of the patient. The active agents of the combined therapy whether administered simultaneously, substantially simultaneously, or sequentially, may involve a regimen calling for administration of one active agent by oral route and the other active agent by intravenous route. Whether the active agents of the combined therapy are adminstered by oral or intravenous route, separately or together, each such active agent will be contained in a suitable pharmaceutical formulation of pharmaceutically-acceptable excipients, diluents or other formulations components. Examples of suitable pharmaceutically-acceptable formulations containing the active components for oral administration are given below. Even though such formulations list both active agents together in the same recipe, it is appropriate for such recipe to be utilized for a formulation containing one of the active components.

### Example 1

An oral dosage may be prepared by screening and then mixing together the following list of ingredients in the amounts indicated. The dosage may then be placed in a hard gelatin capsule.

| Ingredients | Amounts |
|---|---|
| captoril | 62.0 mg |
| eplerenone | 12.5 mg |
| magnesium stearate | 10 mg |
| lactose | 100 mg |

### Example 1A

An oral dosage may be prepared by screening and then mixing together the following list of ingredients in the amounts indicated. The dosage may then be placed in a hard gelatin capsule.

| Ingredients | Amounts |
|---|---|
| captoril | 62.0 mg |
| eplerenone | 12.5 mg |
| furosemide | 73.9 mg |
| magnesium stearate | 10 mg |
| lactose | 100 mg |

### Example 2

An oral dosage may be prepared by mixing together granulating with a 10% gelatin solution. The wet granules are screened, dried, mixed with starch, talc and stearic acid, screened and compressed into a tablet.

| Ingredients | Amounts |
|---|---|
| captoril | 62.0 mg |
| eplerenone | 12.5 mg |
| calcium sulfate dihydrate | 100 mg |
| sucrose | 15 mg |
| starch | 8 mg |
| talc | 4 mg |
| stearic acid | 2 mg |

### Example 2A

An oral dosage may be prepared by mixing together granulating with a 10% gelatin solution. The wet granules are screened, dried, mixed with starch, talc and stearic acid, screened and compressed into a tablet.

| Ingredients | Amounts |
|---|---|
| captoril | 62.0 mg |
| eplerenone | 12.5 mg |
| furosemide | 73.9 mg |
| calcium sulfate dihydrate | 100 mg |
| sucrose | 15 mg |
| starch | 8 mg |
| talc | 4 mg |
| stearic acid | 2 mg |

### Example 3

An oral dosage may be prepared by screening and then mixing together the following list of ingredients in the amounts indicated. The dosage may then be placed in a hard gelatin capsule.

| Ingredients | Amounts |
|---|---|
| enalapril | 14.3 mg |
| eplerenone | 12.5 mg |
| magnesium stearate | 10 mg |
| lactose | 100 mg |

### Example 3A

An oral dosage may be prepared by screening and then mixing together the following list of ingredients in the amounts indicated. The dosage may then be placed in a hard gelatin capsule.

| Ingredients | Amounts |
|---|---|
| enalapril | 14.3 mg |
| eplerenone | 12.5 mg |
| furosemide | 73.9 mg |
| magnesium stearate | 10 mg |
| lactose | 100 mg |

### Example 4

An oral dosage may be prepared by mixing together granulating with a 10% gelatin solution. The wet granules are screened, dried, mixed with starch, talc and stearic acid, screened and compressed into a tablet.

| Ingredients | Amounts |
|---|---|
| enalapril | 14.3 mg |
| eplerenone | 12.5 mg |
| calcium sulfate dihydrate | 100 mg |
| sucrose | 15 mg |
| starch | 8 mg |
| talc | 4 mg |
| stearic acid | 2 mg |

### Example 4A

An oral dosage may be prepared by mixing together granulating with a 10% gelatin solution. The wet granules are screened, dried, mixed with starch, talc and stearic acid, screened and compressed into a tablet.

| Ingredients | Amounts |
|---|---|
| enalapril | 14.3 mg |
| eplerenone | 12.5 mg |
| furosemide | 73.9 mg |
| calcium sulfate dihydrate | 100 mg |
| sucrose | 15 mg |
| starch | 8 mg |
| talc | 4 mg |
| stearic acid | 2 mg |

### Treatment of Specific Conditions and Disorders

The pharmaceutical compositions of the present invention are useful where administration of an aldosterone receptor blocker is indicated. It has been found that these compositions are particularly effective in the treatment of cardiovascular diseases such as heart failure; hypertension (especially the management of mild to moderate hypertension); edema associated with liver insufficiency; post-myocardial infarction; cirrhosis of the liver; stroke prevention; and reduction of heart rate for subjects exhibiting an accelerated heart rate.

For the treatment of heart failure, the pharmaceutical composition preferably provides a daily dosage of eplerenone in the amount of about 25 mg to about 200 mg, more preferably about 25 mg to about 75 mg, and still more preferably about 50 mg. A daily dose of about 0.33 to 2.67 mg/kg body weight (based upon an average body weight of about 75 kg), preferably between about 0.33 and about 1.00 mg/kg body weight and most preferably 0.67 mg/kg body weight, may be appropriate. The daily dose can be administered in one to four doses per day, preferably one dose per day.

For the treatment of hypertension, the pharmaceutical composition preferably provides a daily dosage of eplerenone in the amount of about 50 mg to about 300 mg, more preferably about 50 mg to about 150 mg, and still more preferably about 100 mg. A daily dose of about 0.67 to 4.00 mg/kg body weight, preferably between about 0.67 and about 2.00 mg/kg body weight and most preferably about 1.33 mg/kg body weight, may be appropriate. The daily dose can be administered in one to four doses per day, preferably one dose per day.

For the treatment of edema associated with liver insufficiency, the pharmaceutical composition preferably provides a daily dosage of eplerenone in the amount of about 50 mg to about 500 mg, more preferably about 100 mg to 400 about mg, and still more preferably about 300 mg. A daily dose of about 0.67 to 6.67 mg/kg body weight, preferably between about 1.33 and about 5.33 mg/kg body weight and most preferably about 4.00 mg/kg body weight, may be appropriate. The daily dose can be administered in one to four doses per day, preferably one dose per day.

It has been found that the pharmaceutical compositions of the present invention provide a therapeutic effect as aldosterone receptor blockers in humans over an interval of about 12 to 24 hours, preferably about 24 hours, after oral administration.

In general, the pharmaceutical compositions of the present invention provide a daily dosage of eplerenone sufficient to cause an increase in blood serum renin and aldosterone concentrations in humans over an interval of about 12 to 24 hours, preferably about 24 hours, after oral administration. Specifically, these compositions provide a daily dosage of eplerenone sufficient to cause an average increase in blood serum renin concentration over an interval of about 12 to 24 hours, preferably about 24 hours, after ingestion of the composition of at least about 10%. Similarly, these compositions provide a daily dosage of eplerenone sufficient to cause an average increase in blood serum aldosterone concentrations over an interval of about 12 to 24 hours, preferably about 24 hours, after ingestion of the composition of at least about 50%.

It also has been found that the pharmaceutical compositions of the present invention provide a daily dosage of eplerenone sufficient to cause an average increase in the urinary log₁₀ (sodium/potassium) ratio in humans over an interval of about 12 to 24 hours, preferably about 24 hours, after ingestion of the composition.

It also has been found that the pharmaceutical compositions of the present invention provide a daily dosage of eplerenone sufficient to cause an average decrease in diasystolic blood pressure in humans over an interval of about 12 to 24 hours, preferably about 24 hours, after ingestion of the composition of at least about 5%.

### Unit Dosages

Dosage unit forms of the pharmaceutical compositions may typically contain, for example, 10, 20, 25, 37.5, 50, 75, 100, 125, 150, 175, 200, 250, 300, 350 or 400 mg of eplerenone. Preferred dosage unit forms contain about 25, 50, 100, or 150 mg of eplerenone. The dosage unit form may be selected to accommodate the desired frequency of administration used to achieve the specified daily dosage. The amount of the unit dosage form of the pharmaceutical composition that is administered and the dosage regimen for treating the condition or disorder will depend on a variety of factors, including the age, weight, sex and medical condition of the subject, the severity of the condition or disorder, the route and frequency of administration, and thus may vary widely.

It has been discovered, however, that the efficacy of the required daily dosage of the pharmaceutical compositions of the present invention does not appear to materially differ for once-a-day administration relative to twice-a-day administration with respect to the compositions described in this application. It is hypothesized that the compositions of the present invention deliver an amount of eplerenone sufficient to inhibit a protracted genomic response caused by aldosterone binding to the aldosterone receptor site. Interruption of aldosterone binding by eplerenone prevents aldosterone-induced gene product synthesis resulting in an extended period of functional aldosterone receptor blockade that does not require a sustained plasma eplerenone concentration. Accordingly, once-a-day administration is preferred for such tablets for convenience of administration.

### Form of Pharmaceutical Compositions

The pharmaceutical compositions of the present invention comprise eplerenone in association with one or more non-toxic, pharmaceutically-acceptable carriers, excipients and/or adjuvants (collectively referred to herein as "carrier materials"). The carrier materials must be acceptable in the sense of being compatible with the other ingredients of the composition and must not be deleterious to the recipient. The pharmaceutical compositions of the present invention may be adapted for administration by any suitable route by selection of appropriate carrier materials and a dosage of eplerenone effective for the treatment intended. For example, these compositions may be prepared in a form suitable for administration orally, intravascularly, intraperitoneally, subcutaneously, intramuscularly (IM) or rectally.

Accordingly, the carrier material employed can be a solid or a liquid, or both, and is preferably formulated with the compound as a unit-dose composition, for example, a tablet, which can contain from about 1% to about 95%, preferably about 10% to about 75%, more preferably about 20% to about 60%, and still more preferably about 20% to about 40%, by weight of eplerenone. Such pharmaceutical compositions of the invention can be prepared by any of the well known techniques of pharmacy, consisting essentially of admixing the components.

### Sold State Forms Of Epoxy-Steroidal Aldosterone Receptor Antagonists

The uses of the claimed composition of the present invention encompass the administration of a therapeutically-effective amount of epoxy-steroidal aldosterone receptor antagonists, particularly eplerenone, in any of its solid state forms, either as one or more solid state forms per se or in the form of a pharmaceutical composition comprising one or more solid state forms of eplerenone. These novel solid state forms include, but are not limited to, solvated crystalline eplerenone, non-solvated crystalline eplerenone, and amorphous eplerenone, and mixtures thereof in any useful ratio.

In one use, the eplerenone administered is a non-solvated crystalline form of eplerenone having the X-ray powder diffraction pattern set forth in Table 1A below (referred to herein as the "higher melting point polymorph" or "Form H").

In another use of the invention, the eplerenone administered is a non-solvated crystalline form of eplerenone having the X-ray powder diffraction pattern set forth in Table 1B below (referred to herein as the "lower melting point polymorph" or "Form L").

Unformulated Form H exhibits a faster dissolution rate (approximately 30% higher) at lower temperatures (i.e., temperatures below the enantiotropic transition temperature as later discussed) than, for example, unformulated Form L. Where dissolution of eplerenone in the gastrointestinal tract is the rate-controlling step for delivery of the eplerenone to the target cells, faster dissolution generally results in improved bioavailability. Form H, therefore, can provide an improved bioavailability profile relative to Form L. In addition, selection of a solid state form of eplerenone having a faster dissolution rate likewise provides greater flexibility in the selection of excipients for, and in the formulation of, immediate release pharmaceutical compositions relative to other solid state forms having a slower dissolution rate.

Form L possesses greater physical stability at lower temperatures (i.e., at temperatures below the enantiotropic transition temperature as later discussed) than, for example, Form H. Solid state forms of eplerenone such as Form L that do not require the use of special processing or storage conditions, and that avoid the need for frequent inventory replacement, are desirable. For example, selection of a solid state form of eplerenone that is physically stable during the manufacturing process (such as during milling of eplerenone to obtain a material with reduced particle size and increased surface area) can avoid the need for special processing conditions and the increased costs generally associated with such special processing conditions. Similarly, selection of a solid state form of eplerenone that is physically stable at different conditions of storage (especially considering the different possible storage conditions during the lifetime of the eplerenone product) can help avoid polymorphic or other degradative changes in the eplerenone that can lead to product loss or deterioration of product efficacy. Therefore, the selection of a solid state form of eplerenone such as Form L having greater physical stability provides a meaningful benefit over less stable eplerenone forms.

In another use of the invention, the eplerenone administered is a solvated crystalline form of eplerenone. Preferably, the solvated crystalline forms are substantially exclusive of solvents that are not pharmaceutically-acceptable solvents. Because Form H and Form L typically are more physically stable than the crystalline solvates at room temperature and under atmospheric pressure, the solvated crystalline forms used in such compositions generally comprise a pharmaceutically acceptable higher boiling and/or hydrogen bonding solvent such as, but not limited to, butanol. It is believed that the solvated crystalline forms collectively can offer a range of different dissolution rates and, where dissolution of eplerenone in the gastrointestinal tract is the rate-controlling step for delivery of the eplerenone to the target cells, bioavailabilities relative to Form H and Form L.

In another use of the invention, the eplerenone administered is amorphous eplerenone. It is hypothesized that amorphous eplerenone possesses a different dissolution rate and, where dissolution of eplerenone in the gastrointestinal tract is the rate-controlling step for delivery of the eplerenone to the target cells, bioavailability relative to Form H and Form L.

In another use, the eplerenone administered is a combination comprising a first solid state form of eplerenone and a second solid state form of eplerenone. Generally, the first and second solid state forms of eplerenone are selected from Form H, Form L, solvated eplerenone and amorphous eplerenone. In general, the weight ratio of said first solid state form to said second solid state form preferably is at least about 1:9, more preferably at least about 1:1, still more preferably at least about 2:1, still more preferably at least about 5:1, and still more preferably at least about 9:1.

In another use, the eplerenone is to be administered in the form of a pharmaceutical composition wherein the entire amount of eplerenone contained in the composition is present as phase pure Form H.

In another use, the eplerenone is to be administered in the form of a pharmaceutical composition wherein the entire amount of eplerenone contained in the composition is present as phase pure Form L.

In another use, the eplerenone is to be administered in the form of a pharmaceutical composition wherein the entire amount of eplerenone contained in the composition is present as a phase pure solvated crystalline eplerenone.

In another use, the eplerenone is to be administered in the form of a pharmaceutical composition wherein the entire amount of eplerenone contained in the composition is present as amorphous eplerenone.

In another use, the eplerenone is to be administered in the form of a pharmaceutical composition wherein the composition comprises a first solid state form of eplerenone and a second solid state form of eplerenone, and the first and second solid state forms of eplerenone are selected from Form H, Form L, solvated eplerenone and amorphous eplerenone. In general, the weight ratio of said first solid state form to said second solid state form preferably is at least about 1:9, preferably about 1:1, more preferably at least about 2:1, more preferably at least about 5:1, and still more preferably at least about 9:1.

In another use, the eplerenone is to be administered in the form of a pharmaceutical composition wherein the composition comprises both Form H and Form L. The ratio of the amount of Form L to Form H in the composition generally is between about 1:20 to about 20:1. In other embodiments, for example, this ratio is between about 10:1 to about 1:10; about 5:1 to about 1:5; about 2:1 to about 1:2; or about 1:1.

Although each of the above uses can embrace the administration of a solid state form of eplerenone over a broad range of eplerenone particle sizes, it has been discovered that coupling the selection of the solid state form of eplerenone with a reduction of the eplerenone particle size can improve the bioavailability of unformulated eplerenone and pharmaceutical compositions comprising that solid state form of eplerenone.

In one such embodiment, the D₉₀ particle size of the unformulated eplerenone or the eplerenone used as a starting material in the pharmaceutical composition generally is less than about 400 microns, preferably less than about 200 microns, more preferably less than about 150 microns, still more preferably less than about 100 microns, and still more preferably less than about 90 microns. In another embodiment, the D₉₀ particle size is between about 40 microns to about 100 microns. In another embodiment, the D₉₀ particle size is between about 30 microns to about 50 microns. In another embodiment, the D₉₀ particle size is between about 50 microns to about 150 microns. In another embodiment, the D₉₀ particle size is between about 75 microns to about 125 microns.

In another such embodiment, the D₉₀ particle size of the unformulated eplerenone or the eplerenone used as a starting material in the pharmaceutical composition generally is less than about 15 microns, preferably less than about 1 micron, more preferably less than about 800 nm, still more preferably less than about 600 nm, and still more preferably less than about 400 nm. In another embodiment, the D₉₀ particle size is between about 10 nm to about 1 micron. In another embodiment, the D₉₀ particle size is between about 100 nm to about 800 nm. In another embodiment, the D₉₀ particle size is between about 200 nm to about 600 nm. In another embodiment, the D₉₀ particle size is between about 400 nm to about 800 nm.

Solid state forms of eplerenone having a particle size less than about 15 microns can be prepared in accordance with applicable particle size reduction techniques known in the art. Such techniques include, but are not limited to those described in U.S. Patents 5,145,684, 5,318,767, 5,384,124 and 5,747,001. U.S. Patents 5,145,684, 5,318,767, 5,384,124 and 5,747,001 are expressly incorporated by reference as if fully set forth at length. In accordance with the method of U.S. Patent 5,145,684, for example, particles of suitable size are prepared by dispersing the eplerenone in a liquid dispersion medium and wet-grinding the mixture in the presence of grinding media to reduce the particles to the desired size. If necessary or advantageous, the particles can be reduced in size in the presence of a surface modifier.

### Definitions

The term "amorphous" as applied to eplerenone refers to a solid state wherein the eplerenone molecules are present in a disordered arrangement and do not form a distinguishable crystal lattice or unit cell. When subjected to X-ray powder diffraction, amorphous eplerenone does not produce any characteristic crystalline peaks.

Where reference is made in this application to the "boiling point" of a substance or solution, the term "boiling point" means the boiling point of the substance or solution under the applicable process conditions.

The term "crystalline form" as applied to eplerenone refers to a solid state form wherein the eplerenone molecules are arranged to form a distinguishable crystal lattice (i) comprising distinguishable unit cells, and (ii) yielding diffraction peaks when subjected to X-ray radiation.

The term "crystallization" as used throughout this application can refer to crystallization and/or recrystallization depending upon the applicable circumstances relating to the preparation of the eplerenone starting material.

The term "digestion" means a process in which a slurry of solid eplerenone in a solvent or mixture of solvents is heated at the boiling point of the solvent or mixture of solvents under the applicable process conditions.

The term "direct crystallization" as used herein refers to the crystallization of eplerenone directly from a suitable solvent without the formation and desolvation of an intermediate solvated crystalline solid state form of eplerenone.

The term "particle size" as used herein refers to particle size as measured by conventional particle size measuring techniques well known in the art, such as laser light scattering, sedimentation field flow fractionation, photon correlation spectroscopy, or disk centrifugation.

The term "D₉₀ particle size" means the particle size of at least 90% of the particles as measured by such conventional particle size measuring techniques.

The term "purity" means the chemical purity of eplerenone according to conventional HPLC assay. As used herein, "low purity eplerenone" generally means eplerenone that contains an effective amount of a Form H growth promoter and/or a Form L growth inhibitor. As used herein, "high purity eplerenone" generally means eplerenone that does not contain, or contains less than an effective amount of, a Form H growth promoter and/or a Form L growth inhibitor.

The term "phase purity" means the solid state purity of eplerenone with regard to a particular crystalline or amorphous form of the eplerenone as determined by the infrared spectroscopy analytical methods described herein.

The term "XPRD" means X-ray powder diffraction.

The term "Tₘ" means melting temperature.

### Characterization of Solid State Form

### 1. Molecular Conformation

Single crystal X-ray analysis indicates that the eplerenone molecular conformation differs between Form H and Form L, particularly with respect to the orientation of the ester group at the 7-position of the steroid ring. The orientation of the ester group can be defined by the C8-C7-C23-02 torsion angle.

In the Form H crystal lattice, the eplerenone molecule adopts a conformation in which the methoxy group of the ester is approximately aligned with the C-H bond at the 7-position and the carbonyl group is approximately positioned over the center of the B-steroid ring. The C8-C7-C23-02 torsion angle is approximately -73.0° in this conformation. In this orientation, the carbonyl oxygen atom of the ester group (01) is in close contact with the oxygen atom of the 9,11-epoxide ring (04). The 01-04 distance is about 2.97 Å, which is just below the van der Waal's contact distance of 3.0 Å (assuming van der Waal's radii of 1.5Å for the oxygen).

In the Form L crystal lattice, the eplerenone molecule adopts a conformation in which the ester group is rotated approximately 150° relative to that of Form H and has a C8-C7-C23-02 torsion angle of approximately +76.9°. In this orientation, the methoxy group of the ester is directed toward the 4,5-alkene segment of the A-steroid ring. In this orientation, the distance between either oxygen atom of the ester group (01,02) and the oxygen atom of the 9,11-epoxide ring is increased relative to the distance determined for Form H. The 02-04 distance is approximately 3.04Å, falling just above the van der Waal's contact distance. The 01-04 distance is about 3.45Å.

The eplerenone molecule appears to adopt a conformation characteristic of Form L in the solvated crystalline forms analyzed by single crystal X-ray diffraction to date.

### 2. X-Ray Powder Diffraction

The various crystalline forms of eplerenone were analyzed with either a Siemens D5000 powder diffractometer or an Inel Multipurpose Diffractometer. For the Siemens D500 powder diffractometer, the raw data was measured for 2q values from 2 to 50, with steps of 0.020 and step periods of two seconds. For the Inel Multipurpose Diffractometer, samples were placed in an aluminum sample holder and raw data was collected for 30 minutes at all two theta values simultaneously.

Tables 1A, 1B and 1C set out the significant parameters of the main peaks in terms of 2q values and intensities for the Form H (prepared by desolvation of the ethanol solvate obtained by digestion of low purity eplerenone), Form L (prepared by desolvation of the methyl ethyl ketone solvate obtained by recrystallization of high purity eplerenone), and methyl ethyl ketone solvate (prepared by room temperature slurry conversion of high purity eplerenone in methyl ethyl ketone) crystalline forms of eplerenone, respectively (X-ray radiation at a wavelength of 1.54056 Angstroms).

Minor shifts in peak positioning may be present in the diffraction patterns of Form H and Form L as a result of imperfections in the spacing of the crystal diffraction planes due to the route of manufacture of Form H and Form L (i.e. desolvation of a solvate). In addition, Form H is isolated from a solvate prepared by digestion of crude eplerenone. This method results in a lower overall chemical purity (approximately 90%) of the Form H. Finally, the solvated forms of eplerenone are expected to show some shifting in the positioning of the diffraction peaks due to the increased mobility of the solvent molecules within the solvent channels in the crystal lattice.

**TABLE 1A:**

| FORM H DATA | | | |
|---|---|---|---|
| Angle 2-theta | d-spacing Angstro m | Intensity | Intensity % |
| 6.994 | 12.628 | 1188 | 7.2 |
| 8.291 | 10.655 | 2137 | 13 |
| 10.012 | 8.827 | 577 | 3.5 |
| 11.264 | 7.849 | 1854 | 11.3 |
| 12.04 | 7.344 | 7707 | 46.8 |
| 14.115 | 6.269 | 3121 | 19 |
| 14.438 | 6.13 | 15935 | 96.8 |
| 15.524 | 5.703 | 637 | 3.9 |
| 16.169 | 5.477 | 1349 | 8.2 |
| 16.699 | 5.305 | 1663 | 10.1 |
| 16.94 | 5.23 | 1692 | 10.3 |
| 17.147 | 5.167 | 2139 | 13 |
| 17.66 | 5.018 | 6883 | 41.8 |
| 17.91 | 4.949 | 16455 | 100 |
| 18.379 | 4.823 | 3106 | 18.9 |
| 18.658 | 4.752 | 1216 | 7.4 |
| 19.799 | 4.48 | 1499 | 9.1 |
| 20.235 | 4.385 | 383 | 2.3 |
| 21.707 | 4.091 | 1267 | 7.7 |
| 21.8 | 4.073 | 1260 | 7.7 |
| 21.959 | 4.044 | 1279 | 7.8 |
| 22.461 | 3.955 | 4264 | 25.9 |
| 23.191 | 3.832 | 1026 | 6.2 |
| 23.879 | 3.723 | 1000 | 6.1 |
| 24.599 | 3.616 | 1688 | 10.3 |
| 25.837 | 3.445 | 931 | 5.7 |
| 26.034 | 3.42 | 686 | 4.2 |
| 26.868 | 3.316 | 912 | 5.5 |
| 27.093 | 3.288 | 1322 | 8 |
| 27.782 | 3.209 | 1236 | 7.5 |
| 28.34 | 3.147 | 1845 | 11.2 |
| 28.861 | 3.091 | 957 | 5.8 |
| 29.866 | 2.9892 | 745 | 4.5 |
| 30.627 | 2.9166 | 992 | 6 |
| 31.108 | 2.8726 | 1205 | 7.3 |
| 33.215 | 2.6951 | 1287 | 7.8 |
| 33.718 | 2.656 | 802 | 4.9 |
| 34.434 | 2.6024 | 914 | 5.6 |

**TABLE 1B:**

| FORM L DATA | | | |
|---|---|---|---|
| Angle 2-Theta | d-spacing Angstrom | Intensit y Cps | Intensity % |
| 7.992 | 11.054 | 11596 | 26.6 |
| 10.044 | 8.799 | 12048 | 27.6 |
| 11.206 | 7.889 | 4929 | 11.3 |
| 12.441 | 7.109 | 1747 | 4 |
| 12.752 | 6.936 | 4340 | 9.9 |
| 13.257 | 6.673 | 2444 | 5.6 |
| 14.705 | 6.019 | 43646 | 100 |
| 15.46 | 5.727 | 2670 | 6.1 |
| 15.727 | 5.63 | 7982 | 18.3 |
| 16.016 | 5.529 | 3519 | 8.1 |
| 17.671 | 5.015 | 8897 | 20.4 |
| 17.9 | 4.951 | 2873 | 6.6 |
| 18.352 | 4.83 | 612 | 1.4 |
| 18.703 | 4.74 | 689 | 1.6 |
| 19.524 | 4.543 | 1126 | 2.6 |
| 20.103 | 4.413 | 3753 | 8.6 |
| 20.63 | 4.302 | 1451 | 3.3 |
| 21.067 | 4.214 | 876 | 2 |
| 21.675 | 4.097 | 2760 | 6.3 |
| 22.232 | 3.995 | 1951 | 4.5 |
| 22.652 | 3.922 | 1657 | 3.8 |
| 23.624 | 3.763 | 827 | 1.9 |
| 24.279 | 3.663 | 1242 | 2.8 |
| 25.021 | 3.556 | 5144 | 11.8 |
| 25.485 | 3.492 | 1702 | 3.9 |
| 25.707 | 3.463 | 2493 | 5.7 |
| 26.251 | 3.392 | 1371 | 3.1 |
| 26.85 | 3.318 | 1970 | 4.5 |
| 27.319 | 3.262 | 1029 | 2.4 |
| 27.931 | 3.192 | 440 | 1 |
| 27.969 | 3.187 | 440 | 1 |
| 28.937 | 3.083 | 1128 | 2.6 |
| 29.703 | 3.005 | 1211 | 2.8 |
| 30.173 | 2.9594 | 1506 | 3.5 |
| 30.584 | 2.9206 | 1602 | 3.7 |
| 30.885 | 2.8928 | 1550 | 3.6 |
| 31.217 | 2.8628 | 1068 | 2.4 |
| 31.605 | 2.8285 | 1038 | 2.4 |
| 32.059 | 2.7895 | 1211 | 2.8 |
| 32.64 | 2.7412 | 684 | 1.6 |
| 32.747 | 2.7324 | 758 | 1.7 |
| 33.46 | 2.6759 | 506 | 1.2 |
| 34.194 | 2.6201 | 1085 | 2.5 |
| 34.545 | 2.5943 | 915 | 2.1 |

**TABLE 1C:**

| METHYL ETHYL KETONE DATA | | | |
|---|---|---|---|
| Angle 2-Theta | d-spacing Angstro m | Intensity Cps | Intensit y % |
| 7.584 | 11.648 | 5629 | 32.6 |
| 7.753 | 11.393 | 15929 | 92.3 |
| 10.151 | 8.707 | 2877 | 16.7 |
| 11.31 | 7.817 | 701 | 4.1 |
| 12.646 | 6.994 | 1027 | 5.9 |
| 13.193 | 6.705 | 15188 | 88 |
| 13.556 | 6.526 | 14225 | 82.4 |
| 14.074 | 6.287 | 1966 | 11.4 |
| 14.746 | 6.002 | 2759 | 16 |
| 15.165 | 5.837 | 801 | 4.6 |
| 15.548 | 5.694 | 1896 | 11 |
| 17.031 | 5.202 | 7980 | 46.2 |
| 17.28 | 5.127 | 17267 | 100 |
| 17.706 | 5.005 | 6873 | 39.8 |
| 18.555 | 4.778 | 545 | 3.2 |
| 18.871 | 4.699 | 1112 | 6.4 |
| 19.766 | 4.488 | 1704 | 9.9 |
| 20.158 | 4.401 | 1396 | 8.1 |
| 20.725 | 4.282 | 2644 | 15.3 |
| 21.787 | 4.076 | 1127 | 6.5 |
| 22.06 | 4.026 | 451 | 2.6 |
| 22.864 | 3.886 | 1542 | 8.9 |
| 23.412 | 3.796 | 14185 | 82.2 |
| 23.75 | 3.743 | 1154 | 6.7 |
| 24.288 | 3.662 | 3063 | 17.7 |
| 25.253 | 3.524 | 1318 | 7.6 |
| 25.503 | 3.49 | 1736 | 10.1 |
| 25.761 | 3.455 | 1225 | 7.1 |
| 26.176 | 3.402 | 1346 | 7.8 |
| 26.548 | 3.355 | 1098 | 6.4 |
| 27.357 | 3.257 | 1944 | 11.3 |
| 27.605 | 3.229 | 2116 | 12.3 |
| 27.9 | 3.195 | 858 | 5 |
| 28.378 | 3.142 | 583 | 3.4 |
| 28.749 | 3.103 | 763 | 4.4 |
| 29.3 | 3.046 | 1182 | 6.8 |
| 29.679 | 3.008 | 2606 | 15.1 |
| 30.402 | 2.9377 | 2184 | 12.6 |
| 30.739 | 2.9063 | 648 | 3.8 |

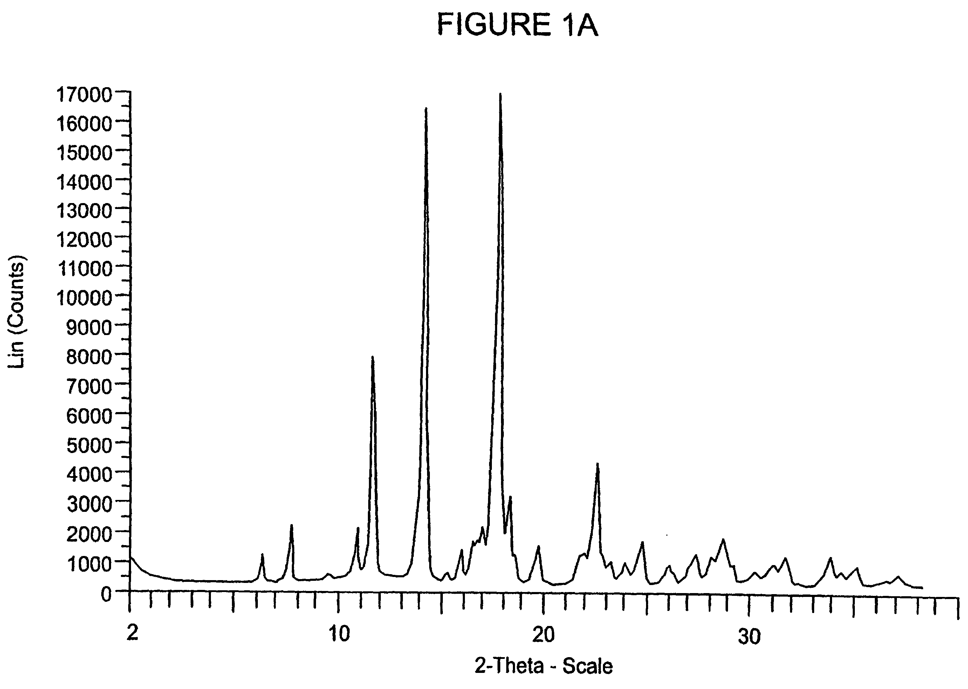

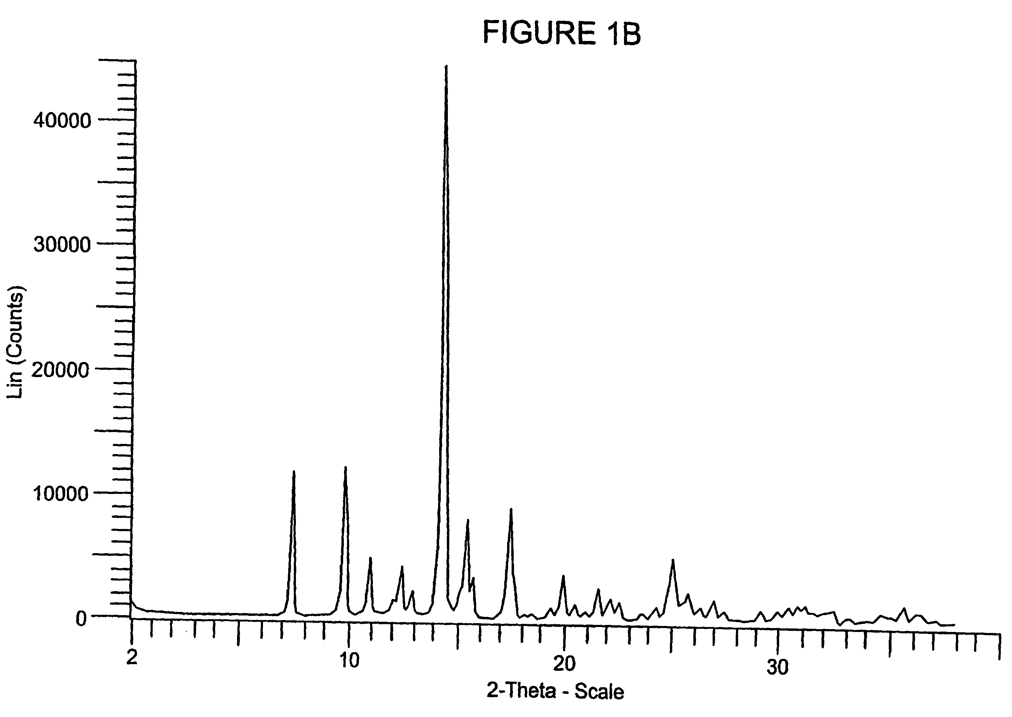

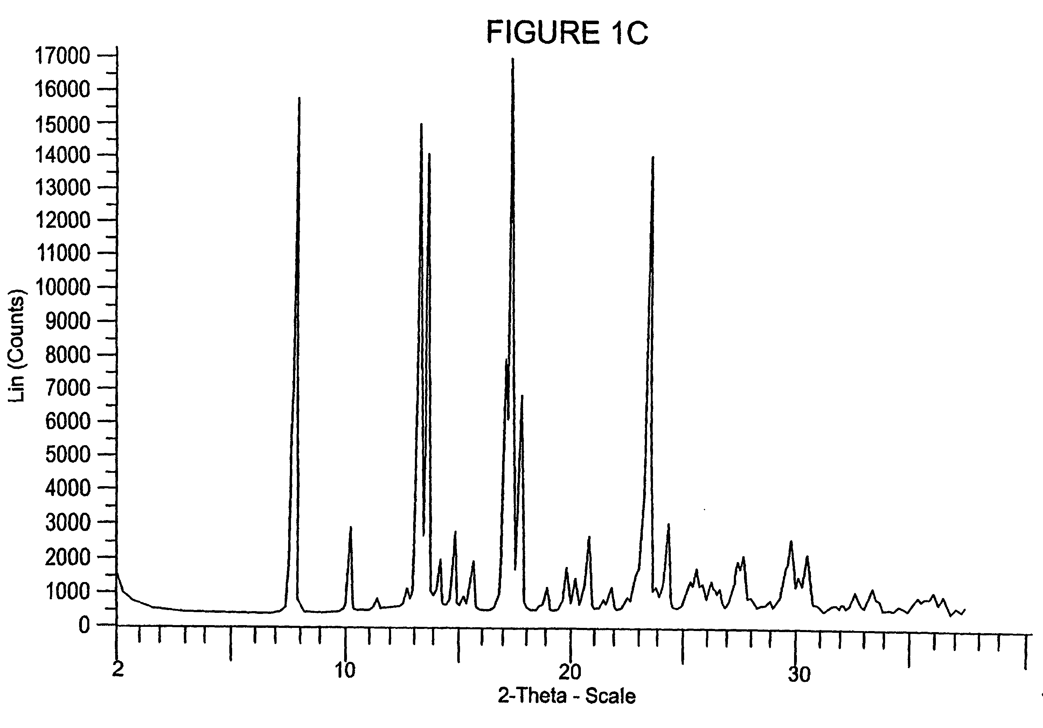

Graphical examples of the x-ray diffraction patterns for Form H, Form L, and the methyl ethyl ketone solvate crystalline forms of eplerenone are shown in Figs. 1-A, 1-B, and 1-C, respectively. Form H shows distinguishing peaks at 7.0 ± 0.2, 8.3 ± 0.2, and 12.0 ± 0.2 degrees two theta. Form L shows distinguishing peaks at 8.0 ± 0.2, 12.4 ± 0.2, 12.8 ± 0.2, and 13.3 ± 0.2 degrees two theta. The methyl ethyl ketone solvated crystalline form shows distinguishing peaks at 7.6 ± 0.2, 7.8 ± 0.2, and 13.6 ± 0.2 degrees two theta.

### 3. Melting/Decomposition Temperature

The temperatures of melting and/or decomposition of non-solvated eplerenone crystalline forms were determined using a TA Instruments 2920 differential scanning calorimeter. Each sample (1-2 mg) was placed in either a sealed or unsealed aluminum pan and heated at 10°C/minute. Melting/decomposition ranges were defined from the extrapolated onset to the maximum of the melting/decomposition endotherm.

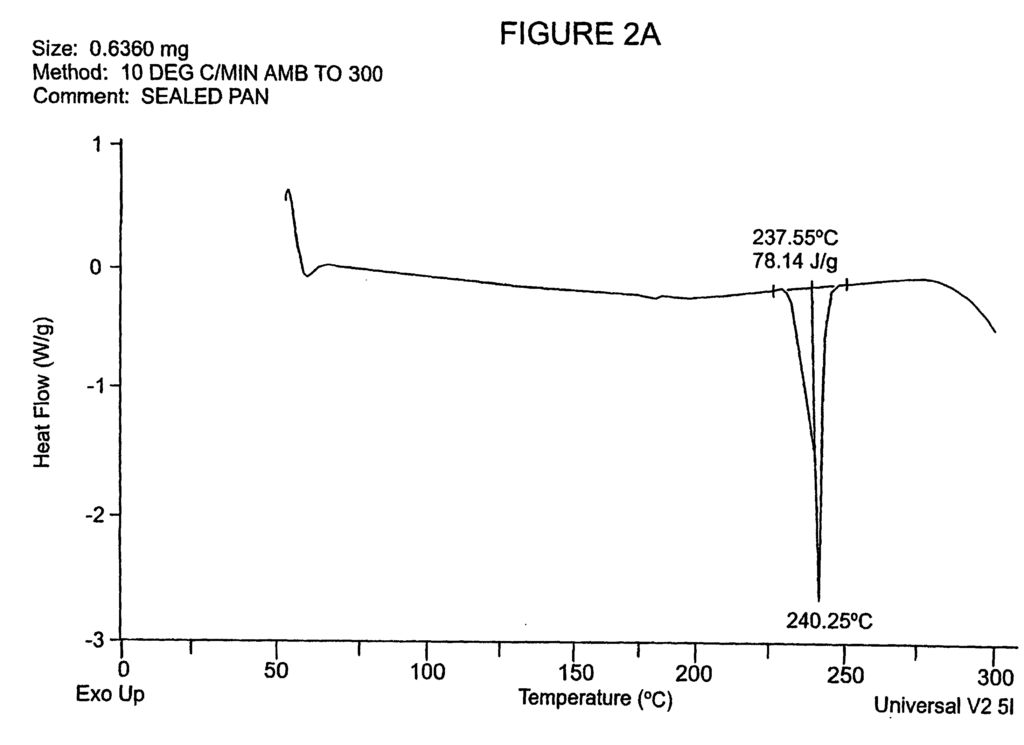

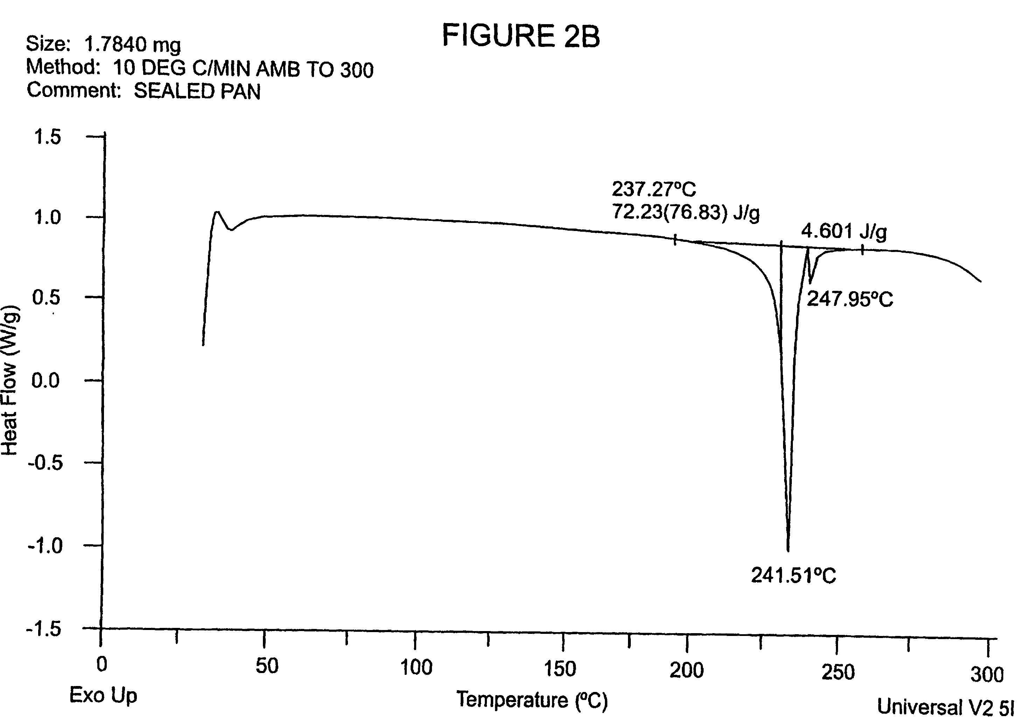

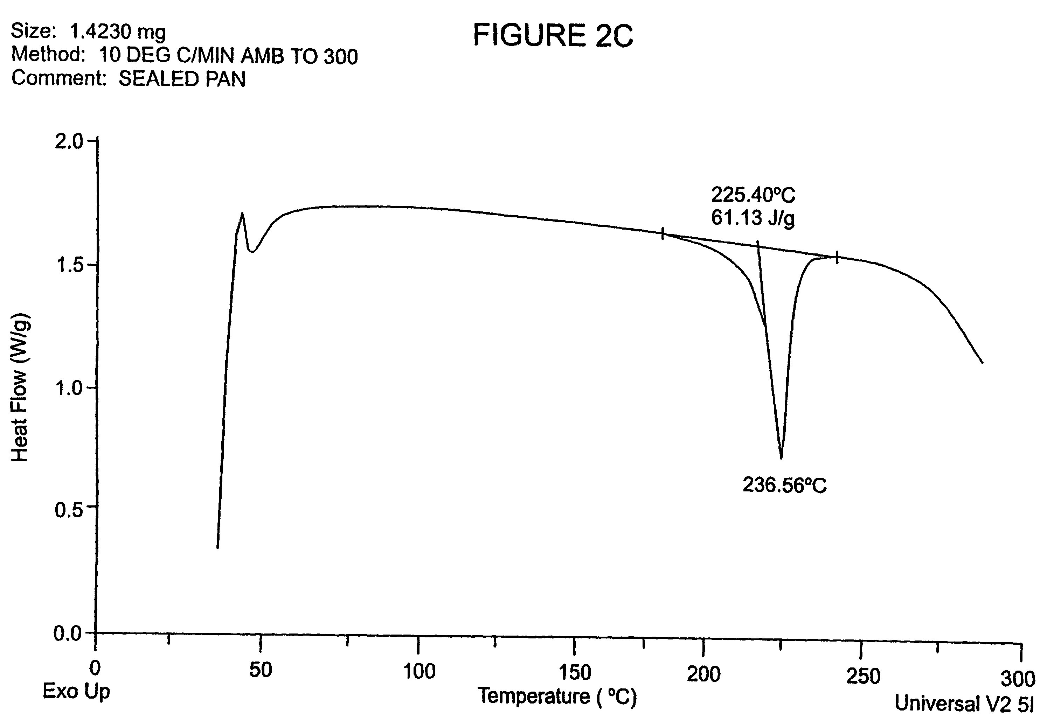

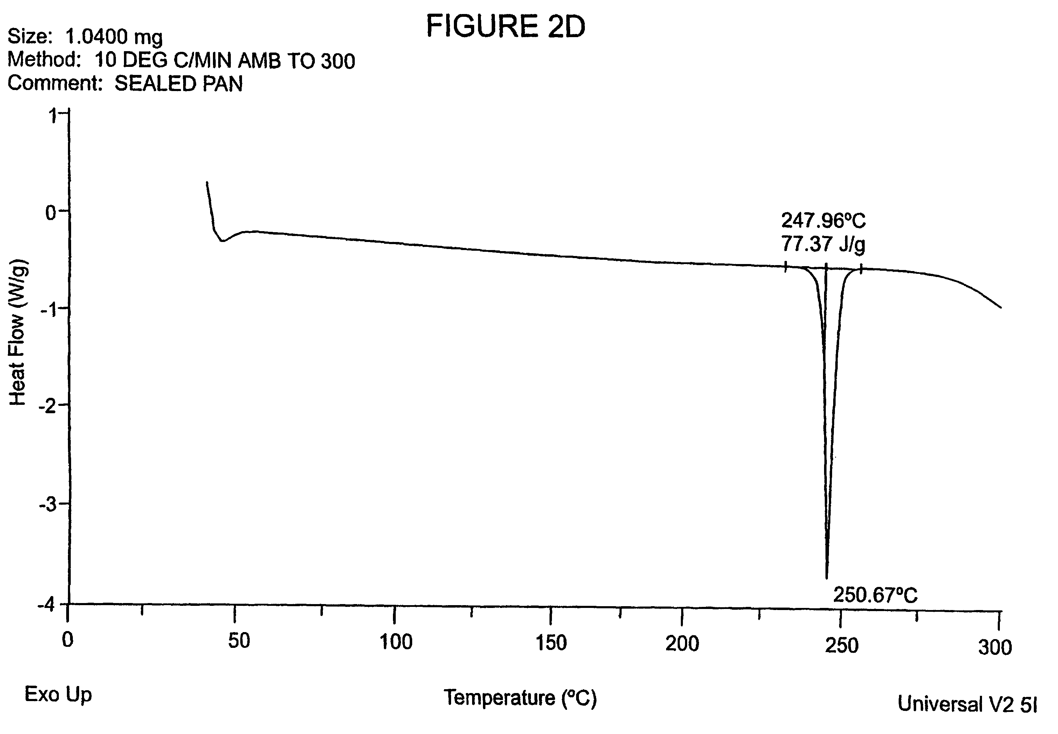

The melting of the non-solvated eplerenone crystals forms (Form H and Form L) was associated with chemical decomposition and loss of trapped solvent from the crystal lattice. The melting/decomposition temperature also was affected by the manipulation of the solid prior to analysis. For example, non-milled Form L (approximate D₉₀ particle size of about 180-450 microns) prepared by direct crystallization from an appropriate solvent or from desolvation of a solvate obtained from crystallization of high purity eplerenone in an appropriate solvent or mixture of solvents generally had a melting range of about 237-242°C. Milled Form L (approximate D₉₀ particle size of about 80-100 microns) (Form L prepared by crystallizing a solvate from a solution of high purity eplerenone in an appropriate solvent or mixture of solvents, desolvating the solvate to yield Form L, and milling the resulting Form L) generally had a lower and broader melting/decomposition range of about 223-234°C. Non-milled Form H (approximate D₉₀ particle size of about 180-450 microns) prepared by desolvation of a solvate obtained by digestion of low purity eplerenone generally had a higher melting/decomposition range of about 247-251°C. Examples of the DSC thermograms of (a) non-milled Form L directly crystallized from methyl ethyl ketone, (b) non-milled Form L prepared by desolvation of a solvate obtained by crystallization of a high purity eplerenone from methyl ethyl ketone, (c) Form L prepared by milling a desolvated solvate obtained by crystallization of high purity eplerenone from methyl ethyl ketone, and (d) non-milled Form H prepared by desolvation of a solvate obtained by digestion of low purity eplerenone from methyl ethyl ketone are given in Figures 2-A, 2-B, 2-C and 2-D, respectively.

DSC thermograms of solvated forms of eplerenone were determined using a Perkin Elmer Pyris 1 differential scanning calorimeter. Each sample (1-10 mg) was placed in an unsealed aluminum pan and heated at 10°C/minute. One or more endothermal events at lower temperatures were associated with enthalpy changes that occurred as solvent was lost from the solvate crystal lattice. The highest temperature endotherm or endotherms were associated with the melting/decomposition of Form L or Form H eplerenone.

### 4. Infrared Absorption Spectroscopy

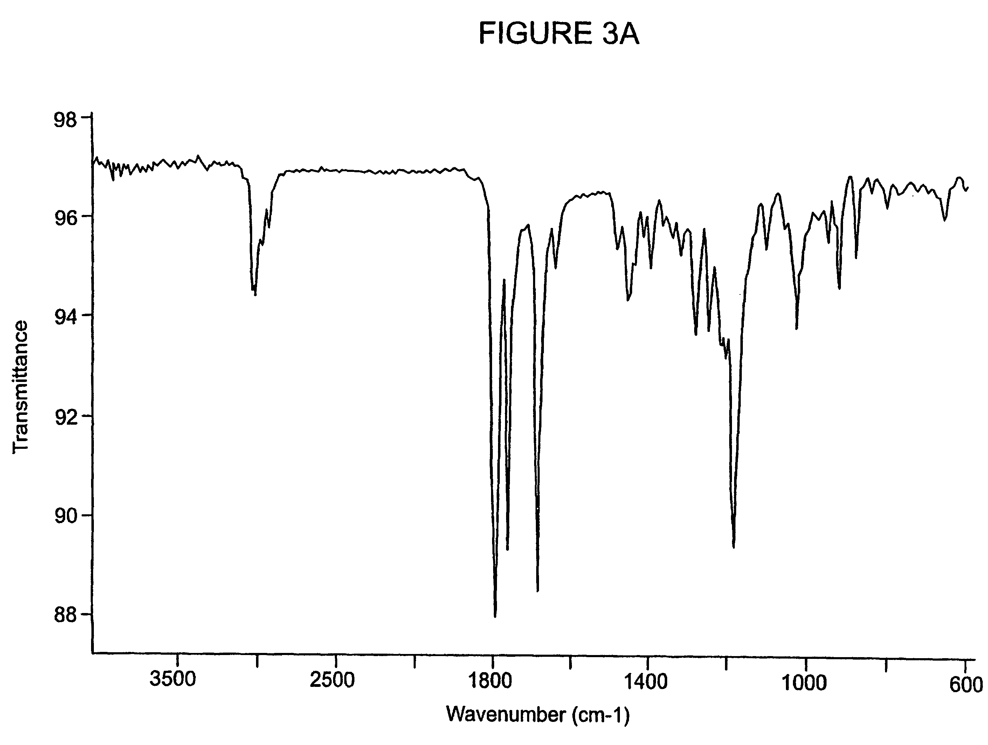

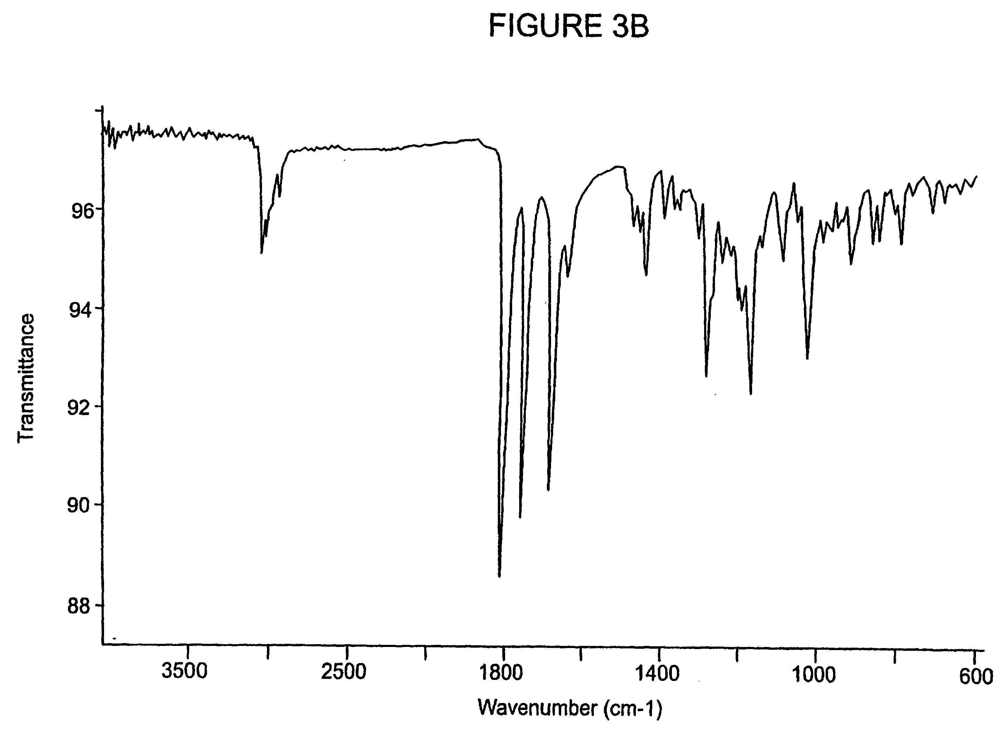

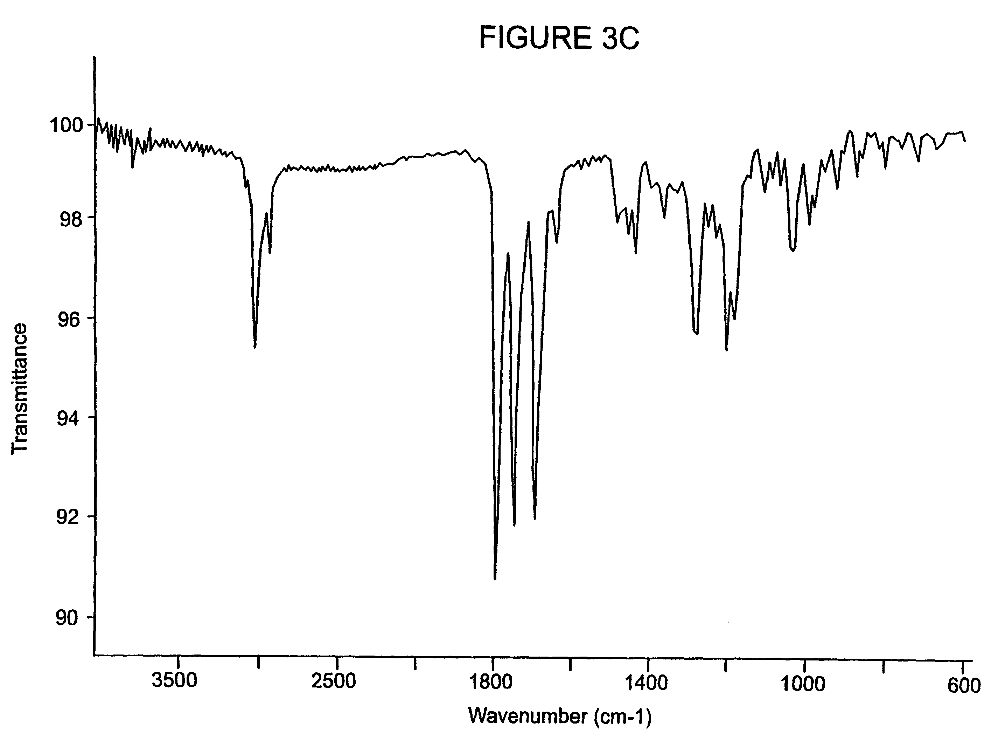

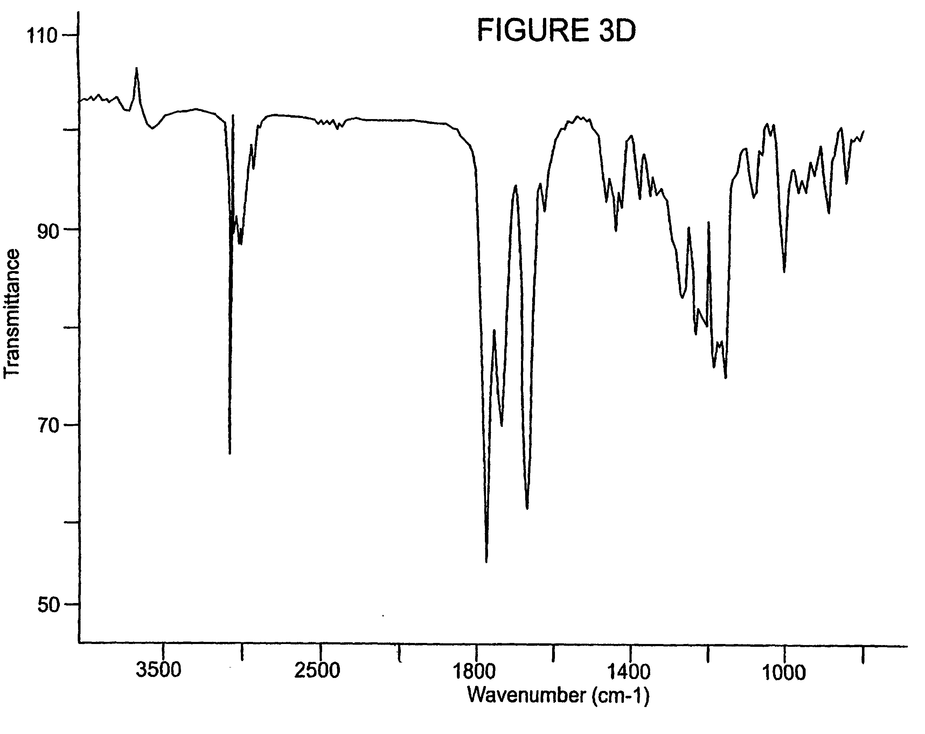

Infrared absorption spectra of the non-solvated forms of eplerenone (Form H and Form L) were obtained with a Nicolet DRIFT (diffuse reflectance infrared fourier transform) Magna System 550 spectrophotometer. A Spectra-Tech Collector system and a microsample cup were used. Samples (5%) were analyzed in potassium bromide and scanned from 400-4000 cm⁻¹ . Infrared absorption spectra of eplerenone in dilute chloroform solution (3%) or in the solvated crystal forms were obtained with a Bio-rad FTS-45 spectrophotometer. Chloroform solution samples were analyzed using a solution cell of 0.2 mm path length with sodium chloride salt plates. Solvate FTIR spectra were collected using an IBM micro-MIR (multiple internal reflectance) accessory. Samples were scanned from 400-4000 cm⁻¹ . Examples of the infrared absorption spectra of (a) Form H, (b) Form L, (c) the methyl ethyl ketone solvate, and (d) eplerenone in chloroform solution are shown in Figures 3-A, 3-B, 3-C and 3-D, respectively.

Table 2 discloses illustrative absorption bands for eplerenone in the Form H, Form L, and methyl ethyl ketone solvate crystal forms. Illustrative absorption bands for eplerenone in chloroform solution are also disclosed for comparison. Differences between Form H and either Form L or the methyl ethyl ketone solvate were observed, for example, in the carbonyl region of the spectrum. Form H has an ester carbonyl stretch of approximately 1739 cm⁻¹ while both Form L and the methyl ethyl ketone solvate have the corresponding stretch at approximately 1724 and 1722 cm⁻¹, respectively. The ester carbonyl stretch occurs at approximately 1727 cm⁻¹ in the eplerenone in chloroform solution. The change in stretching frequency of the ester carbonyl between Form H and Form L reflects the change in orientation of the ester group between the two crystal forms. In addition, the stretch of the ester of the conjugated ketone in the A-steroid ring shifts from approximately 1664-1667 cm⁻¹ in either Form H or the methyl ethyl ketone solvate to approximately 1655 cm⁻¹ in Form L. The corresponding carbonyl stretch occurs at approximately 1665 cm⁻¹ in dilute solution.

Another difference between Form H and Form L was seen in the C-H bending region. Form H has an absorption at approximately 1399 cm⁻¹ which is not observed in Form L, the methyl ethyl ketone solvate, or the eplerenone in chloroform solution. The 1399 cm⁻¹ stretch occurs in the region of CH₂ scissoring for the C2 and C21 methylene groups adjacent to carbonyl groups.

**TABLE 2**

| Absorption Region | Form H (cm⁻¹) | Form L (cm⁻¹) | Methyl Ethyl Ketone Solvate (cm⁻¹) | Eplerenone in Chloroform (cm⁻¹) |
|---|---|---|---|---|
| ν C=O(lactone) | 1773 1773 | 1775 | 1767 | 1768 |
| ν C=O(ester) | 1739 | 1724 | 1722 | 1727 |
| ν C=O(3keto) | 1664 | 1655 | 1667 | 1665 |
| ν C=C (3,4-olefin) | 1619 | 1619 | 1622 | 1623 |
| δₐₛCH3,δCH2, | 1460, | 1467, | 1467, | 1464, |
| δCH2(α to | 1444, | 1438, | 1438, | 1438, |
| carbonyl) | 1426 | 1422, 1399 | 1422, | 1422 |
| δₛCH3 | 1380 | 1381 | ∼1380 | 1378 |

### 5. Nuclear Magnetic Resonance

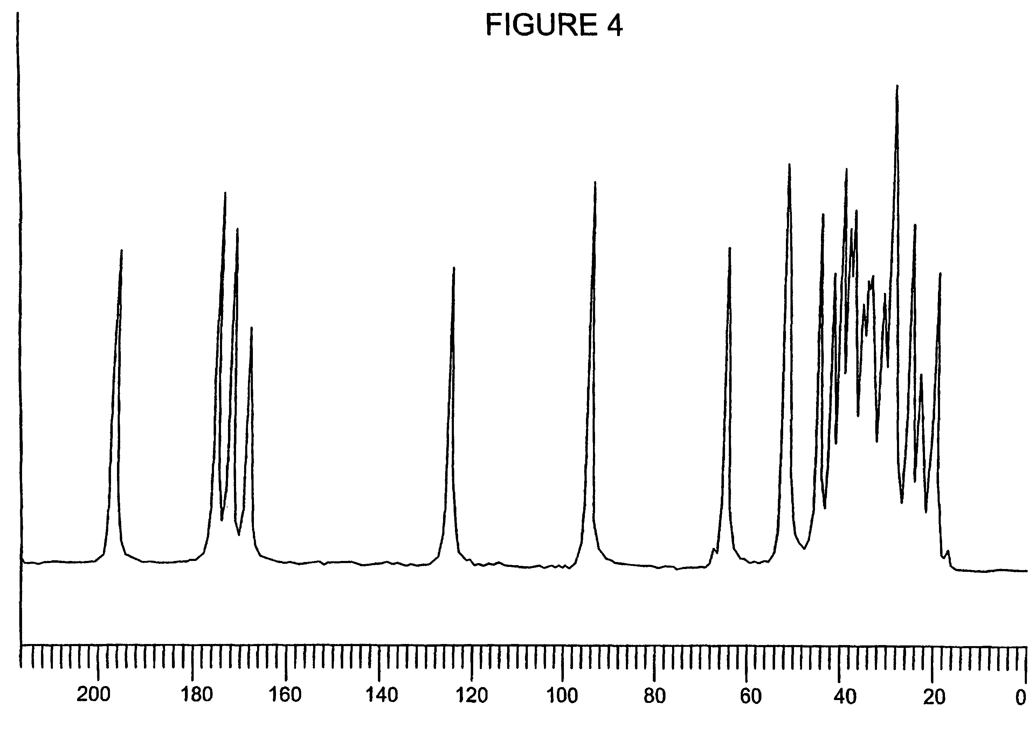

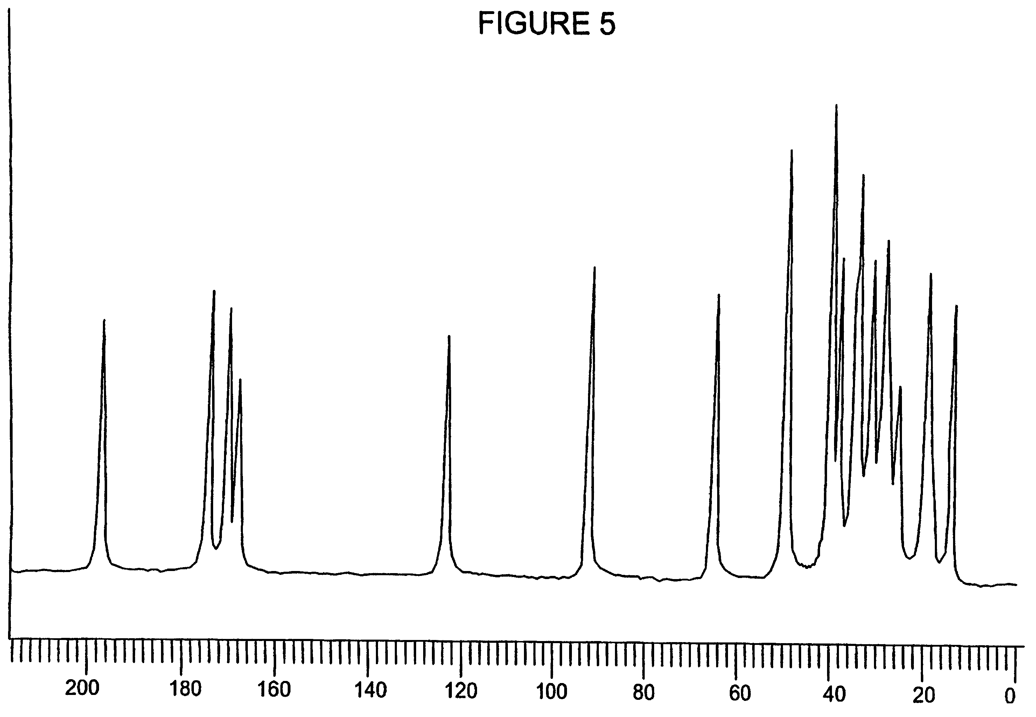

¹³C NMR spectra were obtained at a field of 31.94 MHz. Examples of the ¹³C NMR spectra of Form H and Form L eplerenone are shown in Figs. 4 and 5, respectively. The Form H eplerenone analyzed to obtain the data reflected in Fig. 4 was not phase pure and included a small amount of Form L eplerenone. Form H is most clearly distinguished by the carbon resonances at around 64.8 ppm, 24.7 ppm and 19.2 ppm. Form L is most clearly distinguished by the carbon resonances at around 67.1 ppm and 16.0 ppm.

### 6. Thermogravimetry

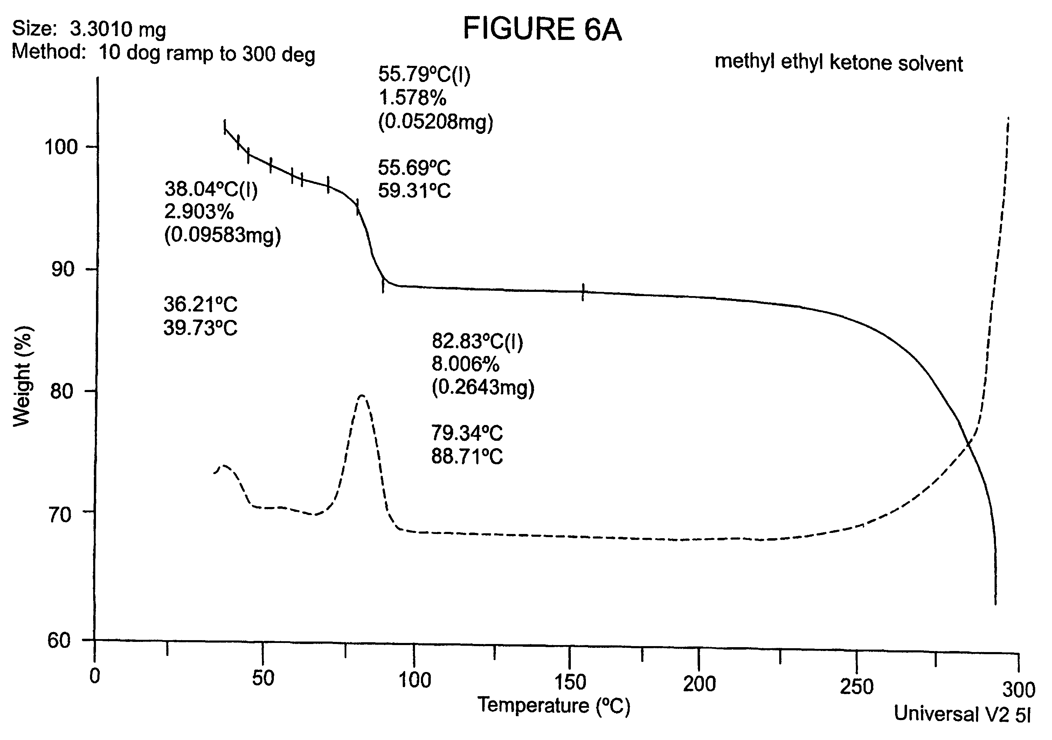

Thermogravimetric analysis of solvates was performed using a TA Instruments TGA 2950 thermogravimetric analyzer. Samples were placed in an unsealed aluminum pan under nitrogen purge. Starting temperature was 25°C with the temperature increased at a rate of about 10°C/minute. An example of the thermogravimetry analysis profile for the methyl ethyl ketone solvate is shown in Fig. 6-A.

### 7. Unit Cell Parameters

Tables 3A, 3B and 3C below summarize the unit cell parameters determined for Form H, Form L, and several solvated crystalline forms.

**TABLE 3A**

| **Parameter** | **Form H** | **Form L** | **Methyl ethyl ketone Solvate** |
|---|---|---|---|
| Crystal system | Orthorhombic | Monoclinic | Orthorhombic |
| Space group | P2₁2₁2₁ | P2₁ | P2₁2₁2₁ |
| a | 21.22 Å | 8.78 Å | 23.53 Å |
| b | 15.40 Å | 11.14 Å | 8.16 Å |
| c | 6.34 Å | 11.06 Å | 13.08 Å |
| α | 90º | 90º | 90º |
| β | 90º | 93.52º | 90º |
| γ | 90º | 90º | 90º |
| Z | 4 | 2 | 4 |
| Volume (Å) | 2071.3 | 1081.8 | 2511.4 |
| ρ (calculated) | 1.329 g/cm³ | 1.275 g/cm³ | 1.287 g/cm³ |
| R | 0.0667 | 0.062 | 0.088 |

**TABLE 3B**

| **Parameter** | **Acetone Solvate** | **Toluene Solvate** | **Butyl Acetate Solvate**^{**1**} |
|---|---|---|---|
| Crystal system | Orthorhombic | Orthorhombic | Orthorhombic |
| Space group | P2₁2₁2₁ | P2₁2₁2₁ | P2₁2₁2₁ |
| a | 23.31 Å | 23.64 Å | 23.07 Å |
| b | 13.13 Å | 13.46 Å | 13.10 Å |
| c | 8.28 Å | 8.16 Å | 8.24 Å |
| α | 90º | 90º | 90º |
| β | 90º | 90º | 90º |
| γ | 90º | 90º | 90º |
| Z | 4 | 4 | 4 |
| Volume (Å) | 2533.7 | 2596.6 | 2490.0 |
| ρ (calculated) | 1.239 g/cm³ | 1.296 g/cm³ | 1.334 g/cm³ |
| R | 0.058 | 0.089 | 0.093 |

| | | | |
|---|---|---|---|
| ¹The solvate molecules were not completely refined due to disorder of the solvent molecules in the channels. | | | |

**TABLE 3C**

| **Parameter** | **Isobutyl Acetate Solvate**^{**1**} | **Isopropanol Solvate**^{**1**} | **Ethanol Solvate**^{**1**} |
|---|---|---|---|
| Crystal system | Orthorhombic | Orthorhombic | Orthorhombic |
| Space group | P2₁2₁2₁ | P2₁2₁2₁ | P2₁2₁2₁ |
| a | 23.19 Å | 23.15 Å | 23.51 Å |
| b | 12.95 Å | 12.73 Å | 13.11 Å |
| c | 8.25 Å | 8.25 Å | 8.27 Å |
| *α* | 90º | 90º | 90º |
| β | 90º | 90º | 90º |
| γ | 90º | 90º | 90º |
| Z | 4 | 4 | 4 |
| Volume (Å) | 2476.4 | 2433.2 | 2548.6 |
| ρ (calculated) | 1.337 g/cm³ | 1.296 g/cm³ | 1.234 g/cm³ |
| R | 0.098 | 0.152 | 0.067 |

| | | | |
|---|---|---|---|
| ¹The solvate molecules were not refined completely due to disorder of the solvent molecules in the channels. | | | |

Additional information on selected solvated crystalline forms of eplerenone is reported in Table 4 below. The unit cell data reported in Table 3A above for the methyl ethyl ketone solvate also are representative of the unit cell parameters for many of these additional eplerenone crystalline solvates. Most of the eplerenone crystalline solvates tested are substantially isostructural to each other. While there may be some minor shifting in the X-ray powder diffraction peaks from one solvated crystalline form to the next due to the size of the incorporated solvent molecule, the overall diffraction patterns are substantially the same and the unit cell parameters and molecular positions are substantially identical for most of the solvates tested.

**TABLE 4**

| **Solvent** | **Stoichiometry (Solvent: Eplerenone)** | **Isostructural to Methyl Ethyl ketone Solvate?** | **Desolvation Temperature**^{**1**} **(°C)** |
|---|---|---|---|
| Methyl Ethyl Ketone | 1:1 | N/A | 89 |
| 2-Pentanone | --- | --- | --- |
| Acetic Acid | 1:2 | Yes | 203 |
| Acetone | 1:1 | Yes | 117 |
| Butyl Acetate | 1:2 | Yes | 108 |
| Chloroform | --- | Yes | 125 |
| Ethanol | 1:1 | Yes | 166 |
| Isobutanol | --- | --- | --- |
| Isobutyl Acetate | 1:2 | Yes | 112 |
| Isopropanol | 1:1 | Yes | 121 |
| Methyl Acetate | 1:1 | Yes | 103 |
| Ethyl Propionate | 1:1 | Yes | 122 |
| n-Butanol | 1:1 | Yes | 103 |
| n-Octanol | --- | Yes | 116 |
| n-Propanol | 1:1 | Yes | 129 |
| Propyl Acetate | 1:1 | Yes | 130 |
| Propylene Glycol | --- | Yes | 188 |
| t-Butanol | --- | --- | --- |
| Tetrahydrofuran | 1:1 | Yes | 136 |
| Toluene | 1:1 | Yes | 83 |
| t-Butyl Acetate | --- | Yes | 109 |

| | | | |
|---|---|---|---|
| ¹Defined as the extrapolated desolvation temperature from the final solvent weight loss step as determined by thermogravimetric analysis at a heating rate of 10° C/minute under nitrogen purge. Desolvation temperatures, however, can be affected by the method of manufacture of the solvate. Different methods can produce different numbers of nucleation sites capable of initiating desolvation in the solvate at lower temperatures. | | | |

The unit cell of the solvate is composed of four eplerenone molecules. The stoichiometry of the eplerenone molecules and solvent molecules in the unit cell is also reported in Table 4 above for a number of solvates. The unit cell of Form H is composed of four eplerenone molecules. The unit cell of Form L is composed of two eplerenone molecules. The solvate unit cells are converted during desolvation into Form H and/or Form L unit cells when the eplerenone molecules undergo translation and rotation to fill the spaces left by the solvent molecules. Table 4 also reports the desolvation temperatures for a number of different solvates.

### 8. Crystal Properties of Impurity Molecules

Selected impurities in eplerenone can induce the formation of Form H during the desolvation of the solvate. In particular, the effect of the following two impurity molecules was evaluated: 7-methyl hydrogen 4α,5α :9α,11α-diepoxy-17-hydroxy-3-oxo-17α-pregnane-7α,21-dicarboxylate, γ-lactone **3** (the "diepoxide"); and 7-methyl hydrogen 11α, 12α-epoxy-17-hydroxy-3-oxo-17α-pregn-4-ene-7 α,21-dicarboxylate, γ-lactone **4** (the "11,12-epoxide").

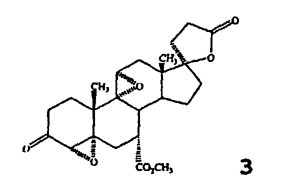

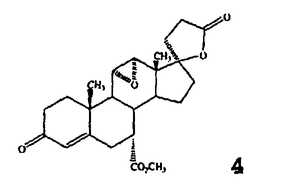

The effect of these impurity molecules on the eplerenone crystalline form resulting from desolvation is described in greater detail in the examples of this application.

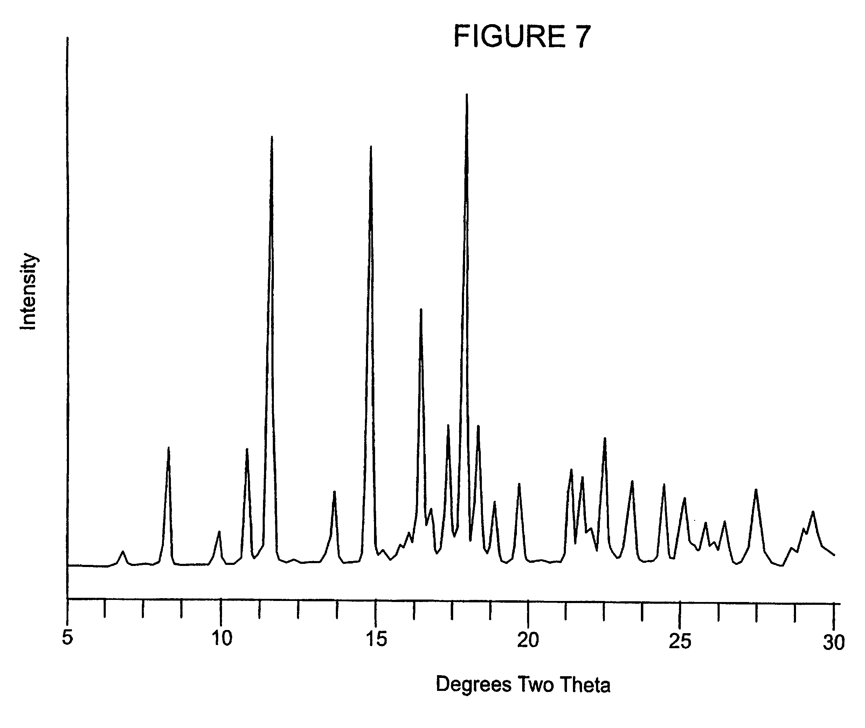

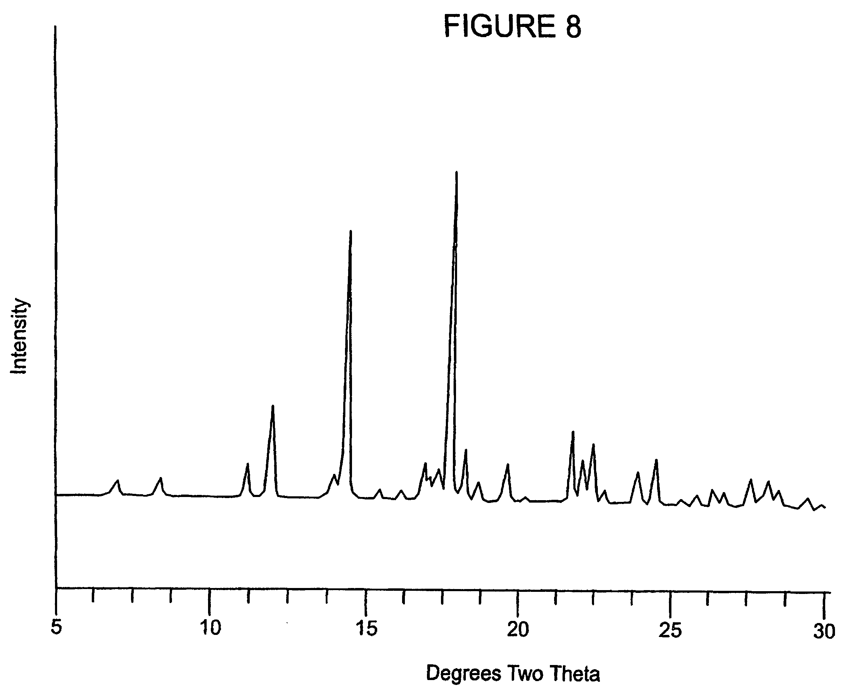

Given the similarity in single crystal structure of 7-methyl hydrogen 17-hydroxy-3-oxo-17α-pregna-4,9(11)-diene-7α,21-dicarboxylate, γ-lactone **5** (the "9,11-olefin") and Form H, it is hypothesized that the 9,11-olefin also can induce the formation of Form H during the desolvation of the solvate. 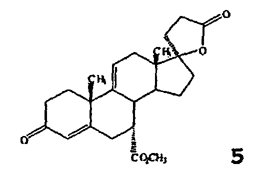 The diepoxide, 11,12-olefin and 9,11-olefin can be prepared as set forth, for example, in Examples 47C, 47B and 37H of Ng et al., WO98/25948, respectively.
A single crystal form was isolated for each impurity compound. Representative X-ray powder diffraction patterns for the crystal forms isolated for the diepoxide, 11,12-epoxide and 9,11-olefin are given in Figs. 7, 8,and 10, respectively. The X-ray powder diffraction pattern of each impurity molecule is similar to the X-ray powder diffraction pattern of Form H, suggesting that Form H and the three impurity compounds have similar single crystal structures.

Single crystals of each impurity compound also were isolated and subjected to X-ray structure determination to verify that these three compounds adopt single crystal structures similar to that of Form H. Single crystals of the diepoxide were isolated from methyl ethyl ketone. Single crystals of the 11,12-epoxide were isolated from isopropanol. Single crystals of the 9,11-olefin were isolated from n-butanol. Crystal structure data determined for the crystalline form of each impurity compound are given in Table 5. The resulting crystal system and cell parameters were substantially the same for the Form H, diepoxide, 11,12-epoxide, and 9,11-olefin crystalline forms.

**(a) TABLE 5**

| **Parameter** | **Form H** | **Diepoxide** | **11,12 Epoxide** | **9,11 olefin** |
|---|---|---|---|---|
| Crystal system | Orthorhombic | Orthorhombic | Orthorhombic | Orthorhombic |
| Space group | P2₁2₁2₁ | P2₁2₁2₁ | P2₁2₁2₁ | P2₁2₁2₁ |
| a | 21.22 Å | 21.328 Å | 20.90 Å | 20.90 Å |
| b | 15.40 Å | 16.16 Å | 15.55Å | 15.74 Å |
| c | 6.34 Å | 6.15 Å | 6.38 Å | 6.29 Å |
| α | 90º | 90º | 90º | 90º |
| β | 90º | 90º | 90º | 90º |
| γ | 90º | 90º | 90º | 90º |
| Z | 4 | 4 | 4 | 4 |
| Volume (Å) | 2071.3 | 2119.0 | 2073.2 | 2069.3 |
| ρ (calculate d) | 1.329 g/cm³ | 1.349 g/cm³ | 1.328 g/cm³ | 1.279 g/cm³ |
| R | 0.0667 | 0.0762 | 0.0865 | 0.0764 |

The four compounds reported in Table 5 crystallize into the same space group and have similar cell parameters (i.e., they are isostructural). It is hypothesized that the diepoxide, 11,12-epoxide and 9,11-olefin adopt a Form H conformation. The relative ease of isolation of a Form H packing (directly from solution) for each impurity compound, indicates that the Form H lattice is a stable packing mode for this series of structurally similar compounds.

### Preparation of Eplerenone

The eplerenone starting material used to prepare the novel crystalline forms of the present invention can be prepared using the methods set forth in Ng et al., WO97/21720; and Ng et al., WO98/25948, particularly scheme 1 set forth in WO97/21720 and WO98/25948.

### Preparation of Crystalline Forms

### 1. Preparation of Solvated Crystalline Form

The solvated crystalline forms of eplerenone can be prepared by crystallization of eplerenone from a suitable solvent or a mixture of suitable solvents. A suitable solvent or mixture of suitable solvents generally comprises an organic solvent or a mixture of organic solvents that solubilizes the eplerenone together with any impurities at an elevated temperature, but upon cooling, preferentially crystallizes the solvate. The solubility of eplerenone in such solvents or mixtures of solvents generally is about 5 to about 200 mg/mL at room temperature. The solvent or mixtures of solvents preferably are selected from those solvents previously used in the process to prepare the eplerenone starting material, particularly those solvents that would be pharmaceutically acceptable if contained in the final pharmaceutical composition comprising the eplerenone crystalline form. For example, a solvent system comprising methylene chloride that yields a solvate comprising methylene chloride generally is not desirable.

Each solvent used preferably is a pharmaceutically acceptable solvent, particularly a Class 2 or Class 3 solvent as defined in "Impurities: Guideline For Residual Solvents", International Conference On Harmonisation Of Technical Requirements For Registration Of Pharmaceuticals For Human Use (Recommended for Adoption at Step 4 of the ICH Process on July 17, 1997 by the ICH Steering Committee). Still more preferably, the solvent or mixture of solvents is selected from the group consisting of methyl ethyl ketone, 1-propanol, 2-pentanone, acetic acid, acetone, butyl acetate, chloroform, ethanol, isobutanol, isobutyl acetate, methyl acetate, ethyl propionate, n-butanol, n-octanol, isopropanol, propyl acetate, propylene glycol, t-butanol, tetrahydrofuran, toluene, methanol and t-butyl acetate. Still more preferably, the solvent is selected from the group consisting of methyl ethyl ketone and ethanol.

To prepare the solvated crystalline form of eplerenone, an amount of the eplerenone starting material is solubilized in a volume of the solvent and cooled until crystals form. The solvent temperature at which the eplerenone is added to the solvent generally will be selected based upon the solubility curve of the solvent or mixture of solvents. For most of the solvents described herein, for example, this solvent temperature typically is at least about 25°C, preferably from about 30°C to the boiling point of the solvent, and more preferably from about 25°C below the boiling point of the solvent to the boiling point of the solvent.

Alternatively, hot solvent may be added to the eplerenone and the mixture can be cooled until crystals form. The solvent temperature at the time it is added to the eplerenone generally will be selected based upon the solubility curve of the solvent or mixture of solvents. For most of the solvents described herein, for example, the solvent temperature typically is at least 25°C, preferably from about 50°C to the boiling point of the solvent, and more preferably from about 15°C below the boiling point of the solvent to the boiling point of the solvent.

The amount of the eplerenone starting material mixed with a given volume of solvent likewise will depend upon the solubility curve of the solvent or mixture of solvents. Typically, the amount of eplerenone added to the solvent will not completely solubilize in that volume of solvent at room temperature. For most of the solvents described herein, for example, the amount of eplerenone starting material mixed with a given volume of solvent usually is at least about 1.5 to about 4.0 times, preferably about 2.0 to about 3.5 times, and more preferably about 2.5 times, the amount of eplerenone that will solubilize in that volume of solvent at room temperature.

After the eplerenone starting material has completely solubilized in the solvent, the solution typically is cooled slowly to crystallize the solvated crystalline form of eplerenone. For most of the solvents described herein, for example, the solution is cooled at a rate slower than about 20°C/minute, preferably at a rate of about 10°C/minute or slower, more preferably at a rate of about 5°C/minute or slower, and still more preferably at a rate of about 1°C/minute or slower.

The endpoint temperature at which the solvated crystalline form is harvested will depend upon the solubility curve of the solvent or mixture of solvents. For most of the solvents described herein, for example, the endpoint temperature typically is less than about 25°C, preferably less than about 5°C, and more preferably less than about -5°C. Decreasing the endpoint temperature generally favors the formation of the solvated crystalline form.

Alternatively, other techniques may be used to prepare the solvate. Examples of such techniques include, but are not limited to, (i) dissolving the eplerenone starting material in one solvent and adding a co-solvent to aid in the crystallization of the solvate crystalline form, (ii) vapor diffusion growth of the solvate, (iii) isolation of the solvate by evaporation, such as rotary evaporation, and (iv) slurry converstion.

The crystals of the solvated crystalline form prepared as described above can be separated from the solvent by any suitable conventional means such as by filtration or centrifugation. Increased agitation of the solvent system during crystallization generally results in smaller crystal particle sizes.

### 2. Preparation of Form L From Solvate

Form L eplerenone can be prepared directly from the solvated crystalline form by desolvation. Desolvation can be accomplished by any suitable desolvation means such as, but not limited to, heating the solvate, reducing the ambient pressure surrounding the solvate, or combinations thereof. If the solvate is heated to remove the solvent, such as in an oven, the temperature of the solvate during this process typically does not exceed the enantiotropic transition temperature for Form H and Form L. This temperature preferably does not exceed about 150°C.

The desolvation pressure and time of desolvation are not narrowly critical. The desolvation pressure preferably is about one atmosphere or less. As the desolvation pressure is reduced, however, the temperature at which the desolvation can be carried out and/or the time of desolvation likewise is reduced. Particularly for solvates having higher desolvation temperatures, drying under vacuum will permit the use of lower drying temperatures. The time of desolvation need only be sufficient to allow for the desolvation, and thus the formation of Form L, to reach completion.

To ensure the preparation of a product that comprises substantially all Form L, the eplerenone starting material typically is a high purity eplerenone, preferably substantially pure eplerenone. The eplerenone starting material used to prepare Form L eplerenone generally is at least 90% pure, preferably at least 95% pure, and more preferably at least 99% pure. As discussed in greater detail elsewhere in this application, certain impurities in the eplerenone starting material can adversely affect the yield and Form L content of the product obtained from the process.
The crystallized eplerenone product prepared in this manner from a high purity eplerenone starting material generally comprises at least 10% Form L, preferably at least 50% Form L, more preferably at least 75% Form L, still more preferably at least 90% Form L, still more preferably at least about 95% Form L, and still more preferably substantially phase pure Form L.

### 2. Preparation of Form H From Solvate

A product comprising Form H can be prepared in substantially the same manner as set forth above for the preparation of Form L by (i) using a low purity eplerenone starting material instead of a high purity eplerenone starting material, (ii) seeding the solvent system with phase pure Form H crystals, or (iii) a combination of (i) and (ii).

### A. Use Of Impurities As Growth Promoters and Inhibitors

The presence and amount of selected impurities in the eplerenone starting material, rather than the total amount of all impurities in the eplerenone starting material, affect the potential for Form H crystal formation during the desolvation of the solvate. The selected impurity generally is a Form H growth promoter or Form L growth inhibitor. It may be contained in the eplerenone starting material, contained in the solvent or mixture of solvents before the eplerenone starting material is added, and/or added to the solvent or mixture of solvents after the eplerenone starting material is added. Bonafede et al., "Selective Nucleation and Growth of an Organic Polymorph by Ledge-Directed Epitaxy on a Molecular Crystal Substate", J. Amer. Chem. Soc., Vol. 117, No. 30 (August 2, 1995) discusses the use of growth promoters and growth inhibitors in polymorph systems and is incorporated by reference herein. For the present invention, the impurity generally comprises a compound having a single crystal structure substantially identical to the single crystal structure of Form H. The impurity preferably is a compound having an X-ray powder diffraction pattern substantially identical to the X-ray powder diffraction pattern of Form H, and more preferably is selected from the group consisting of the diepoxide, the 11,12-epoxide, the 9,11-olefin and combinations thereof.

The amount of impurity needed to prepare Form H crystals typically can depend, in part, upon the solvent or mixture of solvents and the solubility of the impurity relative to eplerenone. In the crystallization of Form H from a methyl ethyl ketone solvent, for example, the weight ratio of diepoxide to low purity eplerenone starting material typically is at least about 1:100, preferably at least about 3:100, more preferably between about 3:100 and about 1:5, and still more preferably between about 3:100 and about 1:10. The 11,12-epoxide has a higher solubility in methyl ethyl ketone than the diepoxide and generally requires a larger amount of the 11,12-epoxide generally is necessary to prepare Form H crystals. Where the impurity comprises the 11,12-epoxide, the weight ratio of the diepoxide to the low purity eplerenone starting material typically is at least about 1:5, more preferably at least about 3:25, and still more preferably between about 3:25 and about 1:5. Where both the diexpoxide and the 11,12-epoxide impurities are used in the preparation of the Form H crystals, the weight ratio of each impurity to the eplerenone starting material may be lower than the corresponding ratio when only that impurity is used in the preparation of the Form H crystals.

A mixture of Form H and Form L is generally obtained when a solvate comprising the selected impurity is desolvated. The weight fraction of Form H in the product resulting from the initial desolvation of the solvate typically is less than about 50%. Further treatment of this product by crystallization or digestion, as discussed below, generally will increase the weight fraction of Form L in the product.

### Seeding

Form H crystals also can be prepared by seeding the solvent system with phase pure Form H crystals (or a Form H growth promoter and/or Form L growth inhibitor as previously discussed above) prior to crystallization of the eplerenone. The eplerenone starting material can be either a low purity eplerenone or a high purity eplerenone. When the resulting solvate prepared from either starting material is desolvated, the weight fraction of Form H in the product typically is at least about 70% and may be as great as about 100%.

The weight ratio of Form H seed crystals added to the solvent system to the eplerenone starting material added to the solvent system generally is at least about 0.75:100, preferably between about 0.75:100 to about 1:20, and more preferably between about 1:100 to about 1:50. The Form H seed crystals can be prepared by any of the methods discussed in this application for the preparation of Form H crystals, particularly the preparation of Form H crystals by digestion as discussed below.

The Form H seed crystals may be added at one time, in multiple additions or substantially continually over a period of time. The addition of the Form H seed crystals, however, generally is completed before the eplerenone begins to crystallize from solution, i.e., the seeding is completed before the cloud point (the lower end of the metastable zone) is reached. Seeding typically is performed when the solution temperature ranges from about 0.5°C above the cloud point to about 10°C above the cloud point, preferably within about 2°C to about 3°C above the cloud point. As the temperature above the cloud point at which the seeds are added increases, the amount of seeding needed for crystallization of Form H crystals generally increases.

The seeding preferably occurs not only above the cloud point, but within the metastable zone. Both the cloud point and the metastable zone are dependent on the eplerenone solubility and concentration in the solvent or mixture of solvents. For a 12 volume dilution of methyl ethyl ketone, for example, the high end of the metastable zone generally is between about 70°C to about 73°C and the lower end of the metastable zone (i.e., the cloud point) is between about 57°C and 63°C. For a concentration of 8 volumes of methyl ethyl ketone, the metastable zone is even narrower because the solution is supersaturated. At this concentration, the cloud point of the solution occurs at about 75°C to about 76°C. Because the boiling point of methyl ethyl ketone is about 80°C under ambient conditions, seeding for this solution typically occurs between about 76.5°C and the boiling point.

An illustrative non-limiting example of seeding with Form H is set forth below in Example 11.
The crystallized eplerenone product obtained using a Form H growth promoter or Form L growth inhibitor, and/or Form H seeding generally comprises at least 2% Form H, preferably at least 5% Form H, more preferably at least 7% Form H, and still more preferably at least about 10% Form H. The remaining crystallized eplerenone product generally is Form L.

### Form H Prepared By Grinding Eplerenone

In yet another alternative, it has been discovered that a small amount of Form H can be prepared by suitable grinding eplerenone. Concentrations of Form H in ground eplerenone as high as about 3% have been observed.

### 4. Preparation of Form L From Solvate Prepared From Low Purity Eplerenone

As discussed above, crystallization of low purity eplerenone to form a solvate followed by desolvation of the solvate generally yields a product comprising both Form H and Form L. A product having a greater Form L content can be prepared from low purity eplerenone in substantially the same manner as set forth above for the preparation of Form H by seeding the solvent system with phase pure Form L crystals, or by using a Form L growth promoter and/or Form H growth inhibitor. The seeding protocol and the weight ratio of the amount of Form L seed crystals added to the solvent system to the amount of the eplerenone starting material added to the solvent system generally are similar to those ratios previously discussed above for the preparation of Form H eplerenone by seeding with phase pure Form H crystals.

The crystallized eplerenone product prepared in this manner generally comprises at least 10% Form L, preferably at least 50% Form L, more preferably at least 75% Form L, more preferably at least 90% Form L, still more preferably at least about 95% Form L, and still more preferably substantially phase pure Form L.

The seeding protocols described in this section and in the prior section relating to the preparation of Form H eplerenone also may allow for improved control of the particle size of the crystallized eplerenone.

### 5. Crystallization of Form L Directly From Solution

Form L eplerenone also can be prepared by the direct crystallization of eplerenone from a suitable solvent or mixture of solvents without the formation of an intermediate solvate and the accompanying need for desolvation. Typically, (i) the solvent has a molecular size that is incompatible with the available channel space in the solvate crystal lattice, (ii) the eplerenone and any impurities are soluble in the solvent at elevated temperatures, and (iii) upon cooling, results in the crystallization of the non-solvated Form L eplerenone. The solubility of eplerenone in the solvent or mixture of solvents generally is about 5 to about 200 mg/mL at room temperature. The solvent or mixture of solvents preferably comprises one or more solvents selected from the group consisting of methanol, ethyl acetate, isopropyl acetate, acetonitrile, nitrobenzene, water and ethyl benzene.

To crystallize Form L eplerenone directly from solution, an amount of the eplerenone starting material is solubilized in a volume of the solvent and cooled until crystals form. The solvent temperature at which the eplerenone is added to the solvent generally will be selected based upon the solubility curve of the solvent or mixture of solvents. For most of the solvents described herein, for example, this solvent temperature typically is at least about 25°C, preferably from about 30°C to the boiling point of the solvent, and more preferably from about 25°C below the boiling point of the solvent to the boiling point of the solvent.

Alternatively, hot solvent may be added to the eplerenone and the mixture can be cooled until crystals form. The solvent temperature at the time it is added to the eplerenone generally will be selected based upon the solubility curve of the solvent or mixture of solvents. For most of the solvents described herein, for example, the solvent temperature typically is at least 25°C, preferably from about 50°C to the boiling point of the solvent, and more preferably from about 15°C below the boiling point of the solvent to the boiling point of the solvent.

The amount of the eplerenone starting material mixed with a given volume of solvent likewise will depend upon the solubility curve of the solvent or mixture of solvents. Typically, the amount of eplerenone added to the solvent will not completely solubilize in that volume of solvent at room temperature. For most of the solvents described herein, for example, the amount of eplerenone starting material mixed with a given volume of solvent usually is at least about 1.5 to about 4.0 times, preferably about 2.0 to about 3.5 times, and more preferably about 2.5 times, the amount of eplerenone that will solubilize in that volume of solvent at room temperature.

To ensure the preparation of a product that comprises substantially phase pure Form L, the eplerenone starting material generally is a high purity eplerenone. The eplerenone starting material preferably is at least 65% pure, more preferably at least 90% pure, still more preferably at least 98% pure, and still more preferably at least 99% pure.

After the eplerenone starting material has completely solubilized in the solvent, the solution typically is cooled slowly to crystallize the solvated crystalline form of eplerenone. For most of the solvents described herein, for example, the solution is cooled at a rate slower than about 1.0°C/minute, preferably at a rate of about 0.2°C/minute or slower, and more preferably at a rate between about 5°C/minute and about 0.1°C/minute.

The endpoint temperature at which the Form L crystals are harvested will depend upon the solubility curve of the solvent or mixture of solvents. For most of the solvents described herein, for example, the endpoint temperature typically is less than about 25°C, preferably less than about 5°C, and more preferably less than about -5°C.

Alternatively, other techniques may be used to prepare the Form L crystals. Examples of such techniques include, but are not limited to, (i) dissolving the eplerenone starting material in one solvent and adding a co-solvent to aid in the crystallization of Form L eplerenone, (ii) vapor diffusion growth of Form L eplerenone, (iii) isolation of Form L eplerenone by evaporation, such as rotary evaporation, and (iv) slurry conversion.

The crystals of the solvated crystalline form prepared as described above can be separated from the solvent by any suitable conventional means such as by filtration or centrifugation.

In addition, Form L eplerenone also can be prepared by digesting (as described below) a slurry of high purity eplerenone in methyl ethyl ketone and filtering the digested eplerenone at the boiling point of the slurry.

### 6. Preparation of Form H Directly From Solution

It is hypothesized that if the crystallization is performed above the enantiotropic transition temperature (Tₜ) for Form H and Form L, particularly if Form H growth promoters or Form L growth inhibitors are present or the solvent is seeded with phase pure Form H crystals, Form H should crystallize directly from solution since Form H is more stable at these higher temperatures. The solvent system used preferably comprises a high boiling solvent such as nitrobenzene. Suitable Form H growth promoters would include, but would not be limited to, the diepoxide and the 11,12-olefin.

### 7. Digestion of Eplerenone With A Solvent

The solvated crystalline forms, Form H and Form L of eplerenone also can be prepared by digestion of an eplerenone starting material in a suitable solvent or mixture of solvents. In the digestion process, a slurry of eplerenone is heated at the boiling point of the solvent or mixture of solvents. For example, an amount of eplerenone starting material is combined with a volume of solvent or mixture of solvents, heated to reflux, and the distillate is removed while an additional amount of the solvent is added simultaneously with the removal of the distillate. Alternatively, the distillate can be condensed and recycled without the addition of more solvent during the digestion process. Typically, once the original volume of solvent has been removed or condensed and recycled, the slurry is cooled and solvated crystals form. The solvated crystals can be separated from the solvent by any suitable conventional means such as by filtration or centrifugation. Desolvation of the solvate as previously described yields either Form H or Form L eplerenone depending upon the presence or absence of the selected impurities in the solvated crystals.

A suitable solvent or mixture of solvents generally comprises one or more of the solvents previously disclosed herein. The solvent may be selected, for example, from the group consisting of methyl ethyl ketone and ethanol.
The amount of eplerenone starting material added to the solvent used in the digestion process generally is sufficient to maintain a slurry (i.e., the eplerenone in the solvent or mixture of solvents is not completely solubilized) at the boiling point of the solvent or mixture of solvents. Illustrative values include, but are not limited to, about one gram of eplerenone per four mL methyl ethyl ketone and about one gram of eplerenone per eight mL ethanol.

The solution generally is cooled slowly once solvent turnover is complete to crystallize the solvated crystalline form of eplerenone. For the solvents tested, for example, the solution is cooled at a rate slower than about 20°C/minute, preferably about 10°C/minute or slower, more preferably about 5°C/minute or slower, and still more preferably about 1°C/minute or slower.

The endpoint temperature at which the solvated crystalline form is harvested will depend upon the solubility curve of the solvent or mixture of solvents. For most of the solvents described herein, for example, the endpoint temperature typically is less than about 25°C, preferably less than about 5°C, and more preferably less than about -5°C.

If a product comprising primarily or exclusively Form L is desired, a high purity eplerenone starting material typically is digested. The high purity eplerenone starting material preferably is at least 98% pure, more preferably at least 99% pure, and still more preferably at least 99.5% pure. The digested eplerenone product prepared in this manner generally comprises at least 10% Form L, preferably at least 50% Form L, more preferably at least 75% Form L, more preferably at least 90% Form L, still more preferably at least about 95% Form L, and still more preferably substantially phase pure Form L.

If a product comprising primarily or exclusively Form H is desired, a low purity eplerenone starting material typically is digested. The low purity eplerenone starting material generally contains only as much Form H growth promoter and/or Form L growth inhibitor as is needed to yield Form H. Preferably, the low purity eplerenone starting material is at least 65% pure, more preferably at least 75% pure, and still more preferably at least 80% pure. The digested eplerenone product prepared in this manner generally comprises at least 10% Form H, preferably at least 50% Form H, more preferably at least 75% Form H, more preferably at least 90% Form H, still more preferably at least about 95% Form H, and still more preferably substantially phase pure Form H.

### 8. Preparation of Amorphous Eplerenone

Amorphous eplerenone can be prepared in small quantities by suitable comminution of solid eplerenone, such as by crushing, grinding and/or micronizing. Phase pure amorphous eplerenone can be prepared, for example, by lyophilizing a solution of eplerenone, particularly an aqueous solution of eplerenone. These processes are illustrated in Examples 17 and 18 below.

### Working Examples

The following examples contain detailed descriptions of the methods of preparation of the various solid state forms of eplerenone described in this application. These detailed descriptions fall within the scope, and serve to exemplify the invention. These detailed descriptions are presented for illustrative purposes only and are not intended as a restriction on the scope of the invention. All parts are by weight and temperatures are in degrees Centigrade unless otherwise indicated. The eplerenone starting material used in each of the following examples was prepared in accordance with scheme 1 set forth in Ng et al., WO98/25948.

### Example 5: Preparation of (a) methyl ethyl ketone solvate from high purity eplerenone starting material and (b) Form L crystalline eplerenone from resulting solvate.

### A. Preparation of Methyl Ethyl Ketone Solvate:

High purity eplerenone (437 mg; greater than 99% purity with less than 0.2% diepoxide and 11,12 epoxide present) was dissolved in 10 mL of methyl ethyl ketone by heating to boiling on a hot plate with magnetic stirring at 900 rpm. The resulting solution was allowed to cool to room temperature with continuous magnetic stirring. Once at room temperature, the solution was transferred to a 1°C bath with maintenance of the stirring for one hour.
After one hour, the solid methyl ethyl ketone solvate was collected by vacuum filtration.

### B. Preparation of Form L crystalline eplerenone:

The solid methyl ethyl ketone solvate prepared in Step A above was dried in an oven at 100°C for four hours at ambient pressure. The dried solid was determined to be pure Form L by DSC and XPRD analysis.

### Example 6: Preparation of additional solvates from high purity eplerenone starting material

Additional solvated crystalline forms were prepared by replacing methyl ethyl ketone with one of the following solvents: n-propanol, 2-pentanone, acetic acid, acetone, butyl acetate, chloroform, ethanol, isobutanol, isobutyl acetate, isopropanol, methyl acetate, ethyl propionate, n-butanol, n-octanol, propyl acetate, propylene glycol, t-butanol, tetrahydrofuran, and toluene and carrying out the crystallization substantially as described above in Step A of Example 5. Form L eplerenone was formed from each of the solvates substantially as described in Step B of Example 5.

### Example 7: Preparation of Methyl Ethyl Ketone Solvate by Vapor Diffusion Growth

Eplerenone (400 mg; greater than 99.9% purity) was dissolved in 20 mL of methyl ethyl ketone by warming on a hot plate to form a stock solution. An 8 mL amount of the stock solution was transferred to a first 20 mL scintillation vial and diluted to 10 mL with methyl ethyl ketone (80%). A 10 mL amount of the stock solution was transferred to a second 20 mL scintillation vial and diluted to 10 mL with methyl ethyl ketone (40%). The final 2 mL of the stock solution was diluted to 10 mL with methyl ethyl ketone (20%). The four vials containing the dilutions were transferred to a dessicator jar containing a small amount of hexane as an anti-solvent. The dessicator jar was sealed and the hexane vapor allowed to diffuse into the methyl ethyl ketone solutions. Methyl ethyl ketone solvate crystals grew in the 80% dilution sample by the next day.

### Example 8: Preparation of Methyl Ethyl Ketone Solvate by Rotary Evaporation

About 400 mg of eplerenone (greater than 99.9% purity) is weighed into a 250 mL round bottom flask. Solvent (150 mL) is added to the flask and, if necessary, the solution is heated gently until the solid is dissolved. The resulting clear solution is placed on a Buchi rotary evaporator with a bath temperature of about 85°C and the solvent is removed under vacuum. Solvent removal is stopped when approximately 10 mL of solvent remain in the round bottom flask. The resulting solids are analyzed by appropriate method (XPRD, DSC, TGA, microscopy, etc.) for determination of form.

### Example 9: Slurry Conversion

Approximately 150 mg of Form L eplerenone and 150 mg of Form H eplerenone were added to 5 mL of ethyl acetate. The resulting slurry was allowed to stir at 300 rpm (magnetic stirring) overnight. The next day a sample of the solid was collected by filtration. Analysis of the sample by XPRD indicated that the sample was entirely composed of Form L eplerenone.

### Example 10: Preparation of (a) solvate from low purity eplerenone starting material and (b) Form H crystalline eplerenone from resulting solvate

Samples containing varying amounts of the impurity 7-methyl hydrogen 4α,5α:9α,11α-diepoxy-17-hydroxy-3-oxo-17α-pregnane-7α,21-dicarboxylate, γ-lactone (the "diepoxide") or the impurity 7-methyl hydrogen 11α,12α-epoxy-17-hydroxy-3-oxo-17α-pregn-4-ene-7α,21-dicarboxylate, γ-lactone (the "11,12-epoxide") were prepared by adding the desired amount of the impurity to a 7 mL scintillation vial together with an amount of eplerenone sufficient to provide a total sample mass of 100 mg. The weight percent of the diepoxide or 11,12-epoxide in each sample is given in Tables 6A and 6B, respectively. A micro-flea magnetic stirrer was added to each scintillation vial along with 1 mL of methyl ethyl ketone. The vials were loosely capped and the solid dissolved by heating to reflux on a hot plate with magnetic stirring. Once the solids were dissolved, the solutions were allowed to cool to room temperature on the hot plate. Magnetic stirring was maintained during the cooling period. After the solutions reached room temperature, the solids were collected by vacuum filtration and immediately analyzed by X-ray powder diffraction (XPRD). The solids were then placed in a 100°C oven and dried for one hour at ambient pressure. The dried solids were analyzed by XPRD for Form H content by monitoring the area of the Form H diffraction peak at about 12.1 degrees two theta. All XPRD diffraction patterns were recorded using an Inel Multipurpose Diffractometer.

**TABLE 6A**

| **Weight Percent Diepoxide** | **Weight Eplerenone (mg)** | **Weight Diepoxide (mg)** |
|---|---|---|
| 0% | 100.44 | -- |
| 1% | 99.08 | 1.24 |
| 2% | 98.09 | 2.24 |
| 3% | 97.08 | 3.04 |
| 5% | 95.09 | 5.04 |

**TABLE 6B**

| **Weight Percent 11,12-Epoxide** | **Weight Eplerenone (mg)** | **Weight 11,12-Epoxide (mg)** |
|---|---|---|
| 0% | 101.38 | 0 |
| 1% | 99.23 | 1.10 |
| 5% | 94.97 | 5.36 |
| 10% | 90.13 | 10.86 |

### A. Diepoxide Results

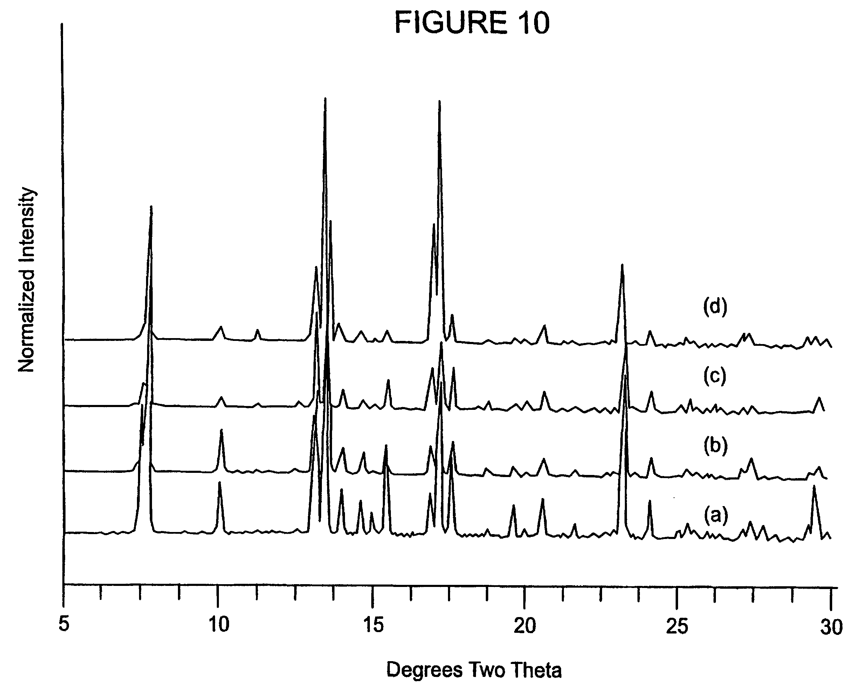

Fig. 10 shows the X-ray powder diffraction patterns for the wet cake (methyl ethyl ketone solvate) obtained from the (a) 0%, (b) 1%, (c) 3%, and (d) 5% diepoxide-doped methyl ethyl ketone crystallizations. The peak intensities have been normalized for ease of comparison. No peaks characteristic of Form H or the diepoxide are present in the diffraction patterns. The patterns are characteristic of the methyl ethyl ketone solvate of eplerenone.

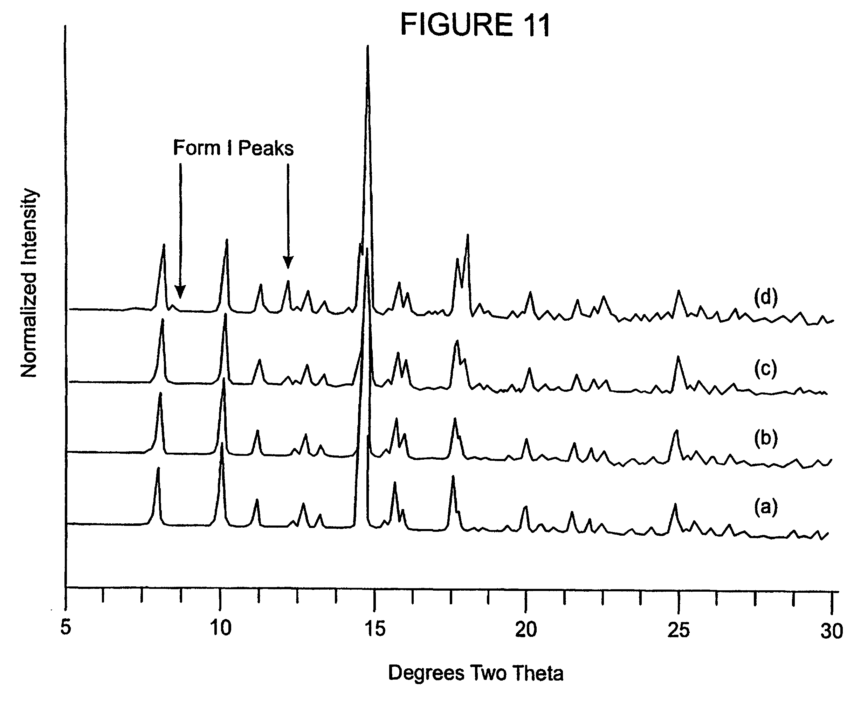

Fig. 11 shows the X-ray powder diffraction patterns for the dried solids obtained from the (a) 0%, (b) 1%, (c) 3%, and (d) 5% diepoxide-doped methyl ethyl ketone crystallizations. The peak intensities have been normalized for ease of comparison. No Form H was detected for the dried samples corresponding to the methyl ethyl ketone crystallizations performed at doping levels of 0 and 1%. Form H was detected in the dried samples corresponding to the methyl ethyl ketone crystallizations performed at doping levels of 3 and 5%. The area for the Form H diffraction peak at about 12.1 degrees two theta and the estimated Form H content for each sample are given in Table 6C below.

**TABLE 6C**

| **Weight Percent of Diepoxide in Starting Eplerenone Mixture** | **Weight Percent of Diepoxide in Resulting Crystals (HPLC)** | **Form H Peak Area 12° Two Theta Peak** | **Estimated Weight Percent of Form H** |
|---|---|---|---|
| 0% | --- | None Detected | None Detected |
| 1% | 0.29% | None Detected | None Detected |
| 3% | 0.58% | 1168 | 10% |
| 5% | 1.05% | 4175 | 30% |

The results reported in Table 6C confirm that the presence of the diepoxide affects the formation of Form H during the desolvation. These results indicate that the diepoxide is effective in inducing the formation of Form H eplerenone when it is incorporated into and/or adsorbed onto the methyl ethyl ketone solvate crystals.

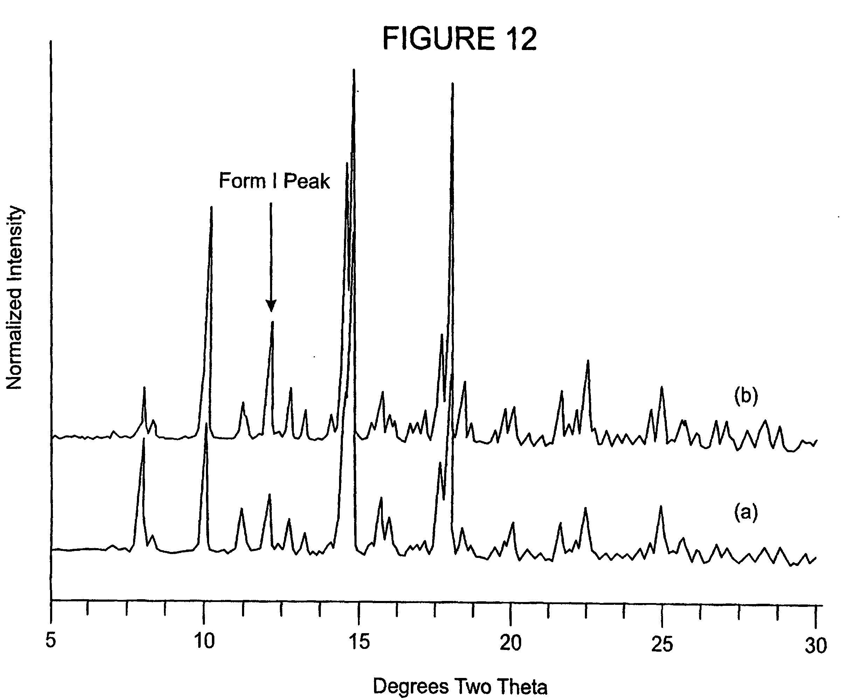

The 3% diepoxide doping experiment was repeated to analyze the impact of the route of preparation on the amount of Form H formed during the desolvation. In this experiment, the methyl ethyl ketone solvate obtained from the doped crystallization was divided into two portions. The first portion was left untreated while the second portion was lightly ground in a mortar and pestle to induce a higher level of crystal defects. The two portions were both dried at 100 °C for one hour at ambient pressure. The dried solids were analyzed by XPRD. The XPRD patterns are given in Fig. 12 for the dried solids from the methyl ethyl ketone crystallization with 3% doping of diepoxide (a) without grinding of the solvate prior to drying, and (b) with grinding of the solvate prior to drying. The XPRD patterns indicated a greater amount of Form H in the ground sample relative to the unground sample. These results suggest that the conditions under which the methyl ethyl ketone solvate is isolated and handled can affect the crystal form that results from the desolvation.

### B. 11,12-Epoxide Results

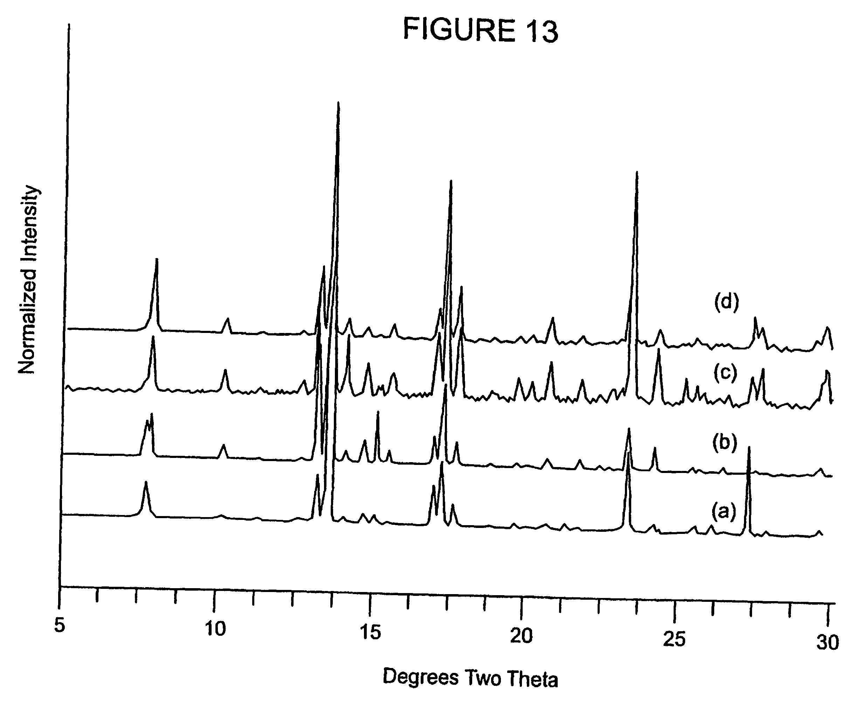

Fig. 13 shows the X-ray powder diffraction patterns for the wet cake (methyl ethyl ketone solvate) obtained from the (a) 0%, (b) 1%, (c) 5%, and (d) 10% 11,12-epoxide-doped methyl ethyl ketone crystallizations. The peak intensities have been normalized for ease of comparison. No peaks characteristic of Form H or the 11,12-epoxide are present in the diffraction patterns. The patterns are characteristic of the methyl ethyl ketone solvate of eplerenone.

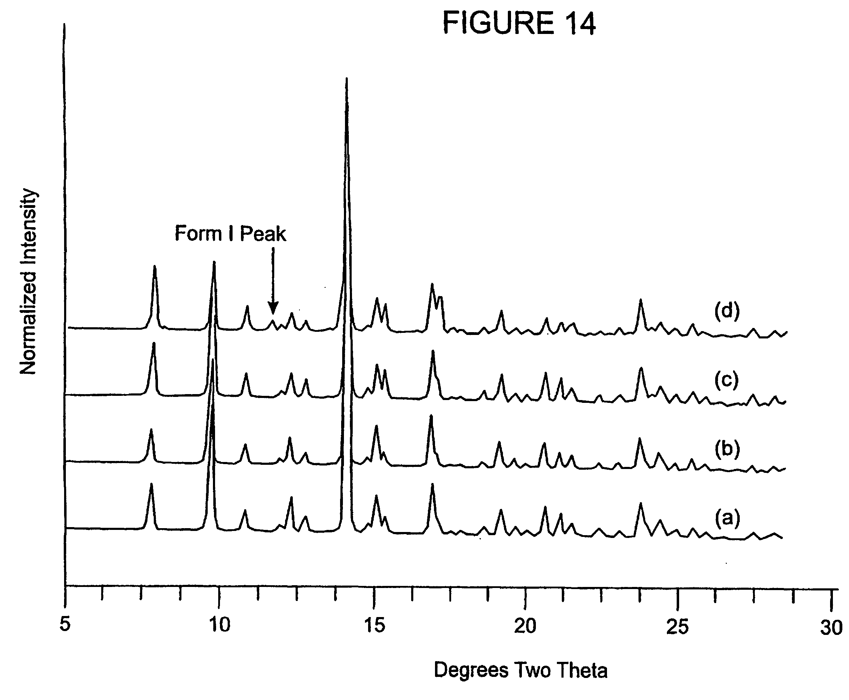

Fig. 14 shows the X-ray powder diffraction patterns for the dried solids obtained from the (a) 0%, (b) 1%, (c) 5%, and (d) 10% 11,12-epoxide-doped methyl ethyl ketone crystallizations. The peak intensities have been normalized for ease of comparison. No Form H was detected for the dried samples corresponding to the methyl ethyl ketone crystallizations performed at doping levels of 0, 1% and 5%. Form H was detected in the dried samples corresponding to the methyl ethyl ketone crystallization performed at a doping level of 10%. The area for the Form H diffraction peak at 12.1 degrees two theta and estimated Form H content for each sample are given in Table 6D.

**TABLE 6D**

| **Weight Percent 11,12-Epoxide in Starting Eplerenone Mixture** | **Weight Percent 11,12-Epoxide in Resulting Crystals (HPLC)** | **Form H Peak Area 12° Two Theta Peak** | **Estimated Weight Percent of Form H** |
|---|---|---|---|
| 0% | Not Available | None Detected | None Detected |
| 1% | Not Available | None Detected | None Detected |
| 5% | Not Available | None Detected | None Detected |
| 10% | Not Available | 1541 | 10-15% |

The results reported in Table 6D confirm that the presence of the 11,12-epoxide impacts the formation of Form H during the desolvation. The percentage of impurity in the methyl ethyl ketone crystallization required to induce the formation of Form H eplerepone appears to be greater for the 11,12-epoxide than for the diepoxide.

### Example 11: Effect of Crystallization and Drying on Final Crystal Form

The following four experiments analyzing the effect of crystallization and drying on the final crystal form were conducted: (i) methyl ethyl ketone crystallization of eplerenone (2³+3 statistical design of experiment), (ii) crystallization of poor quality mother liquor residue, (iii) crystallization of high purity eplerenone with Form H seeding, and (iv) crystallization of low purity eplerenone with Form L seeding. Variables in the design of the experiments included cooling rate, starting material purity level, and end point temperature of crystallization. For purposes of this Example, high purity eplerenone was defined as ultra-pure milled eplerenone (HPLC analysis showed this material to be 100.8% pure) and low purity eplerenone was defined as 89% pure eplerenone. To prepare the low purity eplerenone, stripped-down mother liquors from the process for the preparation of eplerenone were analyzed and blended to yield a material that was 61.1% eplerenone, 12.8% diepoxide and 7.6% 11,12-epoxide. This material was then blended with a sufficient amount of high purity eplerenone to yield the 89% eplerenone.

### A. Methyl Ethyl Ketone Crystallization

In the methyl ethyl ketone crystallization experiment, all runs were performed using 60 g of high purity eplerenone. High endpoint was defined as 45°C and low endpoint was defined as 5°C. High cooling rate was defined as 3°C/minute cooling and low cooling rate was defined as 0.1°C/minute cooling. Center points were 1.5° C/minute cooling, 94.5% pure eplerenone, and a 25°C endpoint.

After a background reading was taken with the FTIR, 250 mL of methyl ethyl ketone was charged to a 1L Mettler RC-1, MP10 reactor and stirred at 100 rpm. After several scans, eplerenone was charged to the reactor followed by an additional 470 mL of methyl ethyl ketone. Agitation was increased to 500 rpm to suspend solids and the batch temperature was increased to 80°C. The batch temperature was held at 80°C to ensure dissolution of the eplerenone. Black or white specks generally were visible in the resulting transparent solution. The batch temperature was then ramp cooled at the desired rate to the desired endpoint, where it was maintained for one hour before being pulled into a transfer flask and filtered. The vacuum was reactor, transfer flask and cake were then washed with 120 mL methyl ethyl ketone. Once the wash was pulled through the cake, the stopped. About 10 grams of each wet cake were dried in a vacuum oven under nominal conditions of 75°C with a light nitrogen bleed. For the "high, high, high" and "low, low, low" experiments described below, fluid bed drying was operated under high and low conditions. High fluid bed drying was defined as 100°C with a blower setting of "4" while low fluid bed drying was defined as 40°C with a blower setting of "1".

### B. Crystallization of Poor Quality Mother Liquor Residue

In the crystallization of poor quality mother liquor residue experiment, 60 g of the 61.1% pure material and 720 mL methyl ethyl ketone were charged directly to a 1L Mettler RC-1, MP10 reactor. The 61.1% pure material was not blended with high purity eplerenone prior to being charged to the reactor. The resulting mixture was heated to 80°C and was an opaque slurry at that temperature. The crystallization continued and the mixture was filtered at 45°C under fast cooling conditions.

### C. Form H Seeding

In the Form H seeding experiment, 60 g of pure (100.8%) eplerenone and 720 mL of methyl ethyl ketone were charged to a 1L Mettler RC-1, MP10 reactor. The mixture was heated to 80°C and then cooled to 25°C at a rate of 1.5°C/minute. When the solution had cooled to 62 °C, it was seeded with 3 g of phase pure Form H crystals to initiate crystallization. The Form H seed crystals were prepared by the digestion process described in Example 13 below.

### D. Form L Seeding

In the Form L seeding experiment, 66.6 g of 89.3% eplerenone (prepared by mixing 48.3 g of 100% eplerenone with 18.3 g of 61.1% eplerenone) and 720 mL of methyl ethyl ketone were charged to a 1L Mettler RC-1, MP10 reactor. The mixture was heated to 80°C and then cooled to 25°C at a rate of 1.5°C/minute. When the solution had cooled to 63°C, it was seeded with 3 g of phase pure Form L crystals to initiate crystallization. The Form L seed crystals were prepared by the crystallization and desolvation process described in Example 5 above.

Results from the experiments are reported in Table 7A. In the n+1 crystallization experiment, Form H was detected only in the experiments employing low purity eplerenone where the product contained the diepoxide. Elevated levels of the diepoxide in the final product were also observed with higher cooling rates.

The crystallization of poor quality mother liquor residue experiment yielded poor quality material that appeared to be a mixture of the diepoxide and Form H when analyzed by X-ray powder diffraction.

The Form H seeding experiment (where high purity eplerenone was seeded with Form H) yielded a product that was 77% Form H based on X-ray powder diffraction analysis, but entirely Form H based on DSC. The X-ray powder diffraction model, however, had not been tested for linearity beyond about 15% Form H. This experiment was the only one of the four experiments of this Example where Form H was created in the absence of the diepoxide.

The Form L seeding experiment (where low purity eplerenone was seeded with Form L) yielded a product that was entirely Form L.

The data obtained for the high fluid bed drying of eplerenone appeared to correspond to the data obtained for the vacuum oven drying. The low fluid bed dryings yielded results that differed from those of the vacuum oven dryings.

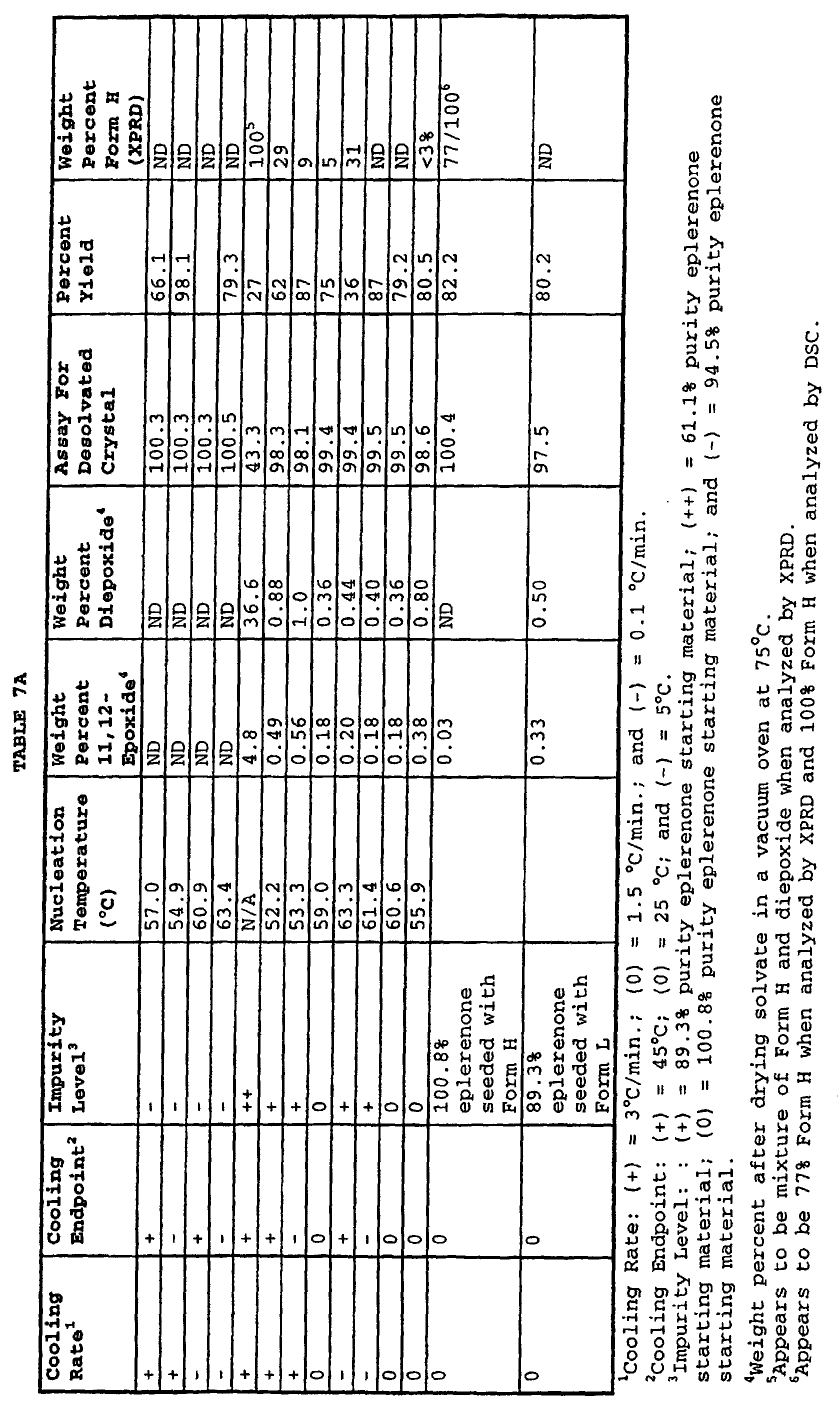

### A. Material Purity

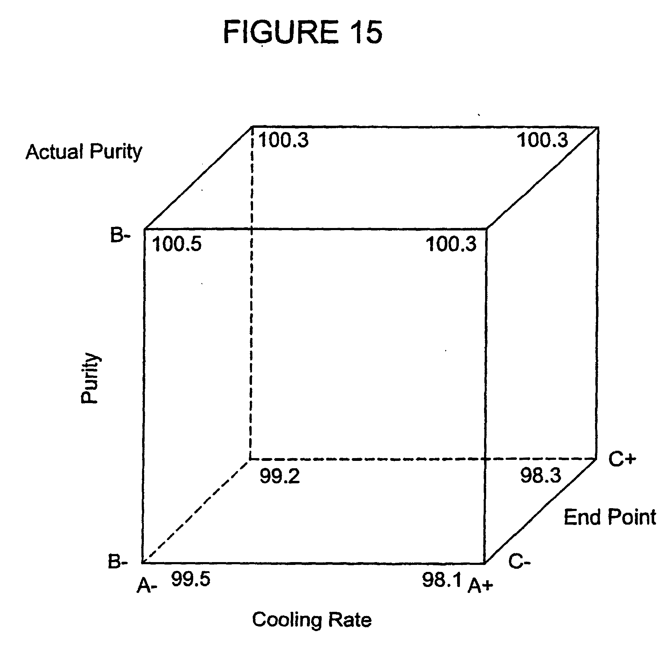

A cube plot of product purity, starting material purity, cooling rate and endpoint temperature based on the data reported in Table 7A is shown in Fig. 15. The cube plot suggests that the use of a higher purity material at the start of crystallization will yield a higher purity product. The endpoint temperature of crystallization does not appear to greatly affect the product purity. The cooling rate, however, appears to have an effect with slightly less pure product resulting from a faster cooling rate. In fact, the level of diepoxide generally was higher with faster cooling rates.

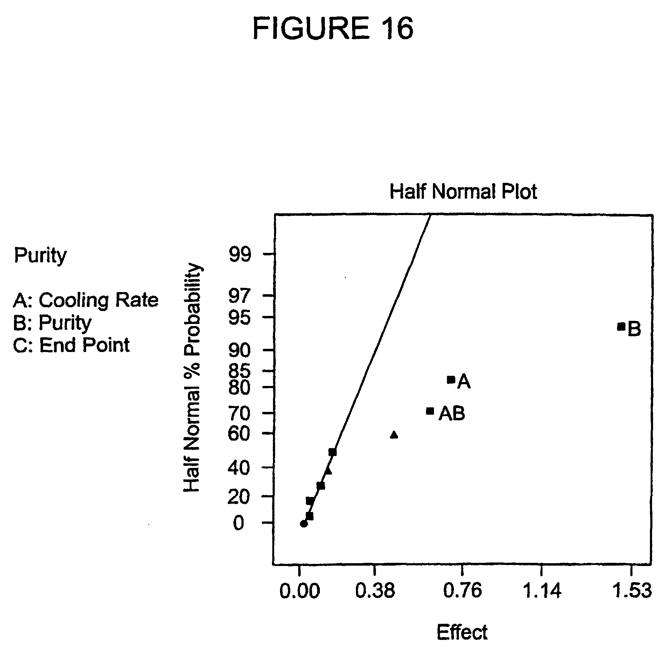

Fig. 16 shows a half normal plot that was prepared using the results of cube plot to determine which variables, if any, had a statistically significant effect on the product purity. Starting material purity had the greatest statistically significant effect on product purity, although cooling rate and the interaction between cooling rate and starting material purity were also seen as statistically significant effects.

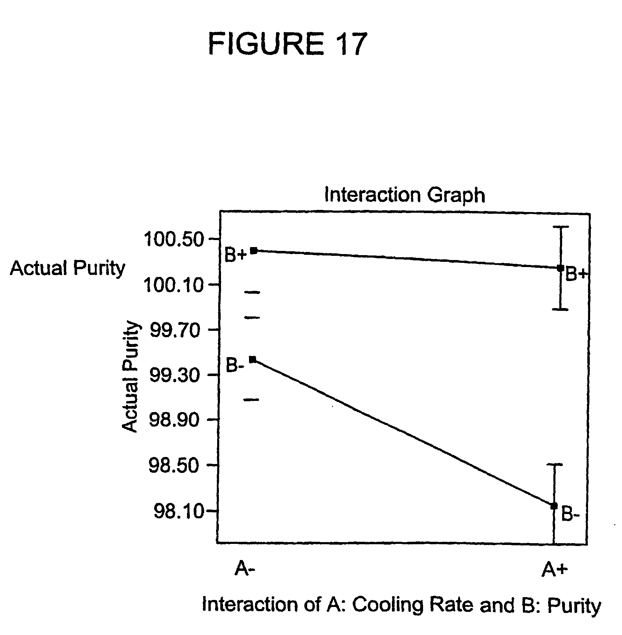

Fig. 17 is an interaction graph based on these results and showing the interaction between starting material purity and cooling rate on product purity. With the high purity eplerenone (100.8% eplerenone starting material) the cooling rate appears to have little or no effect on final purity. With the low purity eplerenone (89.3% eplerenone starting material), however, the product purity decreases as cooling rate increases. This result suggests that more impurities crystallize out in eplerenone crystallizations conducted at higher cooling rates.

### B. Form H Content

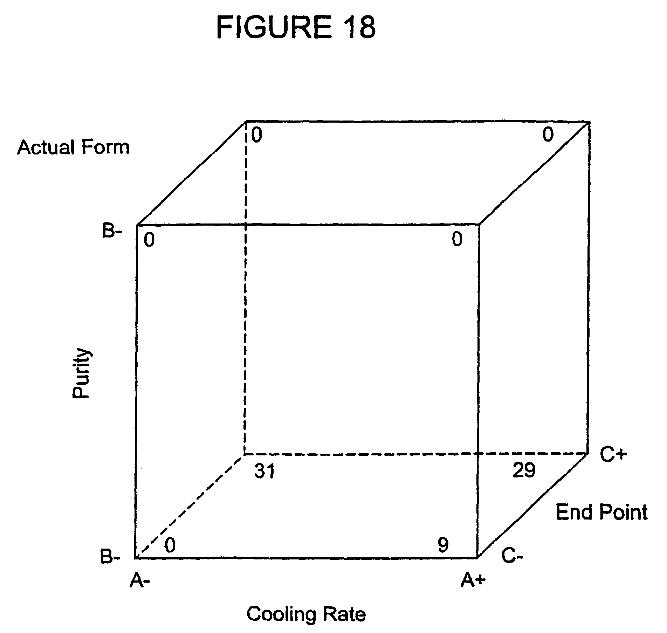

A cube plot of Form H weight fraction, starting material product purity, cooling rate and endpoint temperature based on the data reported in Table 7A is shown in Fig. 18. The cube plot suggests that the use of a higher purity eplerenone at the start of crystallization will yield a lower amount of Form H. The endpoint temperature of crystallization also appears to have an effect on the form of the final product. The cooling rate does not appear to greatly affect the formation of Form H although some Form H may result from faster cooling at the low endpoint temperature in the presence of impurities.

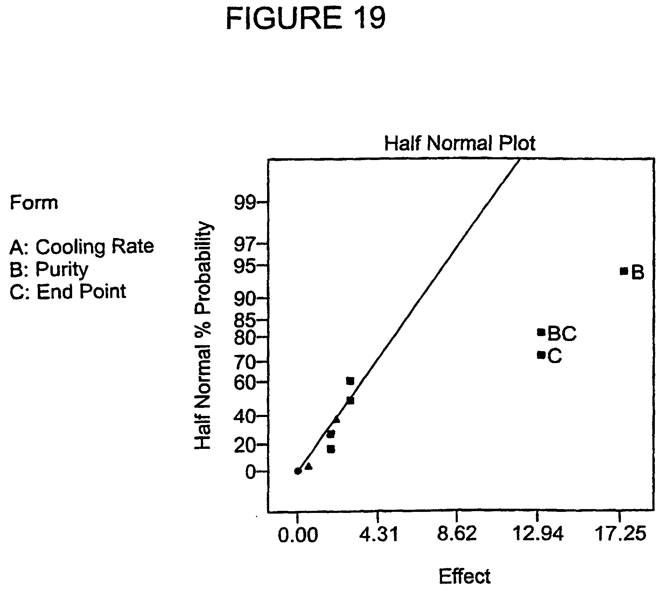

Fig. 19 shows a half normal plot that was prepared using the results of the cube plot to determine which variables, if any, had a statistically significant effect on the amount of Form H in the final material. Starting material purity, endpoint temperature of the crystallization and the interaction between the two variables were seen as statistically significant effects.

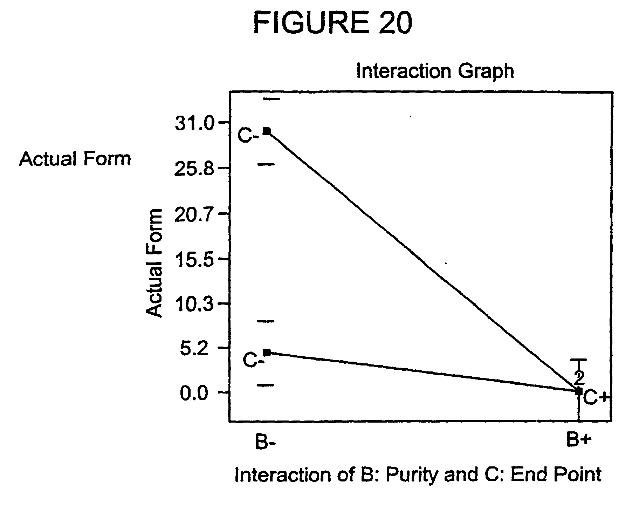

Fig. 20 is an interaction graph based on these results and showing the interaction between starting material purity and endpoint temperature on final Form H content. With the high purity eplerenone (100.8% eplerenone starting material), endpoint temperature appears to have little effect on Form H content. No Form H resulted in either case with pure eplerenone. With low purity eplerenone (89.3% eplerenone starting material), however, Form H was present in both cases, with significantly more Form H at higher endpoint temperatures.

Table 7B reports the weight fraction of Form H measured in materials dried using either a fluid bed (LAB-LINE/P.R.L. Hi-Speed Fluid Bed Dryer, Lab-Line Instruments, Inc.) or a vacuum oven (Baxter Scientific Products Vacuum Drying Oven, Model DP-32). Similar Form H content was observed for comparable materials dried in either the high fluid bed or the vacuum oven. A difference was observed, however, for comparable materials dried in the low fluid bed relative to the vacuum oven.

**TABLE 7B**

| **Cooling Rate** | **End Point** | **Impurity Level** | **Drying Type** | **Weight Percent Form H** |
|---|---|---|---|---|
| High | High | High | Vacuum Oven | 29% |
| High | High | High | High Fluid Bed | 25% |
| High | High | High | Low Fluid Bed | 4.7% |
| Low | Low | Low | Vacuum Oven | ND |
| Low | Low | Low | High Fluid Bed | ND |
| Low | Low | Low | Low Fluid Bed | 5.5% |

### Example 12: Crystallization of a Mixture of Form H and Form L From Methyl Ethyl Ketone To Prepare a Solvate, and (b) Desolvation of the Solvate to Prepare Form L

Form H eplerenone (10 g) was combined with 80 mL of methyl ethyl ketone. The mixture was heated to reflux (79°C) and stirred at this temperature for about 30 minutes. The resulting slurry was then cooled with a stepwise, holdpoint protocol by maintaining the slurry at 65°C, 50°C, 35°C and 25°C for about 90 minutes at each temperature. The slurry was filtered and rinsed with about 20 mL methyl ethyl ketone. The isolated solid was initially dried on the filter and then in a vacuum oven at 40-50°C. The drying was completed in the vacuum oven at 90-100°C. The desolvated solid was obtained with an 82% recovery. XPRD, MIR and DSC confirmed that the solid had a Form L crystalline structure.

### Example 13: Digestion of Low Purity Eplerenone Starting Material With a Solvent to Prepare Form H

### A. Digestion With Ethanol Solvent:

Low purity eplerenone (24.6 g; 64% by weight assay via HPLC) was combined with 126 mL of ethanol 3A. The slurry was heated to reflux and the distillate removed. An additional 126 mL of ethanol 3A was simultaneously added as 126 ml of solvent was removed via atmospheric distillation. Upon completion of the solvent turnover, the mixture was cooled to 25 °C and stirred for one hour. The solid was filtered and rinsed with ethanol 3A. The solid was air-dried to give the ethanol solvate. The solvate was further dried in a vacuum oven at 90-100°C for six hours to obtain 14.9 g of Form H eplerenone.

### B. Digestion With Methyl Ethyl Ketone Solvent

In an alternative digestion process, 1 gram of low purity eplerenone (about 65% pure) was digested in 4 mL of methyl ethyl ketone for two hours.
After the two hours, the mixture was allowed to cool to room temperature. Once cooled, the solid was collected by vacuum filtration and determined to be the methyl ethyl ketone solvate by XPRD analysis. The solid was dried at 100°C for 30 to 60 minutes. The dried solids were determined to be pure Form H by XPRD.

### Example 14: Digestion of High Purity Eplerenone Starting Material With a Solvent to Prepare Form L

### A. Digestion With Ethanol Solvent:

High purity eplerenone (1 gram) was digested in 8 mL of ethanol for approximately two hours. The solution was then allowed to cool to room temperature and the solids were collected by vacuum filtration. Analysis of the solids by XPRD immediately after filtration indicated that the solids were a solvate (presumably the ethanol solvate). The solids were subsequently dried at 100°C at atmospheric pressure for 30 minutes. The dried solid was analyzed by XPRD and determined to be predominately Form L (no Form H detected).

### B. Digestion with Methyl Ethyl Ketone Solvent:

High purity eplerenone (1 gram) was digested in 4 mL of methyl ethyl ketone for two hours. After the two hours, the solution was allowed to cool to room temperature and the solids collected by vacuum filtration. The solid was immediately analyzed by XPRD and determined to be a solvate of eplerenone (presumably the methyl ethyl ketone solvate). The solvate was subsequently dried at 100°C at ambient pressure for 30 to 60 minutes. The dried solids were analyzed by XPRD and determined to be primarily Form L with no diffraction peaks for Form H present.

### Example 15: Crystallization of Form L Directly From Solution

Procedure A: Eplerenone (2.5 g) was dissolved in ethyl acetate by heating to 75°C. Once the eplerenone dissolved, the solution was held at 75°C for 30 minutes to ensure complete dissolution. The solution was then cooled at 1 °C/min to 13°C. Once at 13°C, the slurry was allowed to stir for two hours at 750 rpm with an overhead stirrer. The crystals were collected by vacuum filtration and dried in a vacuum oven at 40°C for one hour. The XPRD pattern and DSC thermogram of the solid were characteristic of Form L eplerenone. Thermal gravimetric analysis (TGA) of the solid indicated no weight loss from the solid up to 200°C.

Procedure B: In an alternative procedure, 2 g of eplerenone was dissolved in 350 mL of 15/85% acetonitrile/water by heating on a hot plate with magnetic stirring. Once the eplerenone was dissolved, the solution was allowed to cool to room temperature overnight with magnetic stirring. The resulting solid was collected by vacuum filtration. The crystals were birefringent and had a triangular, plate-like crystal habit. The solid had an XPRD and DSC characteristic of Form L eplerenone. TGA indicated no weight loss up to

### 200°C.

Procedure C: In an alternative procedure, 640 mg of eplerenone was placed in a 50 mL flask with 20 mL of ethyl benzene. The resulting slurry was heated to 116° C and became a clear solution. The clear solution was cooled to 25°C over 30 minutes. Nucleation began at 84 °C during the cooling period. The resulting solids were filtered from the solution and air-dried to give 530 mg of solids (83% recovery). Hot-stage microscopy and XPRD confirmed that the solids were Form L crystals.

Procedure D: In an alternative procedure, 1.55 g of eplerenone was added to 2.0 mL of nitrobenzene and heated to 200°C. The resulting slurry was stirred overnight at 200°C. The solution was allowed to cool to room temperature (natural air convection) the following day and the solid was isolated. The solid was determined to be Form L eplerenone by XPRD and polarized light microscopy.

Procedure E: In an alternative procedure, 5.0 g of eplerenone (purity greater than 99%) was added to 82 g of methanol (104 mL). Under stirring action (210 rpm), the solution was heated to 60°C and held at that temperature for 20 minutes to ensure complete dissolution. The solution was then cooled to -5°C at a rate of 0.16°C/minute under stirring. The crystals were collected by filtration and dried in a vacuum oven at 40°C for 20 hours. The dried solids were determined to be pure Form L eplerenone by DSC and XPRD analysis.

Procedure F: In an alternative procedure, 6.0 g of eplerenone (ethanol solvate containing 9% ethanol and having a corrected purity of 95.2%) was added to 82 g of methanol (104 mL). Under stirring action (210 rpm), the solution was heated to 60°C and held at that temperature for 20 minutes to ensure complete dissolution. The solution was then cooled to 50°C at a rate of 0.14°C/minute and then held at that temperature for about 2.5 hours. The solution was then cooled to -5°C at a rate of 0.13°C/minute under stirring. The crystals were collected by filtration and dried in a vacuum oven at 40°C for 16 hours. The dried solids were determined to be pure Form L eplerenone by DSC and XPRD analysis.

### Example 16: Crystallization of Form H Directly From Solution

150.5 mg of the diepoxide and 2.85 g of eplerenone were added to 1.5 mL of nitrobenzene. The mixture was magnetically stirred at 200°C for several hours. The slurry was then allowed to cool to room temperature by natural air convection. The sample was dried and analyzed by polarized light microscopy and XPRD. The XPRD indicated that the sample was a mixture of Form H and Form L. The crystals were translucent by microscopy, indicating that desolvation (and conversion to either Form H or Form L) did not occur.

### Example 17: Preparation of Amorphous Eplerenone By Comminution

Approximately one-half of a steel Wig-L-Bug container was filled with about 60 g of eplerenone (greater than 99.9% purity). A steel ball and cap were placed on the sample container and agitated for 30 seconds by the Wig-L-Bug apparatus. The eplerenone was scraped off the surface of the Wig-L-Bug container and the container agitated for an additional 30 seconds. The resulting solid was analyzed by XPRD and DSC and determined to be a mixture of amorphous eplerenone and Form L crystalline eplerenone.

### Example 18: Preparation of Amorphous By Lyophilization

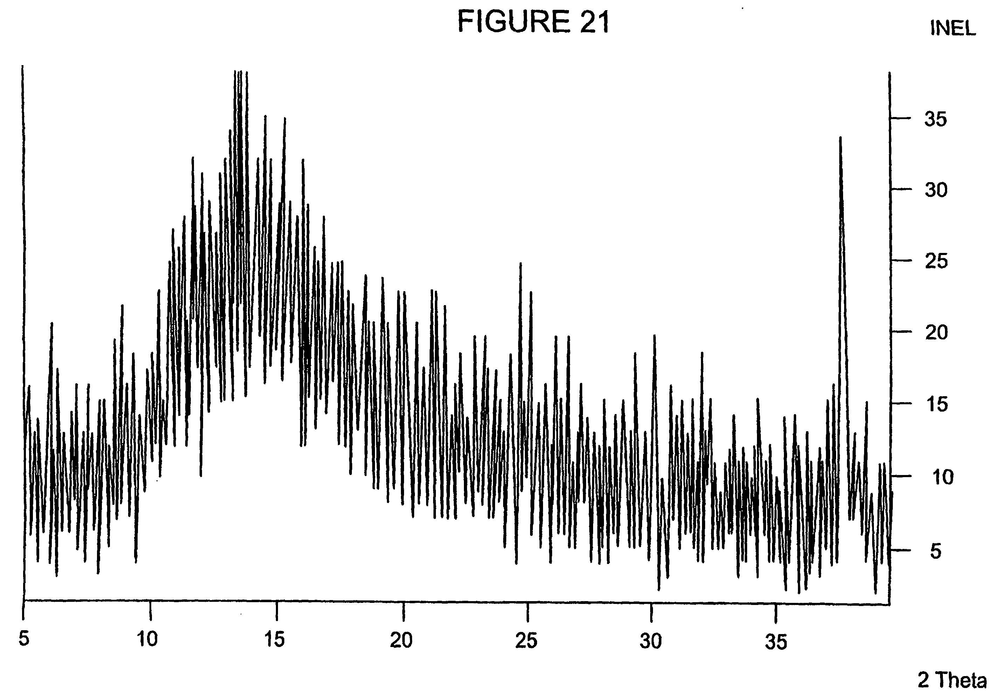

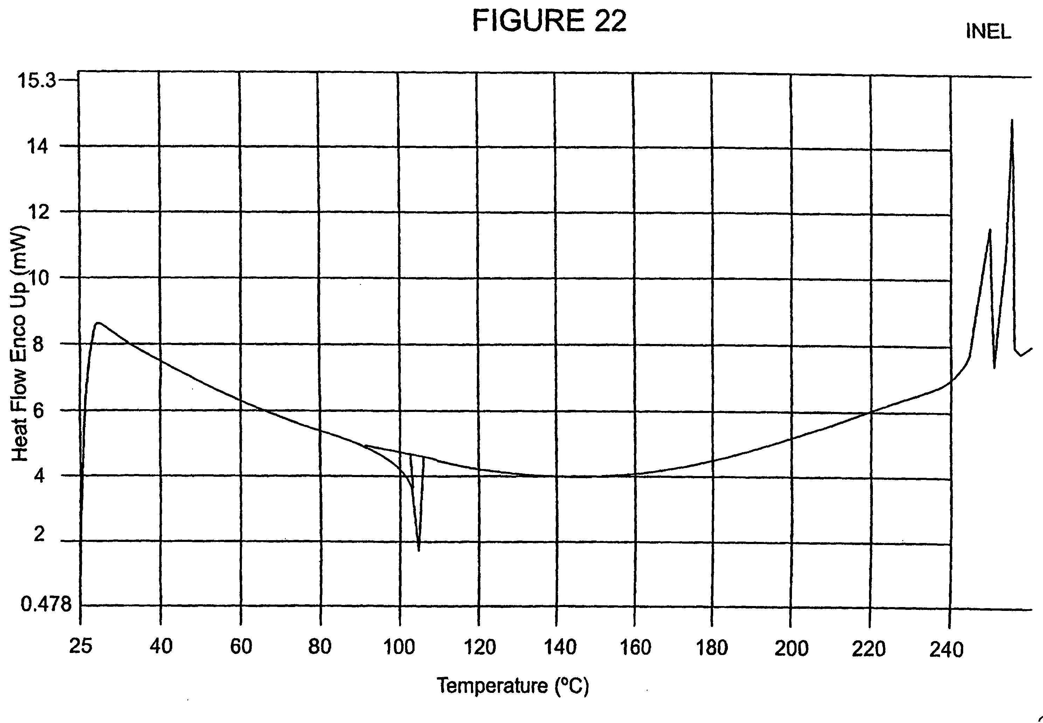

Approximately 100 mg of crude eplerenone was weighed into a beaker containing 400 mL of water. The solution was heated slightly for five minutes, and then sonicated and heated with stirring for an additional five minutes. Approximately 350 mL of the eplerenone solution was filtered into a 1000 mL round bottom flask containing 50 mL of HPLC water. The solution was flashed frozen in a dry ice/acetone bath over a time period of one to two minutes. The flask was attached to a Labconco Freezone 4.5 freeze dryer and dried overnight. The solids in the flask were transferred to a small brown bottle. A small aliquot was observed under polarized light microscopy at 10X, 1.25X optivar in cargille oil (1.404) and observed to be at least 95% amorphous eplerenone. Figures 21 and 22 show the XPRD pattern and DSC thermogram obtained for the amorphous eplerenone. The peak observed at 39 degrees two theta in Figure 21 is attributable to the aluminum sample container.

### II.

### Example 19: Eplerenone Polymorph Composition

Tablets containing 25 mg, 50 mg, 100 mg and 200 mg doses of Form L eplerenone are prepared and have the following composition:

| **Ingredient** | **Weight % of Tablet** |
|---|---|
| Form L Eplerenone | 29.41 |
| Form H Eplerenone | Not Detected |
| Lactose Monohydrate (#310, NF) | 42.00 |
| Microcrystalline Cellulose (NF, Avicel PH101) | 18.09 |
| Croscarmellose Sodium (NF, Ac-Di-Sol) | 5.00 |
| Hydroxypropyl Methylcellulose (#2910, USP, Pharmacoat 603) | 3.00 |
| Sodium Lauryl Sulfate (NF) | 1.00 |
| Talc (USP) | 1.00 |
| Magnesium Stearate (NF) | 0.5 |
| Total | 100.00 |

### Example 20: Eplerenone Polymorph Composition

Capsules (hard gelatin capsule, #0) are prepared containing a 100 mg dose of eplerenone and have the following composition:

| **Ingredient** | **Amount (mg)** |
|---|---|
| Form L Eplerenone | 90.0 |
| Form H Eplerenone | 10.0 |
| Lactose, Hydrous, NF | 231.4 |
| Microcrystalline Cellulose, NF | 45.4 |
| Talc, USP | 10.0 |
| Croscarmellose Sodium, NF | 8.0 |
| Sodium Lauryl Sulfate, NF | 2.0 |
| Colloidal Silicon Dioxide, NF | 2.0 |
| Magnesium Stearate, NF | 1.2 |
| Total Capsule Fill Weight | 400.0 |

### Example 21: Eplerenone Polymorph Composition

Capsules (hard gelatin capsule, size #0) are prepared containing a 200 mg dose of eplerenone and have the following composition:

| **Ingredient** | **Amount (mg)** |
|---|---|
| Form L Eplerenone | 190.0 |
| Form H Eplerenone | 10.0 |
| Lactose, Hydrous, NF | 147.8 |
| Microcrystalline Cellulose, NF | 29.0 |
| Talc, USP | 10.0 |
| Croscarmellose Sodium, NF | 8.0 |
| Sodium Lauryl Sulfate, NF | 2.0 |
| Colloidal Silicon Dioxide, NF | 2.0 |
| Magnesium Stearate, NF | 1.2 |
| Total Capsule Fill Weight | 400.0 |

### Example 22: Preparation of Milled Eplerenone

Dried methyl ethyl ketone solvate is first delumped by passing the solvate through a 20 mesh screen on a Fitzmill. The delumped solid is then pin milled using an Alpine Hosakawa stud disk pin mill operating under liquid nitrogen cooling at a feed rate of approximately 250 kilograms/hour. Pin milling produces milled eplerenone with a D₉₀ size of approximately 65-100 microns.

The following examples illustrate aspects of the present invention but should not be construed as limitations. The experimental procedures used to generate the data shown are discussed in more detail below. The symbols and conventions used in these examples are consistent with those used in the contemporary pharmacological literature. Unless otherwise stated, (i) all percentages recited in these examples are weight percents based on total composition weight, (ii) total composition weight for capsules is the total capsule fill weight and does not include the weight of the actual capsule employed, and (iii) coated tablets are coated with a conventional coating material such as Opadry White YS-1-18027A and the weight fraction of the coating is about 3% of the total weight of the coated tablet.

### Example 23: 25 Mg Dose Immediate Release Tablet

A 25 mg dose immediate release tablet (tablet diameter of 7/32") was prepared having the following composition:

**Table 8**

| **INGREDIENT** | **WEIGHT % OF TABLET** | **Amount (mg)** |
|---|---|---|
| Eplerenone | 29.41 | 25.00 |
| Lactose Monohydrate (#310, NF) | 42.00 | 35.70 |
| Microcrystalline Cellulose (NF, Avicel PH101) | 18.09 (7.50% intragranular plus 10.59% extragranular) | 15.38 |
| Croscarmellose Sodium (NF, Ac-Di-Sol) | 5.00 | 4.25 |
| Hydroxypropyl Methylcellulose (#2910, USP, Pharmacoat 603) | 3.00 | 2.55 |
| Sodium Lauryl Sulfate (NF) | 1.00 | 0.85 |
| Talc (USP) | 1.00 | 0.85 |
| Magnesium Stearate (NF) | 0.50 | 0.42 |
| Total | 100 | 85 |
| Opadry White YS-1-18027A | 3.00 | 2.55 |

### Example 24: 50 Mg Dose Immediate Release Tablet

A 50 mg dose immediate release tablet (tablet diameter of 9/32") was prepared having the following composition:

**Table 9**

| **INGREDIENT** | **WEIGHT % OF TABLET** | **Amount (mg)** |
|---|---|---|
| Eplerenone | 29.41 | 50.00 |
| Lactose Monohydrate (#310, NF) | 42.00 | 71.40 |
| Microcrystalline Cellulose (NF, Avicel PH101) | 18.09 (7.50% intragranular plus 10.59% extragranular) | 30.75 |
| Croscarmellose Sodium (NF, Ac-Di-Sol) | 5.00 | 8.50 |
| Hydroxypropyl Methylcellulose (#2910, USP, Pharmacoat 603) | 3.00 | 5.10 |
| Sodium Lauryl Sulfate (NF) | 1.00 | 1.70 |
| Talc (USP) | 1.00 | 1.70 |
| Magnesium Stearate (NF) | 0.50 | 0.85 |
| Total | 100 | 170 |
| | | |
| Opadry White YS-1-18027A | 3.00 | 5.10 |

### Example 25: 100 Mg Dose Immediate Release Tablet

A 100 mg dose immediate release tablet formulation (tablet diameter of 12/32") was prepared having the following composition:

**TABLE 9A**

| **INGREDIENT** | **WEIGHT % OF TABLET** | **Amount (mg)** |
|---|---|---|
| Eplerenone | 29.41 | 100.00 |
| Lactose Monohydrate (#310, NF) | 42.00 | 142.80 |
| Microcrystalline Cellulose (NF, Avicel PH101) | 18.09 (7.50% intragranular plus 10.59% extragranular) | 61.50 |
| Croscarmellose Sodium (NF, Ac-Di-Sol) | 5.00 | 17.00 |
| Hydroxypropyl Methylcellulose (#2910, USP, Pharmacoat 603) | 3.00 | 10.20 |
| Sodium Lauryl Sulfate (NF) | 1.00 | 3.40 |
| Talc (USP) | 1.00 | 3.40 |
| Magnesium Stearate (NF) | 0.50 | 1.70 |
| Total | 100 | 340 |
| | | |
| Opadry White YS-1-18027A | 3.00 | 10.20 |

### Example 26: 10 mg Dose Immediate Release Capsule

A 10 mg dose immediate release capsule formulation was prepared having the following composition:

**Table 10**

| **INGREDIENT** | **AMOUNT (mg)** | **REPRESENTATIVE BATCH AMOUNT (kg)** |
|---|---|---|
| Eplerenone | 10.0 | 1.00 |
| Lactose, Hydrous NF | 306.8 | 30.68 |
| Microcrystalline Cellulose, NF | 60.0 | 6.00 |
| Talc, USP | 10.0 | 1.00 |
| Croscarmellose Sodium, NF | 8.0 | 0.80 |
| Sodium Lauryl Sulfate, NF | 2.0 | 0.20 |
| Colloidal Silicon Dioxide, NF | 2.0 | 0.20 |
| Magnesium Stearate, NF | 1.2 | 0.12 |
| Total Capsule Fill Weight | 400.0 | 40.00 |
| Hard Gelatin Capsule, Size #0, White Opaque | 1 Capsule | 100,000 Capsules |

### Example 27: 25 mg Dose Immediate Release Capsule

A 25 mg dose immediate release capsule formulation was prepared having the following composition:

**Table 11**

| **INGREDIENT** | **AMOUNT (mg)** | **REPRESENTATIVE BATCH AMOUNT (kg)** |
|---|---|---|
| Eplerenone | 25.0 | 2.50 |
| Lactose, Hydrous NF | 294.1 | 29.41 |
| Microcrystalline Cellulose, NF | 57.7 | 5.77 |
| Talc, USP | 10.0 | 1.00 |
| Croscarmellose Sodium, NF | 8.0 | 0.80 |
| Sodium Lauryl Sulfate, NF | 2.0 | 0.20 |
| Colloidal Silicon Dioxide, NF | 2.0 | 0.20 |
| Magnesium Stearate, NF | 1.2 | 0.12 |
| Total Capsule Fill Weight | 400.0 | 40.00 |
| Hard Gelatin Capsule, Size #0, White Opaque | 1 Capsule | 100,000 Capsules |

### Example 28: 50 mg Dose Immediate Release Capsule

A 50 mg dose immediate release capsule formulation was prepared having the following composition:

**Table 12**

| **INGREDIENT** | **AMOUNT (mg)** | **REPRESENTATIVE BATCH AMOUNT (kg)** |
|---|---|---|
| Eplerenone | 50.0 | 5.00 |
| Lactose, Hydrous NF | 273.2 | 27.32 |
| Microcrystalline Cellulose, NF | 53.6 | 5.36 |
| Talc, USP | 10.0 | 1.00 |
| Croscarmellose Sodium, NF | 8.0 | 0.80 |
| Sodium Lauryl Sulfate, NF | 2.0 | 0.20 |
| Colloidal Silicon Dioxide, NF | 2.0 | 0.20 |
| Magnesium Stearate, NF | 1.2 | 0.12 |
| Total Capsule Fill Weight | 400.0 | 40.00 |
| Hard Gelatin Capsule, Size #0, White Opaque | 1 Capsule | 100,000 Capsules |

### Example 29: 100 mg Dose Immediate Release Capsule

A 100 mg dose immediate release capsule formulation was prepared having the following composition:

**Table 13**

| **INGREDIENT** | **AMOUNT (mg)** | **REPRESENTATIVE BATCH AMOUNT (kg)** |
|---|---|---|
| Eplerenone | 100.0 | 10.00 |
| Lactose, Hydrous NF | 231.4 | 23.14 |
| Microcrystalline Cellulose, NF | 45.4 | 4.54 |
| Talc, USP | 10.0 | 1.00 |
| Croscarmellose Sodium, NF | 8.0 | 0.80 |
| Sodium Lauryl Sulfate, NF | 2.0 | 0.20 |
| Colloidal Silicon Dioxide, NF | 2.0 | 0.20 |
| Magnesium Stearate, NF | 1.2 | 0.12 |
| Total Capsule Fill Weight | 400.0 | 40.00 |
| Hard Gelatin Capsule, Size #0, White Opaque | 1 Capsule | 100,000 Capsules |

### Example 30: 200 mg Dose Immediate Release Capsule

A 200 mg dose immediate release capsule formulation was prepared having the following composition:

**Table 14**

| **INGREDIENT** | **AMOUNT (mg)** | **REPRESENTATIVE BATCH AMOUNT (kg)** |
|---|---|---|
| Eplerenone | 200.0 | 20.00 |
| Lactose, Hydrous NF | 147.8 | 14.78 |
| Microcrystalline Cellulose, NF | 29.0 | 2.90 |
| Talc, USP | 10.0 | 1.00 |
| Croscarmellose Sodium, NF | 8.0 | 0.80 |
| Sodium Lauryl Sulfate, NF | 2.0 | 0.20 |
| Colloidal Silicon Dioxide, NF | 2.0 | 0.20 |
| Magnesium Stearate, NF | 1.2 | 0.12 |
| Total Capsule Fill Weight | 400.0 | 40.00 |
| Hard Gelatin Capsule, Size #0, White Opaque | 1 Capsule | 100,000 Capsules |

### Biological Evaluation I

A combination therapy of ACE inhibitor, eplerenone, and furosemide, was evaluated in humans as described in the following clinical trials.

### MATERIALS AND METHODS

### Study Design

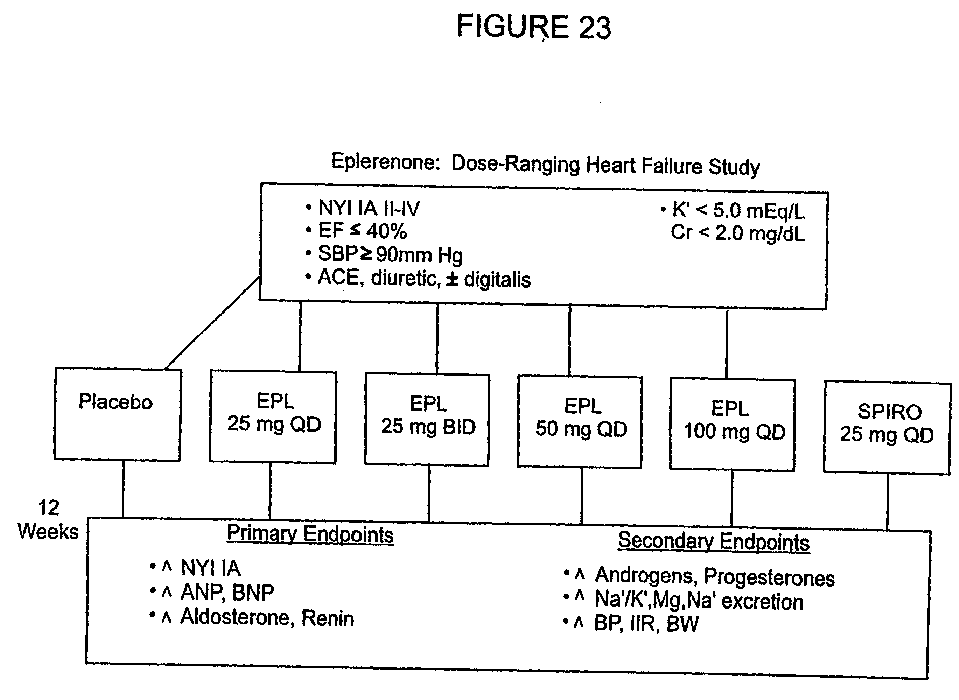

This was a randomized, double-blind, multi-center, placebo and active drug-controlled parallel group trial evaluating four different daily doses of eplerenone as compared to placebo. A 25 mg dose of spironolactone administered once/day served as the active control. The double-blind treatment period was 12†weeks in duration. The study is diagramed in Figure 23.

### Study Population

### Subject Enrollment

A total of 321 subjects were randomized into the double-blind treatment period. This sample size provided a 90% power at a two-sided 5% significance level to detect a 7†nmol/day mean difference in urinary aldosterone.

### Criteria for Inclusion

To qualify for inclusion in this study candidates met the following criteria:
1. The patient was a male or female ≥18 years of age. Female patients were post-menopausal, surgically sterile, or using effective barrier-type contraceptives.
2. The patient had all of the following evidence of symptomatic heart failure: 1) ejection fraction ≤40% (based on contrast ventriculography, radionuclide scan, or echocardiography) within the six months prior to the first dose of study medication; 2) a history of NYHA functional classification III or IV within the six months prior to study enrollment; and 3) a NYHA functional classification of II-IV at the time of study enrollment.
3. The patient was receiving and will continue the following therapy at the time of study enrollment: a) a loop diuretic for which the dose was unchanged during the two weeks prior to the first dose of study medication; and b) an ACE inhibitor for which the dose was unchanged during the one month prior to the first dose of study medication.
4. If the patient was on digitalis therapy, the therapy was begun at least three months prior to enrollment and the dose was unchanged during the one month prior to the first dose of study medication.
5. The patient's serum potassium was <5 mEq/L.
6. The patient's sitting systolic BP ≥90 mmHg.
7. The patient has provided witnessed written informed consent prior to diagnostic procedures and the administration of the first dose of the study medication.

### Criteria for Exclusion

A candidate was excluded from this study if he/she met any one of the criteria listed below:
1. Females of childbearing potential not using contraception or using oral or depot (hormonal) contraceptive measures.
2. The patient had any acute, life-threatening disease (including patients with an automatic implantable cardioverter/defibrillator) or has primary hepatic failure.
3. The patient had a heart transplant or was awaiting heart transplant surgery.
4. The patient had hemodynamically significant valvular disease. The patient with surgically corrected valvular disease was eligible.
5. The patient had experienced a myocardial infarction or had undergone coronary angioplasty or bypass surgery within the three months prior to the first dose of study medication.
6. The patient had unstable angina.
7. The patient had a systolic BP >160†mmHg or a diastolic BP >95†mmHg, taken in the sitting position.
8. The patient had intrinsic renal disease (defined as a serum creatinine concentration >180 Fmol/L or > 2.0 mg/dL).
9. The patient had uncontrolled diabetes defined as a fasting blood sugar >200†mg/L, insulin-dependent diabetes, or any other uncontrolled endocrine disorder.
10. The patient had an active malignancy of any type, or history of malignancy. (Patients with a history of basal cell carcinoma that was surgically removed were acceptable. Patients with a history of other malignancies that were surgically removed or otherwise treated for cure and show no evidence of recurrence for at least five years prior to study enrollment were also acceptable).
11. The patient's hepatic enzyme measurements (SGOT, SGPT, GTT) were >1.5 x upper limits of normal.
12. The patient had other clinically significant abnormalities in hematology or biochemistry values.
13. The patient was receiving potassium supplement, or antiarrhythmic therapy (except amiodarone). Drugs were stopped at least 5 half-lives before baseline visit and potassium supplements at least 48 hours before.
14. The patient had received a potassium-sparing diuretic within the one month prior to the first dose of study medication.
15. The patient was receiving, on a regular basis, any of the following at the time of study enrollment: a) nonsteroidal anti-inflammatory drugs (including aspirin > 300 mg/day), b) steroids, c) dopamine agonists or antagonists, d) heparin, or e) †insulin.
16. The patient had received any investigational medication within 30 days prior to the first dose of study medication or is scheduled to receive an investigational drug other than study medication during the course of this study.
17. The patient had known hypersensitivity to eplerenone, spironolactone, or related compounds.
18. The patient had previously been admitted to this study.

### Randomization Procedures

Patients were assigned, in the order of enrollment, to receive the 12-week dosing regimen of eplerenone 25 mg once/day, 25 mg BID, 50 mg once/day, 100 mg once/day, 25 mg spironolactone once/day or placebo, according to a computer-generated randomization schedule prepared at Searle prior to the start of the study. The randomization was 1:1 for all groups. Patients were assigned study numbers at the baseline visit.

### Description of Clinical Supplies

Investigational drug supplies were provided by Searle and consisted of the following:
- capsules containing eplerenone in doses of 25 mg, 50 mg, 100 mg, or 200 mg, all identical in appearance.
- placebo capsules, identical in appearance to the eplerenone capsules.
- tablets containing spironolactone in a dose of 25 mg.
- placebo tablets, identical in appearance to spironolactone tablets.
New study medication was dispensed at baseline visit, Weeks 2, 4, and 8 visits.

### Drug Administration

Study medication was taken without regard to mealtimes. The morning dose was taken between 6:00 a.m. and 10:00 a.m. in the morning, upon arising. The evening dose was taken 12 hours later.

For all dosing periods patients were instructed to take one capsule from bottle 1 and one tablet from bottle 2 in the morning, upon arising, and take one capsule from bottle 3 twelve†hours later in the evening. If a dose was missed, the patient did not double-dose the next day. However, a missed dose could be taken before the end of the day, if at least an eight-hour interval was kept between the two daily doses. Only two doses, one morning and one evening, were taken in one day. Patients were encouraged to take the study medication at the same times every day.

### STUDY PLAN

### Pretreatment Period

### Screening Visit

Written informed consent was obtained prior to initiation of any study related procedures.
- Medical history was taken not more than two weeks preceding initiation of double-blind treatment. The medical history included specific questions to allow baseline identification of underlying cardiac disease leading to the syndrome of CHF, and to identify the duration and clinical course of CHF prior to admission into the trial.
- Physical examination was also performed during this pretreatment period.
- Body weight and vital signs included sitting heart rate and blood pressure by cuff (sphygmomanometer).
- Ejection Fraction (EF) measurement completed within six months prior to the study was acceptable. If no EF was available within six months, it was performed to confirm an EF ≤40%.
- NYHA Functional Classification was determined.
- ECG (12-lead) was performed always after completion of the blood pressure and heart rate and temperature measurements.

The inclusion/exclusion criteria were reviewed and used to determine each patientís potential eligibility for the study.

### Clinical Laboratory Tests

Fasted clinical laboratory tests were performed (blood and urine) for the following analysis:

| **Hematology** | |
|---|---|
| WBC with Differential | Platelet Count |
| RBC | Hematocrit |
| Hemoglobin | |

| **Biochemistry** | |
|---|---|
| Sodium indirect | Total Bilirubin/direct and |
| Potassium | Uric Acid |
| Chloride | Glucose |
| Calcium | Alkaline Phosphatase |
| Magnesium | Glutamyl Transferase (GGT) |
| Phosphate (inorganic) | SGOT (AST) |
| Urea (BUN) | SGPT (ALT) |
| Creatinine | LDH |
| Total Protein | Creatine Kinase (CK) |
| Albumin | Cholesterol Triglycerides |

| **Urinalysis** | |
|---|---|
| pH | Glucose |
| Specific Gravity | Ketones |
| Protein | Blood |

### Procedures and Observations

### NYHA Functional Classification

The patient's current NYHA functional classification was determined at selected visits throughout the study. The following was used in evaluating a patient's functional class:
Class I: No symptoms with ordinary physical activity
Class II: Symptoms with ordinary physical activity but not at rest
Class III: Symptoms with less than ordinary physical activity but not at rest
Class IV: Symptoms present at rest.

### Sodium Retention Score

A scoring system was used to determine the current sodium retention score for each patient. The scoring system is shown in Table 15.

**Table 15.**

| **Sodium Retention Scoring System** | | |
|---|---|---|
| **Parameter** | **Grade** | **Assessment** |
| Rales | 0 | absent |
| | 1 | in lower 1/3 of lungs |
| | 2 | in lower 2/3 of lungs |
| | 3 | in all lung fluids |
| Peripheral Pitting | 0 | absent |
| Edema | 1 | trace |
| | 2 | limited to ankles |
| | 3 | not limited to ankles |
| | 4 | anasarca |
| Weight Change | -1 | decreased |
| | 0 | unchanged |
| | 1 | increased |
| Hepatomegaly | 0 | absent |
| | 1 | present |
| S3 Gallop | 0 | absent |
| | 1 | present |
| Increased Jugular | 0 | absent |
| Venous Pressure (JVP) | 1 | present |

The sum of all the grades was the patient's sodium retention score. The range of possible scores is -1 to 11.

### Urine Collection

A 24-hour urine collection was started two days prior to Day 0, Week 12 and Week 16 visits. The urine volume and duration of collection were measured and samples were analyzed for the following:
1. Sodium
2. Potassium
3. Aldosterone.
A 24-hour sodium excretion rate and a sodium/potassium ratio were subsequently determined.

### Plasma Renin, Atrial Natriuretic Peptide, and Brain Natriuretic Peptide

Blood samples were drawn in the morning following an overnight fast but prior to taking any medication. The patient was in a supine position for 30 minutes prior to the collection. Samples were analyzed for determination of active and total renin, N-terminal ANP and BNP levels.

### Concurrent Medication

The use of any medication other than the drugs required by this study were avoided, if possible, during the treatment period. The following drugs were specifically excluded:
1. Potassium sparing diuretics (e.g., spironolactone, triamterene)
2. Non-steroidal anti-inflammatory drugs (including chronic aspirin intake in excess of 300†mg/day)
3. Steroids
4. Antiarrhythmics (except amioderone)
5. Dopamine agonists or antagonists
6. Heparin
7. Insulin.
Potassium supplements were excluded at entry, but could be used at the Investigator's discretion as rescue therapy during the study, particularly in the event of hypokalemia <†3.5†mEq/L. Digitalis, nitrates, calcium and beta blockers were acceptable concomitant medications. Changes in ongoing ACE inhibitor, loop diuretic, or digitalis therapy were allowed if considered necessary. If a decline in BP required adjustment of medication, it was recommended to decrease the dose of diuretic first, or the ACE inhibitor second without interruption of dosing of the study medication.

### EVALUATION OF RESULTS

### Analysis Populations

All patients who took at least one dose of study drug were included in the intent-to-treat cohort. An evaluable cohort consisted of randomized patients who satisfied all of the following criteria:
1. Exhibited evidence of symptomatic heart failure at time of enrollment, defined as:
   a.Ejection fraction # 40% (based on contrast ventriculography, radionuclide, scan, or echocardiography) within six months prior to the first dose of study medication
   b.NYHA functional class III or IV within six months prior to the first dose of study medication, and
   c.NYHA functional class II-IV at time of study enrollment.
2. Continued to receive an ACE inhibitor and a loop diuretic, for which doses were unchanged during the one month prior to the first dose of study medication. If on digitalis at study entry, the therapy must have begun at least three months prior to enrollment and the dose remain unchanged for at least one month prior to the first dose of study medication.
3. Did not have:
   a.Hemodynamically significant and uncorrected valvular disease upon entry
   b.A myocardial infarction within three months prior to the first dose of study medication
   c.Unstable angina upon entry
4. Had not received a potassium sparing diuretic during the trial nor within one month prior to the first dose of study medication.
5. Had not received any of the following at the time of study enrollment nor during the interval under consideration:
   a.Chronic use of nonsteroidal anti-inflammatory drugs (including chronic aspirin use at a dose > 300 mg/day). Chronic is defined as use for greater than three consecutive days.
   b.Steroids
   c.Dopamine agonists or antagonists
   d.Insulin
   e.Heparin
   f.Antiarrhythmics (except amiodarone)
6. Had the pre-study cardiac assessments within 14 days prior to the first dose of study medication.
7. Took at least 80% of the prescribed doses of study medication for the interval under consideration.

### Evaluation of Efficacy

Measures of clinical efficacy in this study are the NYHA functional classifications and the sodium retention score at baseline and after 12 weeks of treatment. Treatment groups will be compared with respect to the number of patients showing improvement, no change, or worsening in each variable from baseline to Week 12. For NYHA functional classification, an improvement in cardiac condition will be defined as a reduction of at least one functional class, and worsening will be defined as an increase of at least one functional class. Similarly, an improvement (worsening) in sodium retention score will be defined as a reduction (increase) in cumulative score. For each variable the Cochran-Mantel-Haenszel (CMH) test stratified by center, will be used to evaluate differences among the treatment groups. In addition, stratified CMH tests will be used to compare each active treatment to placebo and each eplerenone dose to spironolactone.

Kruskall-Wallis tests were used to compare treatment groups with respect to changes from baseline at each visit in vital signs (blood pressure, heart rate, and weight), urinary sodium excretion rate, urine Na/K ratio, 24-hour urinary aldosterone, plasma ANP, BNP, plasma renin and serum magnesium. Dose response were tested using Spearman rank correlation. Each active treatment was compared to placebo and each eplerenone dose was compared to spironolactone using Wilcoxon rank sum tests.

### RESULTS

### Recruitment

Three-hundred and twenty-one patients were recruited with stable NYHA Class II-IV heart failure.

### Patient Characteristics

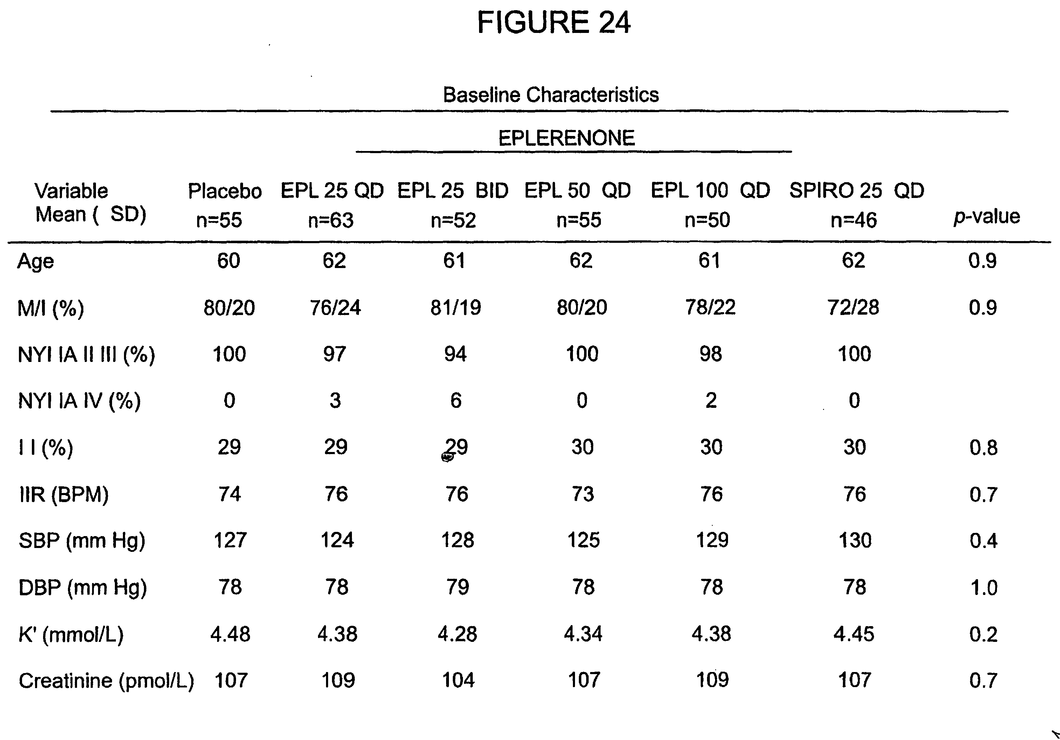

Patient demographic, vital signs, and cardiac status at baseline are summarized in Figure 24.

Patients had a mean age of 61 years and approximately 80% were male. At baseline approximately 97% of the patients were NYHA Class II-III. With respect to sodium retention score, there was no statistically significant change seen for any of the groups after 12 weeks of treatment. There was no significant change in NYHA Class for any of groups after 12 weeks of treatment.

### Patient Therapy

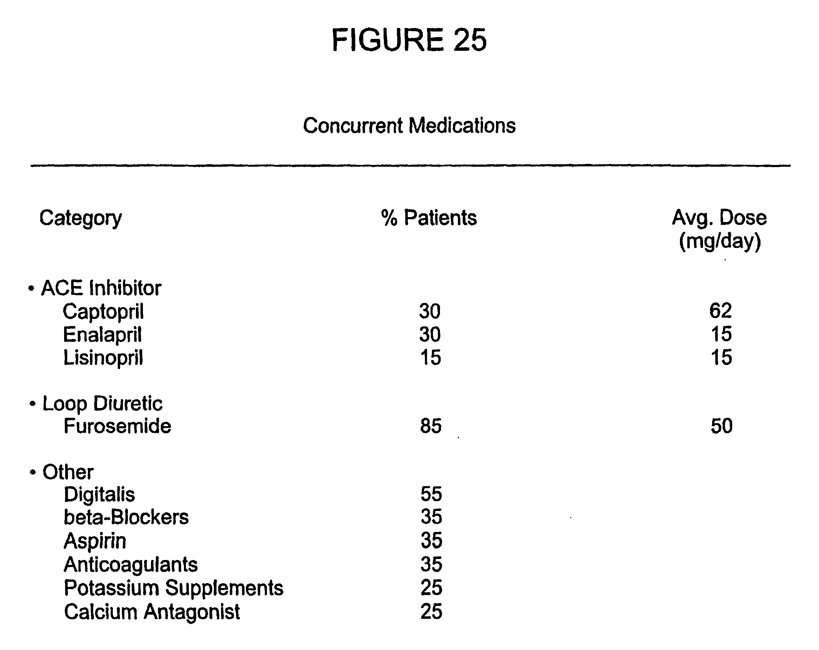

The concurrent medications taken by the patients in this study are shown in Figure 25. Seventy-five percent of the patients took captopril, enalapril or lisinopril. The remaining 25% took one of a variety of other ACE inhibitors. Eighty-five percent took the loop diuretic furasemide. Fifty-five percent took digitalis.

### Changes in Vital Signs

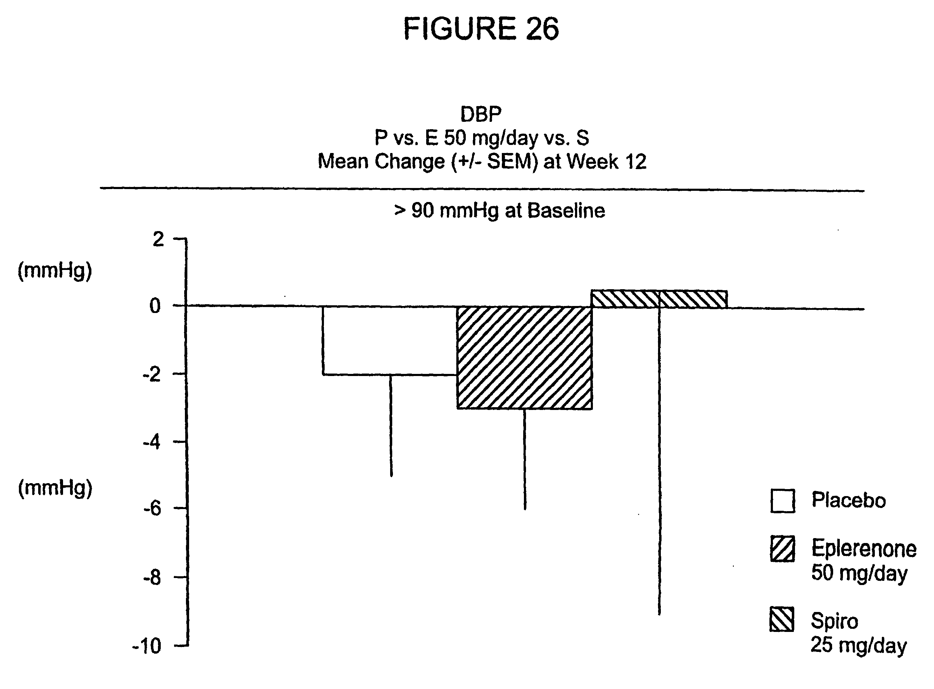

There were no significant changes observed in body weight or blood pressure (see Figure 26) for any of the groups after 12 weeks of treatment.

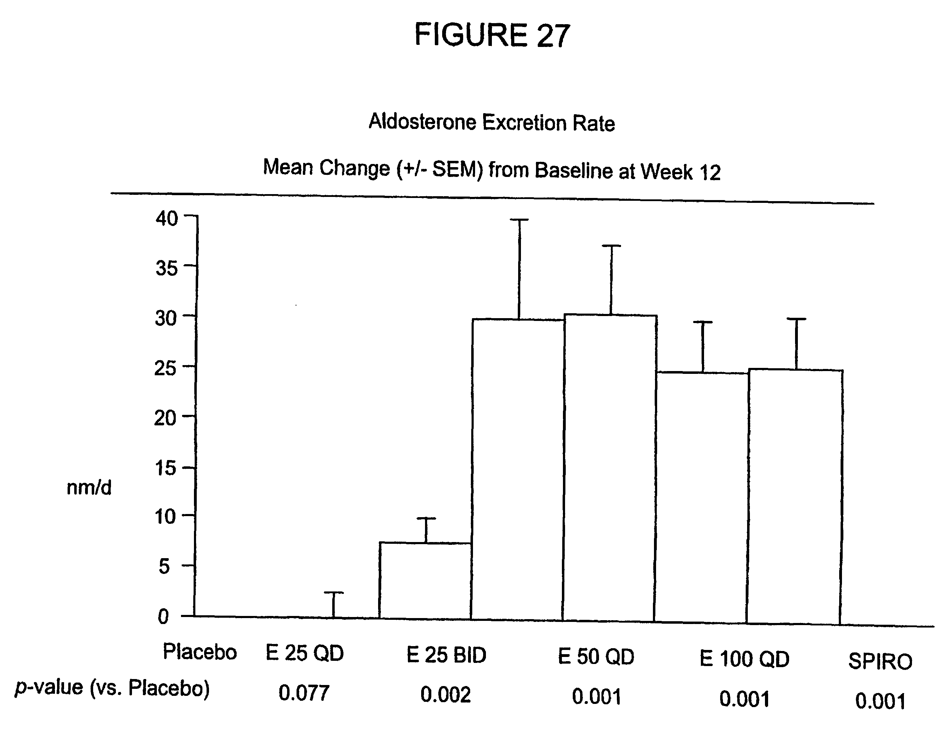

### Urinary Aldosterone (See Figure 27)

Urinary aldosterone was determined for baseline and the 12 week visit. Urinary aldosterone excretion showed mean increases from baseline in all treatment groups, although values for those receiving 25 mg eplerenone QD did not reach statistical significance (P>0.05). Greater increases were seen at eplerenone doses of 50 and 100 mg.

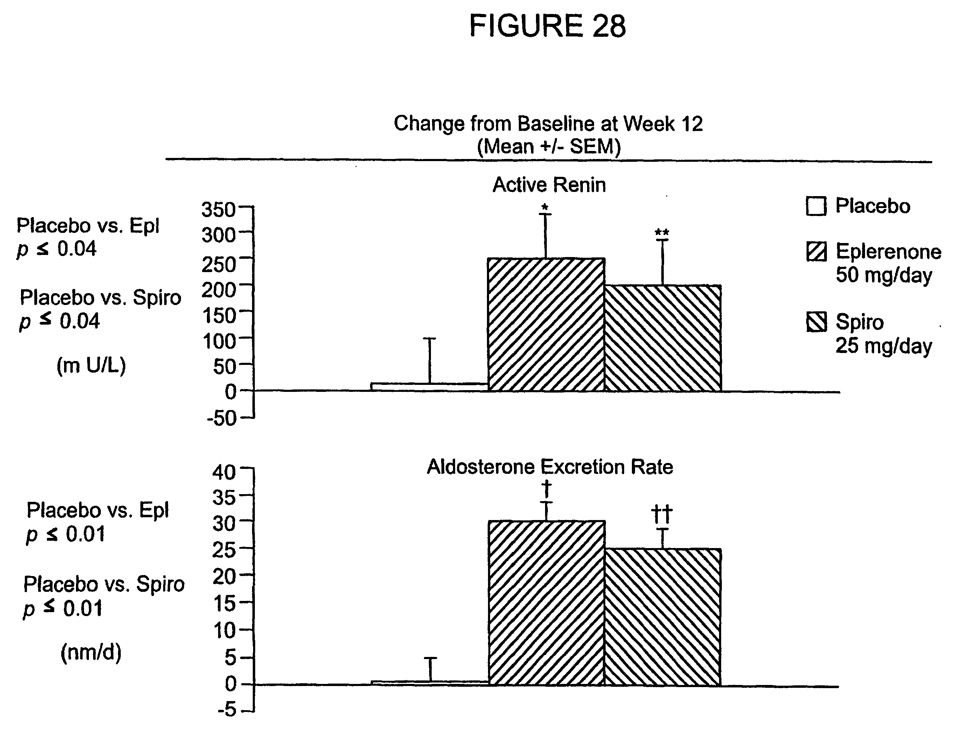

### Plasma Renin Activity (See Figure 28)

Plasma renin activity (PRA) was significantly elevated by both eplerenone and spironolactone therapy. The increased PRA correlated with the increased aldosterone excretion, indicating activation of the renin-angiotensin,aldosterone system in response to drug treatment.

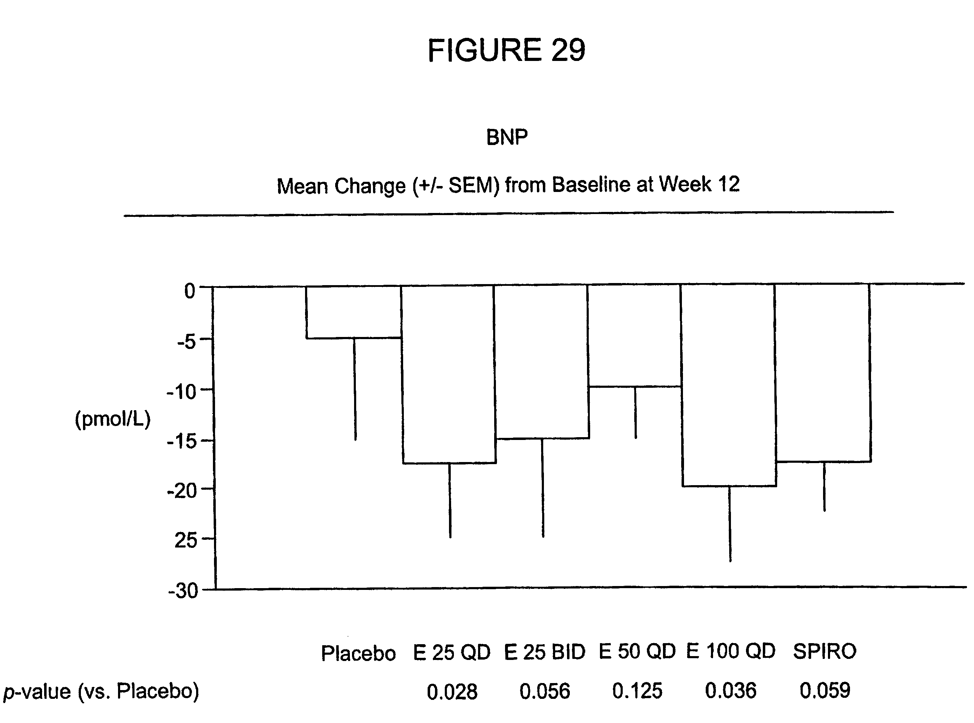

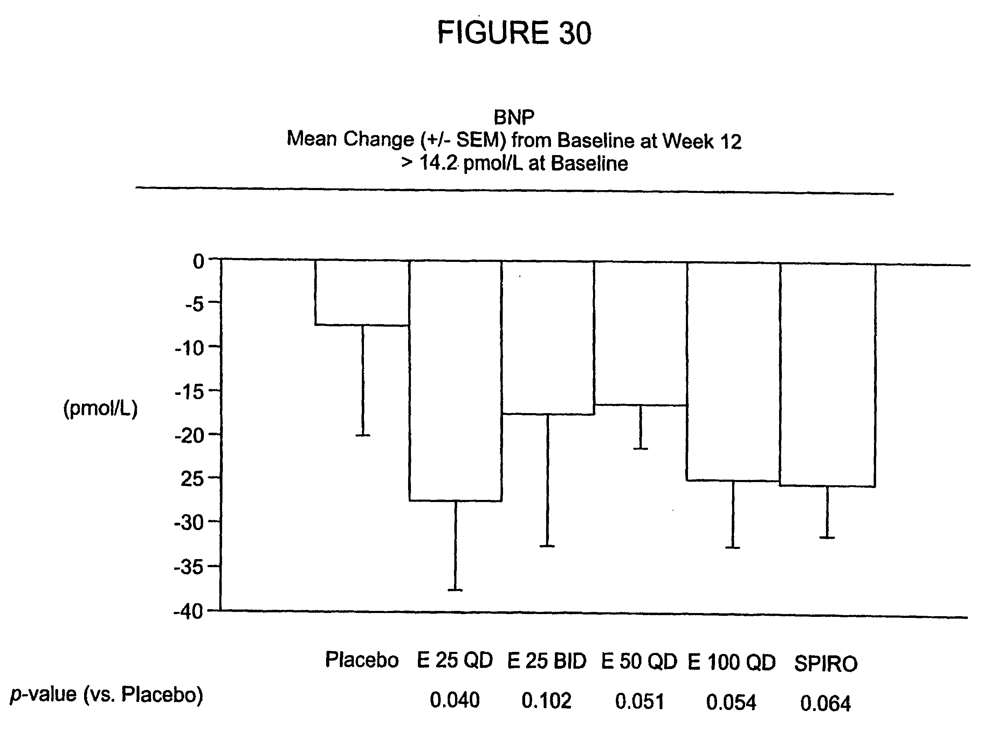

### N-Terminal Atrial Natriuretic Peptide (ANP) and Brain Natriuretic Peptide (BNP) (See Figures 29 and 30)

After 12 weeks of treatment all groups showed a non-significant reduction in ANP levels.

The reduction in BNP was highly significant for eplerenone at 25 mg and 100 mg (see Figure 29). For patients with high baseline BNP levels the reduction was even greater (see Figure 30). ANP and BNP are counterrulatory hormones that are unregulated in heart failure to counteract vasoconstriction and fluid retention induced by other hormones. Blood levels of ANP and BNP are predictive of heart failure. BNP, synthesized in the ventricle, is more sensitive than ANP for predicting decreases in the left ventricular ejection fraction, a measure of heart failure. The significant decrease in BNP levels seen in groups treated with eplerenone indicates that at 25 mg/day eplerenone, given in conjunction with an ACE inhibitor and a loop diuretic, provides a significant benefit in heart failure patients.

### Testosterone

In males there was a significant increase in testosterone levels for the spironolactone treated group, compared to the eplerenone treated patients. This result reflects the additional binding of spironolactone to the testosterone receptor, which can give rise to unwanted side-effects such as prostate reduction, loss of libido and gynecomastia. The lack of effect on testosterone levels seen with eplerenone treatment indicates that eplerenone does not interfere with the binding of testosterone to receptors nor does eplerenone produce any related, unwanted side-effects.

### Serum Potassium

There was no significant difference in serum potassium levels for any of the groups after 12 weeks of treatment.

The lactose monohydrate used in each of the examples of the application is commercially available from Formost Farms, Baraboo, Wisconsin. The Avicel brand of microcrystalline cellulose and the Ac-Di-Sol brand of croscarmellose sodium were used in each of the examples of the application. Both compounds are commercially available from FMC Corporation, Chicago, Illinois. The Pharmacoat 603 brand of hydroxypropyl methylcellulose was used in each of the examples of the application. This compound is commercially available from Shin-Etsu Chemical Co. Ltd.. The sodium lauryl sulfate used in each of the examples of the application is commercially available from Henkel Corporation, Cincinnati, Ohio. The talc used in each of the examples of the application is commercially available from Cyprus Foote Mineral Co., Kings Mountain, North Carolina, or Luzenac America, Inc., Englewood, Colorado. The magnesium stearate used in each of the examples of the application is commercially available from Mallinckrodt Inc., St. Louis, Missouri. The Opadry White YS-1-18027A used to prepare the coated tablets disclosed in the examples of this application is a ready to coat coating formulation commercially available from Colorcon, West Point, Pennsylvania.

Although this invention has been described with respect to specific embodiments, the details of these embodiments are not to be construed as limitations.

### Biological Evaluation II

### MATERIALS AND METHODS

### Study Design and Procedures

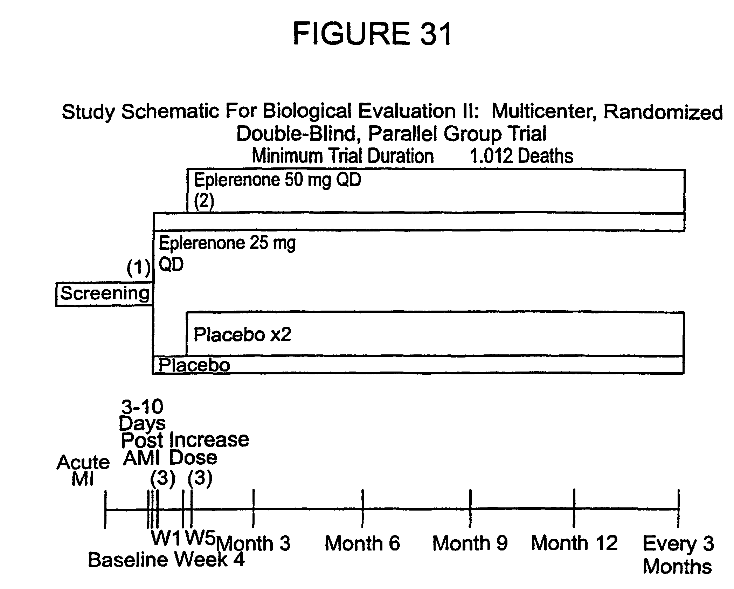

This multicenter, randomized, double-blind, placebo-controlled, two-arm, parallel group trial will continue until 1,012 deaths occur, which is estimated to require approximately 6,200 randomized patients followed for an average of approximately 2.5 years. See Figure 31.

Eligible patients can be identified for inclusion at any time following emergency room evaluation and presumptive diagnosis of AMI with HF. Patients eligible for this study, (per Hall AS, Winter C, Bogle SM, Mackintosh AF, Murray GD, Ball SG on behalf of the AIRE Study Investigators. The acute infarction ramipril efficacy (AIRE) study: rationale, design, organization, and outcome definitions. J Cardiovasc Pharmacol 1991;18(Suppl 2) :S105-S109), must have, :
- AMI (the index event) documented by:
   - abnormal cardiac enzymes (creatine phosphokinase [CPK] >2x upper limit of normal [ULN] and/or CPK-MB [CPK MB isozyme band] >10% of total CPK); and
   - evolving electrocardiogram (ECG) diagnostic of MI (i.e., progressive changes in the ST segment and T wave compatible with AMI with or without the presence of pathological Q waves)
- LV dysfunction, documented by LV ejection fraction (LVEF) ≤40% by echocardiogram, radionuclide angiography, or LV angiography determined following the index AMI and before randomization.
- HF documented by at least one of the following:
   - pulmonary edema (bilateral posttussive crackles extending at least one-third of the way up the lung fields in the absence of significant chronic pulmonary disease); OR
   - chest x-ray showing pulmonary venous congestion with interstitial or alveolar edema; OR
   - auscultatory evidence of a third heart sound (S₃) with persistent tachycardia (>100 beats per minute).

Patients will receive standard therapy which may include ACE-I, diuretics, nitrates, and β-blockers, and may have received anticoagulants and antiplatelet agents, and may have received thrombolytics or emergency angioplasty.

Eligible patients may be identified for inclusion at any time following emergency room evaluation and presumptive diagnosis of AMI with HF. Patients who qualify for the study will be randomized between 3 (>48 hours) and 10 days post-AMI if their clinical status is stable, e.g., no vasopressors or inotropes, intra-aortic balloon pump (IABP), hypotension (SBP<90 mmHg), or recurrent chest pain likely to lead to acute coronary arteriography. Patients with implanted cardiac defibrillators are excluded. Randomization should preferably occur prior to hospital discharge.

Patients will be randomized to receive eplerenone 25 mg QD (once daily) or placebo. At four weeks, the dose of study drug will be increased to 50 mg QD (two tablets) if serum potassium <5.0 mEq/L. If at any time during the study the serum potassium is >5.5 mEq/L but <6.0 mEq/L, the dose of study drug will be reduced to the next lower dose level, i.e., 50 mg QD to 25 mg QD (one tablet), 25 mg QD to 25 mg QOD (every other day), or 25 mg QOD to temporarily withheld (see *Study Drug Administration* for detailed dosing instructions). If at any time during the study the serum potassium is ≥ 6.0 mEq/L, study medication is to be temporarily withheld, and may be restarted at 25 mg QOD when the serum potassium level is <5.5 mEq/L, and increased according to the schema presented in *Study Drug Administration,* Table 16. If at any time during the study the serum potassium is persistently ≥6.0 mEq/L, study medication is to be permanently discontinued. The potassium level may be repeated if the potassium increase is thought to be spurious (i.e., due to hemolysis or recent dosing with a potassium supplement). If the patient becomes intolerant of study medication, alterations in the dose of concomitant medications should be considered prior to dose adjustment of study medication. Serum potassium will be determined at 48 hours after initiation of treatment, at 1, 4 and 5 weeks, at all other scheduled visits, and within one week following any dose change. Dosing adjustments are to be made based on the most recent potassium level, as described in *Study Drug Administration.*

Study visits will occur at screening, baseline (randomization), 1 and 4 weeks, 3 months, and every 3 months thereafter until the study is terminated. At screening, medical history, cardiac enzymes, Killip class, time to reperfusion (if applicable), documentation of AMI and of HF, determination of LVEF, and a serum pregnancy test for women of childbearing potential will be done. A physical examination will be done at screening and at the final visit (cessation of study drug). Hematology and biochemistry evaluations and urinalysis for safety will be done at screening, Week 4, Months 3 and 6, and every 6 months thereafter until the study is terminated. An additional blood sample for DNA analysis will be collected during screening. Vital signs (seated heart rate and BP), New York Heart Association (NYHA) functional class, adverse events, and selected concurrent medications will be recorded at every visit. Quality of Life (QOL) assessments will be completed during screening, at Week 4, Months 3, 6, and 12, and at the final visit. All randomized patients will be followed for all study endpoints every 3 months until the study is terminated.
1. Concurrent therapy may include: ACE, ± diuretics, ± β-blockers, ± aspirin, ± other usual post-AMI regimen.
2. Increase dose if current dose is tolerated and serum potassium is <5.0 mEq/L.
3. Serum potassium must be determined at 48 hours, Week 1, Week 5 and within 1 week following any study medication dose change. Dose adjustments are to be made based on the most recent potassium level. If at any time during the study, serum potassium is >5.5 mEq/L but <6.0, reduce the dose of the medication to the next lower dose. If at any time during the study, serum potassium is ≥6.0 mEq/L, temporarily withhold study medication. If study medication is temporarily withheld, it may be restarted at one tablet QOD when serum potassium is < 5.5 mEq/L and titrated as directed in Table 16. If at any time during the study the serum potassium is persistently ≥6.0 mEq/L, permanently discontinue study medication.

Serum potassium determination at 48 hours after initiation of dosing.

### Study Population

### Patient Enrollment

A sufficient number of patients will be enrolled to ensure that a total of 1,012 deaths will occur over the course of the study.

### Criteria for Inclusion

1. The patient has had an AMI, as documented by:
   a. cardiac enzyme rise (total CPK >2xULN and/or CPK-MB >10% of total CPK), AND
   b. evolving ECG diagnostic of MI (i.e., progressive changes in the ST segment and T wave compatible with AMI with or without the presence of pathological Q waves)
2. The patient has LV dysfunction, as documented by LVEF ≤40% by echocardiogram, radionuclide angiography, or LV angiography determined following the index AMI and before randomization.
3. The patient has clinical evidence of HF, demonstrated by at least one of the following:
   a. pulmonary edema (bilateral posttussive crackles extending at least one-third of the way up the lung fields in the absence of significant chronic pulmonary disease); OR
   b. chest x-ray showing pulmonary venous congestion with interstitial or alveolar edema; OR
   c. auscultatory evidence of a third heart sound (S₃) with persistent tachycardia (>100 beats per minute).
   Clinical evidence of HF post AMI can be transient, occurring at any time from the onset of the index AMI prior to randomization. Evidence of HF does not necessarily need to be present at the time of randomization.
4. Stable clinical status at the time of randomization 3 (>48 hours) to 10 days following the AMI. Stable clinical status excludes the use of vasopressors and inotropes. Stable status also excludes the use of an IABP, hypotension (SBP<90 mmHg), and recurrent chest pain likely to lead to acute coronary arteriography.
5. The patient is a male or nonpregnant female ≥21 years of age.
6. If the patient is a female, she is post-menopausal, or if of childbearing potential, she is using adequate contraception (hormonal, e.g., oral contraceptives or hormonal implants, or barrier method, e.g., diaphragm, IUD, etc.), or is surgically sterile, and is not lactating. Abstinence is not an acceptable form of contraception.
7. If the patient is a female of childbearing potential, she has had a negative serum pregnancy test within 72 hours prior to the first scheduled dose of double-blind study drug.
8. The patient has no clinically significant abnormal clinical laboratory values which in the Investigator's opinion precludes the patient from safely participating in this study.
9. The patient is willing and able to participate for the duration of the study.
10.The patient has provided written informed consent prior to any test or procedure being performed, or medication being changed, for this study.

### Criteria for Exclusion

1. The patient has HF of primary valvular or congenital etiology.
2. The patient has current evidence of clinical instability (e.g., arrhythmias other than atrial fibrillation, cardiogenic shock, etc.).
3. The patient has post-infarct angina likely to lead to acute coronary arteriography.
4. A coronary artery bypass graft (CABG) is planned for the index AMI.
5. The patient has an implanted cardiac defibrillator (ICD).
6. The patient has uncontrolled hypotension (SBP<90 mmHg).
7. The patient requires the use of potassium-sparing diuretics or spironolactone.
8. The patient has a serum creatinine level >2.5 mg/dL during the screening period.
9. The patient has a serum potassium level >5.0 mEq/L during the screening period.
10. The patient has a planned cardiac transplantation.
11. The patient has current evidence of alcohol or drug abuse problems, which in the Investigator's opinion, precludes study participation.
12. The patient has any condition which, in the Investigator's opinion, makes participation in this study not in the best interest of the patient.
13. The patient has known hypersensitivity to eplerenone or spironolactone.
14. The patient has a severe organic disorder or has had surgery or disease of the gastrointestinal tract which, in the opinion of the Investigator, may interfere with the absorption, pharmacokinetics, or elimination of the study medication.
15. The patient has chronic psychoses or behavioral conditions that in the opinion of the Investigator would limit the ability of the patient to comply with the requirements of this study.
16. The patient has a comorbid condition that would be expected to result in death during the next three years (e.g., terminal cancer, AIDS, etc.) including patients receiving immunosuppressive or antineoplastic therapy.
17. The patient has received any investigational medication or investigational device within 30 days prior to the first dose of study medication or is actively participating in any investigational drug or device study, or is scheduled to receive an investigational drug other than eplerenone or be treated with an investigational device during the course of this study.
18. The patient has been previously admitted to the study.

### Randomization Procedures

Patients will be assigned at each site to a double-blind treatment arm in the order in which they meet criteria for randomization. They will receive their allocated treatment according to a computer-generated randomization schedule prepared at Searle prior to the start of the study.

### Generation of Randomization Code

The Searle clinical database administrator will generate the patient randomization schedule using Searle's standard randomization program. The Searle statistician will generate the randomization schedule for medication kit identification numbers, separate from the patient randomization schedule. The randomizations will be provided to a drug packaging contractor and to the Interactive Voice Response System (IVRS) center for drug assignments. The Searle statistician will not have access to the randomization codes after patient recruitment begins. The Searle clinical database administrator will keep both the patient randomization schedule and the medication identification schedule in a locked file for the duration of the study. A sealed copy of the patient randomization schedule will be provided to the U.S. Food and Drug Administration (FDA) prior to the start of the study. The randomization schedule will also be made available to the unblinded statistical group performing the statistical analyses for the Data Safety Monitoring Board (DSMB). A designated individual in the Searle pharmacy will have access to the medication kit identification randomization for drug packaging purposes only. No other Searle personnel will have access to the randomization schedules until the study is completed.

In general, no study site personnel will have access to the randomization codes. However, if it is medically necessary to unblind a particular patient, a sealed treatment assignment is provided with each study medication package.

### Interactive Voice Randomization System (IVRS)

A 24-hour IVRS will be used to assign patient numbers and blinded study drug to patients, track inventories of study drug at sites, and track recruitment and progress of patients.

Site personnel will call the IVRS center to randomize each patient. The Investigator will provide the IVRS center with various identifiers for each patient and confirm that inclusion/exclusion criteria have been met. The IVRS center will assign the patient to a treatment according to a patient randomization schedule described above. The IVRS system will then select a blinded medication identification number appropriate to the patient's treatment assignment from those available at the study site.

Following randomization, sites are to call the IVRS center whenever study drug is dispensed. The IVRS center must also be notified if the patient withdraws from treatment or is unblinded. Furthermore, the IVRS center must be called to confirm receipt of study drug supplies.

Additional details of the IVRS system and procedures will be provided in a separate IVRS manual.

### Emergency Code Break

In the event of an emergency, the site will be provided a telephone number to call in order to request to have the study blind broken. The code may be broken if an emergency situation arises that in the Investigator's opinion requires knowledge of the code. In these cases, the Investigator should attempt to contact the Sponsor before breaking the code. The date and reason for the code break must be submitted on the appropriate CRF to the data coordinating center by the Investigator as soon as possible. See Appendix 3.0 for additional information.

### Description of Clinical Supplies

Searle will provide eplerenone 25 mg tablets and matching placebo for eplerenone 25 mg tablets, as follows:

### Initial Phase Study Medication (Weeks 0 through 4)

Bottles of 40 tablets with child-resistant closure to be dispensed at baseline (Day 0).

### Maintenance Phase Study Medication (After Week 4)

Bottles of 200 tablets with child-resistant closure to be dispensed for the remainder of the study.

Double-blind eplerenone or matching placebo study medication will be supplied in bottles pre-labeled with appropriate kit numbers for each treatment arm. Prior to dispensing, all study medication must be stored according to labeled storage conditions in a secure area with limited access. At home, the patient must keep the medication free from environmental extremes. When the study is completed or discontinued, all used and unused supplies of drug must be returned or disposed of, as directed by the Searle Monitor or monitors designated by Searle.

### Labeling of Clinical Supplies

Two-part labels will be computer-generated for this blinded study. One part of the label, containing study and patient information will be attached to the container; the other part is a tear-off portion that contains the same information plus a sealed pouch containing the identity of the assigned treatment. This tear-off tab is to be removed at the time of dispensing, attached to the patient's appropriate CRF and retained in the Investigator's study file.

The following dosage instructions will appear on the label in the language(s) appropriate to the country in which the drug will be used:

### Initial Phase Bottle

"Take one tablet every morning with water or as otherwise directed by your physician."

### Maintenance Phase Bottle

"Take two tablets every morning with water or as otherwise directed by your physician."
See Section 3.3 and Appendix 3, Section 3.0 for detailed information on randomization and the blinding safeguards.

### Study Drug Administration

Patients will receive eplerenone 25 mg QD or placebo (one tablet) for the first four weeks of treatment. At four weeks, the dose of study drug will be increased to 50 mg QD (two tablets) if serum potassium <5.0 mEq/L. If the serum potassium is ≥5.0 mEq/L at Week 4 but <5.0 mEq/L at Week 5, the dose of study drug will be increased to 50 mg QD (two tablets). In this case, serum potassium is to be checked at Week 6.

Table 16 summarizes mandated dosing changes for serum potassium levels. Serum potassium will be determined at 48 hours after initiation of treatment, at 1 and 5 weeks, and within one week following any dose change. If at any time during the study the serum potassium is >5.5 mEq/L, the dose of study drug will be reduced to the next lower dose level, i.e., 50 mg QD to 25 mg QD, 25 mg QD to 25 mg QOD, or 25 mg QOD to temporarily stopped. Study medication is to be restarted at 25 mg QOD when the serum potassium level is <5.5 mEq/L and increased according to the scheme presented in Table10. The potassium level may be repeated if the potassium increase is thought to be spurious (i.e., due to hemolysis or recent dosing with a potassium supplement).

If the patient becomes intolerant of study medication, alterations in the dose of concomitant medications (e.g., potassium supplements, ACE-I, etc.) should be considered prior to dose adjustment of study medication. If at any time during the study the serum potassium level is ≥6.0 mEq/L, study medication is to be temporarily withheld. If serum potassium level is persistently ≥6.0 mEq/L, the patient is to discontinue study medication. If elevated potassium levels are observed <6.0 mEq/L, potassium supplements, if any, should be stopped and the patient should continue to receive study medication. If study medication is stopped, concurrent medications should be reviewed and the doses adjusted if possible according to good clinical practice.

**Table 16.**

| **Study Medication Dosing Adjustment for Serum Potassium Levels** | | | |
|---|---|---|---|
| **If Serum Potassium Level Is:** | **And Current Dose Is:** | **Dose Change:** | **Number of Tablets:** |
| <5.0 mEq/L | Withhold | Increase | 1 tablet QOD |
| <5.0 mEq/L | 1 tablet QOD | Increase | 1 tablet QD |
| <5.0 mEq/L | 1 tablet QD | Increase | 2 tablets QD |
| <5.0 mEq/L | 2 tablets QD | No change | 2 tablets QD |
| ≥5.0 and <5.5 mEq/L | Withhold | Increase | 1 tablet QOD |
| ≥5.0 and <5.5 mEq/L | 1 tablet QOD | No change | 1 tablet QOD |
| ≥5.0 and <5.5 mEq/L | 1 tablet QD | No change | 1 tablet QD |
| ≥5.0 and <5.5 mEq/L | 2 tablets QD | No change | 2 tablets QD |
| ≥5.5 and <6.0 mEq/L | Withhold | No change | None |
| ≥5.5 and <6.0 mEq/L | 1 tablet QOD | Decrease | None |
| ≥5.5 and <6.0 mEq/L | 1 tablet QD | Decrease | 1 tablet QOD |
| ≥5.5 and <6.0 mEq/L | 2 tablets QD | Decrease | 1 tablet QD |
| ≥6.0 mEq/L | Any dose | | None |
| If persistent elevation, discontinue medication. If single elevation, withhold dosing. | | | |

### STUDY PLAN

### Schedule of Observations and Procedures

### Screening Period

The Screening Period is defined as the period after AMI and prior to randomization. This section describes the procedures that must be done during the Screening Period:

Written informed consent must be obtained for each patient prior to any study-related procedure or change in medication for the purpose of this study.

The inclusion/exclusion criteria will be reviewed and used to determine each patient's potential eligibility for the study.

The time to reperfusion following AMI will be recorded, if applicable.

### Medical History, Physical Examination, Vital Signs, and Electrocardiogram

Medical history will include specific questions to allow identification of underlying cardiac disease.

Physical examination will include measurement of body weight and height, and assessment of presence or absence of pulmonary rales, gallop sound (S₃) with persistent tachycardia, and peripheral edema.

Vital signs will include measurement of seated heart rate and BP by cuff (sphygmomanometer).

12-Lead ECG will be performed.

### Documentation of Acute Myocardial Infarction, Left Ventricular Dysfunction, and Heart Failure

Documentation of the index AMI, (per Hall AS, Winter C, Bogle SM, Mackintosh AF, Murray GD, Ball SG on behalf of the AIRE Study Investigators. The acute infarction ramipril efficacy (AIRE) study: rationale, design, organization, and outcome definitions. J Cardiovasc Pharmacol 1991;18(Suppl 2):S105-S109), includes:
1. Cardiac enzyme rise:
   - total CPK >2xULN and/or
   - CPK-MB >10% of total CPK, AND
2. ECG diagnostic of MI (i.e., progressive ST segment and T wave compatible with AMI with or without the presence of pathological Q waves)

LV dysfunction is to be documented by LVEF ≤40% by echocardiogram, radionuclide angiography, or LV angiography determined following the index AMI and before randomization.

Documentation of HF, (per Hall AS, Winter C, Bogle SM, Mackintosh AF, Murray GD, Ball SG on behalf of the AIRE Study Investigators. The acute infarction ramipril efficacy (AIRE) study: rationale, design, organization, and outcome definitions. J Cardiovasc Pharmacol 1991;18(Suppl 2) :S105-S109) includes at least one of the following:
1. Pulmonary edema (bilateral posttussive crackles extending at least one-third of the way up the lung fields in the absence of significant chronic pulmonary disease);
2. Chest x-ray showing pulmonary venous congestion with interstitial or alveolar edema; OR
3. Auscultatory evidence of a third heart sound (S₃) with persistent tachycardia (>100 beats per minute).

Clinical evidence of HF post AMI can be transient, occurring at any time from the onset of the index AMI prior to randomization. Evidence of HF does not necessarily need to be present at the time of randomization.

### Clinical Laboratory Tests

Clinical safety laboratory tests will be performed for the following parameters. If possible, blood collection should occur in the morning:

| **Hematology** | |
|---|---|
| WBC with differential not acceptable) | Platelet count (estimate |
| RBC | |
| Hemoglobin | |
| Hematocrit | |

| **Biochemistry** | |
|---|---|
| Sodium | Glucose |
| Potassium | Alkaline phosphatase |
| Chloride transferase (γ-GT) | Gamma glutamyl |
| Calcium | SGOT (AST) |
| Magnesium | SGPT (ALT) |
| Phosphate (inorganic) | LDH |
| BUN (urea) phosphokinase (CPK) | Creatine |
| Creatinine only) | CPK-MB (screening |
| Total protein | Total cholesterol |
| Total bilirubin (direct and indirect) | LDL |
| Cholesterol | |
| Albumin | HDL Cholesterol |
| Uric acid | Triglycerides |

Serum Pregnancy Test for women of childbearing potential.

| **Urinalysis** | |
|---|---|
| pH | Protein |
| Specific gravity | Glucose |
| Ketones | Blood |

The Investigator will review all laboratory test results and initial each laboratory report. Any abnormal pretreatment values that require clinical intervention or that the Investigator considers clinically significant will exclude the patient from study participation. All laboratory tests will be performed by the designated central laboratory or local laboratory as appropriate. For sites using the central laboratory, instructions and materials for collecting and shipping of samples will be provided to each study site by the central laboratory.

### Killip Class

The patient's current Killip class, (per Killip T and Kimball JT. Treatment of myocardial infarction in a coronary care unit. A two year experience with 250 patients. Am J Cardiol 1967;20:457-467), will be determined as follows:
Class I: absence of rales and a third heart sound
Class II: rales up to 50% of each lung field or the presence of the third heart sound
Class III: rales in more than 50% of each lung field
Class IV: cardiogenic shock (resulting from decline in cardiac output secondary to serious heart disease, usually AMI)

### New York Heart Association (NYHA) Functional Classification

The patient's current NYHA functional classification will be determined, (per Silber EN, Katz LN. The physician and the cardiac patient. Chapter 12, p. 234 in Heart Disease. New York:MacMillan Publishing Co., Inc., 1975), as follows:
Class I: no symptoms with ordinary physical activity
Class II: symptoms with ordinary physical activity but not at rest
Class III: symptoms with less than ordinary physical activity but not at rest
Class IV: symptoms present at rest

### Quality of Life Assessments

Patients enrolled in the trial in the United States, United Kingdom, Spain, Canada, Brazil, Germany, Belgium, France, The Netherlands and Argentina will complete the following QOL assessments during the Screening period and before the first dose of study medication.

### 1. The Kansas City Cardiomyopathy Questionnaire (KCCQ) :

This disease-specific instrument for patients with HF has been extensively tested and validated to ensure the accuracy of its assessments. It quantifies the full range of health status as impacted upon by the syndrome of HF. The 23-item KCCQ specifically quantifies symptoms (their frequency, severity, and change over time), function (physical and social), and quality of life. Disease-specific measures have repeatedly been demonstrated to be more sensitive to clinical change than generic health status measures and the KCCQ should provide a robust assessment of eplerenone's impact on the health status of patients. (Guyatt GH, Feeny DH, Patrick DL. Measuring health-related quality of life. Ann Intern Med 1993;118:622-629; Spertus JA, Winder JA, Dewhurst TA, Deyo RA, Fihn SD. Monitoring the quality of life in patients with coronary artery disease. Am J Cardiol 1994;74:1240-1244)

### 2. The Short Form - 12 Health Survey (SF-12):

The SF-12 is a generic 12-item questionnaire that can generate an overall summary score of physical and mental health. (Ware J Jr., Kosinksi M, Keller SD. A 12-item short-form health survey: construction of scales and preliminary tests of reliability and validity. Med Care 1996;34:220-233; Jenkinson C, Layte R, Jenkinson D, et al. A shorter form health survey: can the SF-12 replicate results from the SF-36 longitudinal studies? J Public Health Med 1997;19:179-186; Gandek B, Ware JE, Aaronson NK, et al. Cross-validation of item selection and scoring for the SF-12 Health Survey in nine countries: results from the IQOLA Project. International Quality of Life Assessment Project. J Clin Epidemiol 1998;51:1171-1178). Unlike the KCCQ, it is not specific for HF and will capture the limitations in health posed by other comorbid conditions. Nevertheless, population norms for this instrument are available and will allow benchmarking of the patient population against other studies (including those for the treatment of different diseases).

### 3. The EuroQoL Health Rating Scale:

The EuroQoL quantifies three levels of function in five different generic domains. (Kind P. The EuroQoL instrument: An index of health-related quality of life. In: Spilker B, ed. Quality of life and pharmacoeconomics in clinical trials. 2^{nd} edition. Philadelphia: Lippincott-Raven; 1996:191-201.) With the inclusion of a "feeling thermometer": it is composed of six questions and can efficiently synthesize the range of health status into a single number (utility) that will be used for the planned economic analyses of this trial. (Torrence GW. Measurement of health state utilities for economic appraisal. J Health Econ 1986;5:1-30).

### 4. The Medical Outcomes Study Depression Scale (MOS-D):

The MOS-D was developed for use in the National Study of Medical Outcomes as a screening device for depressive disorders in a medical patient population. (Burnham MA, Wells KB, Leake B, Landsverk J.
Development of a brief screening instrument for detecting depressive disorders. Med Care 1988;26:775-789). It consists of eight items that were incorporated items from the Diagnostic Interview Schedule (DIS) and the Center for Epidemiological Studies Depression Scale (CES-D), which do not use somatic indicators of depression, a potential confounding marker of depression among patients with HF. The MOD-D has excellent sensitivity (93% (95% CI 86-97)) and good specificity (72% (95% CI 68-76)). The MOS-D will be used both for discriminating clinically relevant subsets at baseline (i.e., depressed vs. nondepressed patients) for the secondary and tertiary objectives of defining baseline predictors of greater benefit (both mortality and health status outcomes) from eplerenone and as an assessment of the responsiveness of depression to treatment with eplerenone - an additional secondary objective of this study.

### 5. Brief Symptom Inventory-Anxiety (BSI-A):

The BSI-A is a shorter alternative to the Symptom Checklist 90 - Revised. It is comprised of six items that measure anxiety. (Derogatis LR, Melisaratos N. The Brief Symptom Inventory: an introductory report. Psychol Med 1983;13:595-605). Like the MOS-D it has the advantage of not using physical indicators of emotional states, which can overestimate the level of mood states in patients with cardiovascular disease. The BSI-A has high internal consistency (Cronbach's alpha = 0.85) and well established construct, convergent, discriminant, and predictive validity. Given the emerging literature on the association between anxiety and cardiovascular disease, baseline anxiety will be used to evaluate differential effects of eplerenone therapy on health status and mortality outcomes (secondary and tertiary objectives of this study). The brevity and excellent psychometric properties of the BSI-A make this measure an exceptional one for broaching this new area of investigation.

### Blood Sample for DNA Analysis

A 30 mL blood sample for DNA analysis will be obtained. From this sample, 20 mL will be put into tubes containing EDTA and 10 mL will be put into tubes containing sodium citrate. The tubes will be placed at 4°C until courier collection, and shipped immediately by courier to the designated laboratory.

### Admission of Patients

After screening, eligible patients are to be given study numbers in sequence. In addition, they are to be identified by first, middle, and last initials. If the patient has no middle initial, a dash is to be used.

### Treatment Period

### Concurrent Medications

Potassium-sparing diuretics and spironolactone are prohibited for the duration of this study. There are no other restrictions on concomitant medications in this trial. Any medication is permitted if, in the opinion of the Investigator, it is necessary. Patients should avoid any additional medication (including over-the-counter [OTC] drugs) without prior approval of the Investigator.

For all concomitant medications, the start and stop dates, as well as the reason for use, must be recorded on the appropriate CRF. For selected concomitant medications (including ACE-I, All antagonists, antiarrhythmics, anticoagulants, antiplatelet agents, β-blockers, calcium channel blockers, digoxin, diuretics, magnesium supplements, potassium supplements, and α-blockers), the dose, as well as start and stop dates must be recorded on the appropriate CRF. All changes in these selected concomitant medications must be recorded on the appropriate CRF.

### Visit 1 Baseline and Randomization (Day 0)

Randomization must occur between 3 days (>48 hours) to 10 days after the onset of an AMI (earliest onset of symptoms), preferably prior to discharge from the hospital.

At Visit 1 (Day 0) patients will be assessed for eligibility for the Treatment Period. Visit 1 assessments include:
1. Seated heart rate and seated cuff BP.
2. Determination of NYHA functional classification.
3. Recording of concurrent medications.
4. Inclusion/exclusion criteria determination.

Each eligible patient will be assigned the next available four-digit patient number and will receive the treatment assigned to that number by a computer-generated randomization schedule prepared at Searle prior to the start of the study.

Patients will receive a four-week supply of double-blind study medication and, again, be instructed to take one tablet every morning with water, or as otherwise directed by the Investigator.

### Safety Laboratory Assessments (48 hours Post-Randomization)

Serum potassium level will be determined at 48 hours after the initiation of dosing. If possible, blood for this measure should be drawn in the morning.

If at any time during the study, the serum potassium level is >5.5 mEq/L but <6.0 mEq/L, the dose of study medication will be reduced to the next lower dose, e.g., 25 mg QD to 25 mg QOD, 25 mg QOD to temporarily withheld. Serum potassium level is to be determined within one week after each dose adjustment. Dose adjustments are to be made based on the most recent serum potassium level. If at any time during the study the serum potassium level is ≥6.0 mEq/L, study medication is to be temporarily withheld. If study medication is temporarily withheld, it may be restarted at one tablet QOD when the serum potassium is <5.5 mEq/L and titrated as directed in Study Drug Administration, Table 16. See *Study Drug Administration* for detailed dosing instructions and for instructions for restarting study medication. If at any time during the study the serum potassium level is persistently ≥6.0 mEq/L, study medication is to be permanently discontinued.

### Visit 2 (1 Week ± 3 Days Post-Randomization)

The following procedures will be performed after one week of treatment:
1. Seated heart rate and seated cuff BP.
2. Blood draw for serum potassium level. If at all possible, blood should be collected in the morning.
3. Determination of NYHA functional classification Assessment of endpoints.
4. Recording of any new concurrent medications and any dose changes for selected medications. Recording of any adverse events.
5. Counting of returned medications and recording of compliance.

If at any time during the study, the serum potassium level is >5.5 mEq/L but <6.0 mEq/L, the dose of study medication will be reduced to the next lower dose, e.g., 50 mg QD (two tablets) to 25 mg QD (one tablet), 25 mg QD to 25 mg QOD, 25 mg QOD to temporarily withheld. Serum potassium level is to be determined within one week after each dose adjustment. Dose adjustments are to be made based on the most recent serum potassium level. If at any time during the study the serum potassium level is ≥6.0 mEq/L, study medication is to be temporarily withheld. If study medication is temporarily withheld, it may be restarted at one tablet QOD when the serum potassium is <5.5 mEq/L and titrated as directed in *Study Drug Administration*, Table 16. See *Study Drug Administration* for detailed dosing instructions and for instructions for restarting study medication. If at any time during the study the serum potassium level is persistently ≥6.0 mEq/L, study medication is to be permanently discontinued.

### Visit 3 (4 Weeks ± 3 Days Post-Randomization)

The following procedures will be performed after four weeks of treatment:
1. Seated heart rate and seated cuff BP.
2. Clinical safety laboratory blood draw including potassium.
3. Clinical safety urine sample for urinalysis.
4. Determination of NYHA functional classification .
5. Assessment of endpoints.
6. QOL assessments.
7. Recording of any new concurrent medications and any dose changes for selected medications.
8. Recording of any adverse events.
9. Counting of returned medications and recording of compliance.
10. Dispensing of a three-month supply of study medication with medication diary card.

At this visit, the patient will be instructed to continue on 25 mg QD eplerenone/placebo. If the serum potassium level measured at this visit is <5.0 mEq/L, the site will contact the patient and instruct the patient to increase to dose to two tablets per day. If the serum potassium is ≥5.0 mEq/L at Week 4 but <5.0 mEq/L at Week 5, the dose of study drug will be increased to 50 mg QD (two tablets). In this case, serum potassium is to be checked at Week 6. If at any time during the study, the serum potassium level is >5.5 mEq/L but <6.0 mEq/L, the dose of study medication will be reduced to the next lower dose, e.g., 50 mg QD (two tablets) to 25 mg QD (one tablet), 25 mg QD to 25 mg QOD, 25 mg QOD to temporarily withheld. Serum potassium level is to be determined within one week after each dose adjustment. Dose adjustments are to be made based on the most recent serum potassium level. If at any time during the study the serum potassium level is ≥6.0 mEq/L, study medication is to be temporarily withheld. If study medication is temporarily withheld, it may be restarted at one tablet QOD when the serum potassium is <5.5 mEq/L and titrated as directed in *Study Drug Administration,* Table 16. See *Study Drug Administration* for detailed dosing instructions and for instructions for restarting study medication. If at any time during the study the serum potassium level is persistently ≥6.0 mEq/L, study medication is to be permanently discontinued.

### Safety Laboratory Assessment (5 Weeks Post-Randomization)

Serum potassium level is to be determined at Week 5 for all patients. For patients whose dose level was not increased at Week 4, the dose of study medication may be increased to two tablets QD if the serum potassium is <5.0 mEq/L. If the dose is increased at this visit, the serum potassium level is to be determined within one week. If possible, blood for this measure should be drawn in the morning.

If at any time during the study, the serum potassium level is >5.5 mEq/L but <6.0 mEq/L, the dose of study medication will be reduced to the next lower dose, e.g., 50 mg QD (two tablets) to 25 mg QD (one tablet), 25 mg QD to 25 mg QOD, 25 mg QOD to temporarily withheld. Serum potassium level is to be determined within one week after each dose adjustment. Dose adjustments are to be made based on the most recent serum potassium level. If at any time during the study the serum potassium level is ≥6.0 mEq/L, study medication is to be temporarily withheld. If study medication is temporarily withheld, it may be restarted at one tablet QOD when the serum potassium is <5.5 mEq/L and titrated as directed in *Study Drug Administration,* Table 16. See *Study Drug Administration* for detailed dosing instructions and for instructions for restarting study medication. If at any time during the study the serum potassium level is persistently ≥6.0 mEq/L, study medication is to be permanently discontinued.

### Visits 4 onward (3 Months ± 10 Days Post-Randomization and Every 3 Months Thereafter)

The following procedures will be performed after three months of treatment and every three months thereafter:
1. Seated heart rate and seated cuff BP.
2. Clinical safety laboratory blood draw including potassium and clinical safety urine sample for urinalysis will be performed at Months 3, 6, 12, 18, 24, and every six months thereafter as long as the study continues. A blood draw for serum potassium only will be performed at Months 9, 15, 21, and every three months thereafter as long as the study continues.
3. Determination of NYHA functional classification.
4. Assessment of endpoints.
5. QOL assessments will be performed at Months 3, 6, and 12.
6. Recording of any new concurrent medications and any dose changes for selected medications.
7. Recording of any adverse events.
8. Counting of returned medications and recording of compliance.
9. Dispensing of a three-month supply of study medication with medication diary card.

If at any time during the study, the serum potassium level is >5.5 mEq/L but <6.0 mEq/L, the dose of study medication will be reduced to the next lower dose, e.g., 50 mg QD (two tablets) to 25 mg QD (one tablet), 25 mg QD to 25 mg QOD, 25 mg QOD to temporarily withheld. Serum potassium level is to be determined within one week after each dose adjustment. Dose adjustments are to be made based on the most recent serum potassium level. If at any time during the study the serum potassium level is ≥6.0 mEq/L, study medication is to be temporarily withheld. If study medication is temporarily withheld, it may be restarted at one tablet QOD when the serum potassium is <5.5 mEq/L and titrated as directed in *Study Drug Administration,* Table 16. See *Study Drug Administration* for detailed dosing instructions and for instructions for restarting study medication. If at any time during the study the serum potassium level is persistently ≥6.0 mEq/L, study medication is to be permanently discontinued.

### Final Visit or Early Cessation of Study Medication

The following procedures will be performed at the final visit:
1. Seated heart rate and seated cuff BP.
2. Physical examination.
3. 12-lead electrocardiogram.
4. Clinical safety laboratory blood draw including potassium.
5. Clinical safety urine sample for urinalysis.
6. Determination of NYHA functional classification.
7. Assessment of endpoints.
8. QOL assessments.
9. Recording of any new concurrent medications and any dose changes for selected medications.
10. Recording of any adverse events.
11. Counting of returned medications and recording of compliance.
Any abnormal findings at the final assessment should be followed by the Investigator until satisfactorily resolved.

### Permanent Discontinuation of Study Medication Prior to Study Termination

If for any reason a patient permanently discontinues study medication before the study is terminated, the reason for such discontinuation must be entered on the Permanent Cessation of Study Drug Form. The patient's permanent discontinuation of study medication must also be registered with the IVRS center by the Coordinator or Investigator. The patient must undergo procedures described in Section 4.3.h, Final Visit or Early Cessation of Study Medication, and appropriate CRFs must be completed. All data for the patient prior to cessation of study medication will be made available to G.D. Searle & Co., and the patient will continue to be followed every three months by telephone for endpoints until the end of the study.

A patient may permanently discontinue study medication for any of the following:
1. Inability to tolerate study medication.
2. Persistent serum potassium level ≥6.0 mEq/L when the patient is receiving the lowest dose (1 tablet QOD).
3. Pregnancy.
4. Administrative reasons.
5. Any other reason which in the opinion of the Investigator is to protect the best interest of the patient.
6. Request of the patient to withdraw. The patient has the right to withdraw at any time for any reason.
7. Treatment with spironolactone.

It is understood by all that excessive stopping of study medication can render the study uninterpretable; therefore, unnecessary medication stoppage should be avoided. Clear description of trial procedures to patients and their understanding of the Informed Consent Form is not only mandatory, but crucial to limiting unnecessary medication stoppage.

### STATISTICS

### Justification of Sample Size

### Sample Size and Mortality Assumptions

Randomized patients will be followed until 1,012 deaths have occurred. This number will provide over 90% power to detect an 18.5% reduction in the rate of death compared to the placebo group. When the first-year placebo mortality rate in the placebo group is 15% or greater and up to 6,200 patients are enrolled over an 18-month period, the target number of 1,012 deaths should occur within the first 30 months of the trial (18 month enrollment plus 12 months follow-up after the last patient is enrolled).

In order to estimate the required number of patients and duration of follow-up to achieve this number of deaths, data from three trials were considered. In these trials, the one-year mortality rate for the groups receiving active treatment ranged from 12% to 23%. In the AIRE trial, conducted in several European countries, and the TRACE trial, conducted in Denmark, the first-year mortality rates in the active (ACE-I) arms were 16% and 23%, respectively. (The Acute Infarction Ramipril Efficacy (AIRE) Study Investigators. Effect of ramipril on mortality and morbidity of survivors of acute myocardial infarction with clinical evidence of heart failure. Lancet 1993;342:821-828; Køber L, Torp-Pedersen C, Carlsen JE, Bagger H, Eliasen P, Lyngborg K, Videbæk J, Cole DS, Auclert L, Pauly NC, Aliot E, Persson S, Camm AJ for the Trandolapril Cardiac Evaluation (TRACE) Study Group. N Engl J Med 1995;333:1670-1676). In the AIRE trial, among all patients randomized who were not receiving digoxin, the first-year rate was about 12% (about 17% for patients receiving digoxin). (Spargias KS, Hall AS, Ball SG. Safety concerns about digoxin after acute myocardial infarction. Lancet 1999;354:391-392). In the GISSI-3 trial, of patient randomized to lisinopril who were alive at Day 3 with Killip class >I, the first-year mortality was 22%; for these patients ACE-I treatment was stopped at 42 days, but more than 50% of them continued treatment long-term. The table below gives the required number of patients and duration of follow-up based on three different estimates for the one-year placebo mortality rate (12%, 15%, and 18%). These results were obtained using software described by Shih, 1995. (Shih JH. Sample size calculation for complex clinical trials with survival endpoints. Controlled Clinical Trials 1995;16:395-407).

The calculations assume a decreasing hazard of mortality, similar to that seen in the active treatment arm of the AIRE trial. Similar results were obtained by using results from the TRACE trial. The active rather than placebo arms of these ACE-I trials were used because the current trial will allow background medication including ACE-I for all patients. It is also assumed that the hazard ratio between the two treatment arms is constant over time (proportional hazards) and that a greater rate of recruitment will occur in the final 12 months of the enrollment period than in the initial six months.

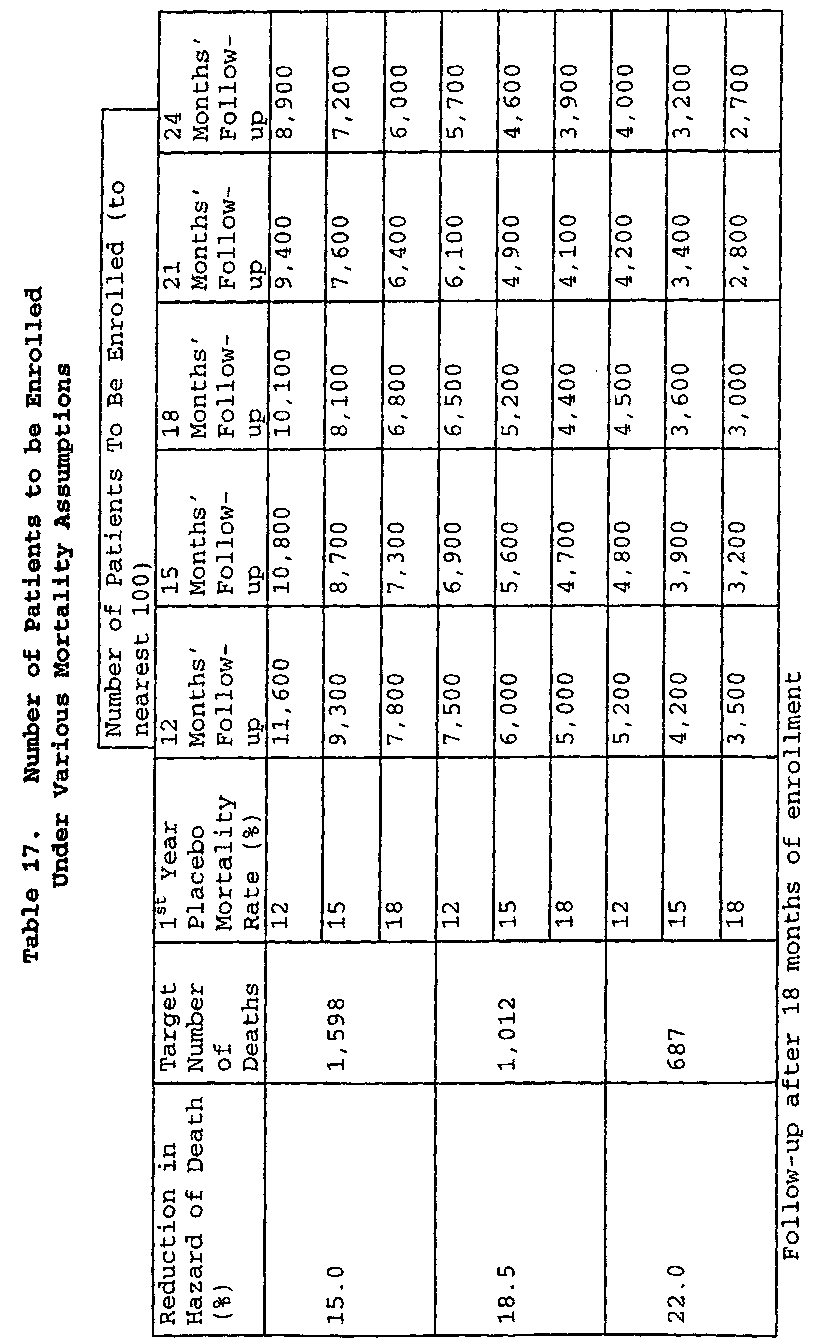

The mortality reduction over the course of the study was 27% in the AIRE trial and 22% in TRACE. Over the course of the current trial, a smaller treatment effect is expected than in the earlier trials, because the active treatment arms in the earlier trials are expected to be more similar to the placebo arm in this trial. Therefore, reductions in first-year mortality of 15%, 18.5% and 22% were considered in the sample size scenarios. Note that in the recent RALES trial, the active agent, with the same mechanism of action as eplerenone, achieved over a 30% reduction in mortality compared to placebo, albeit in a different patient population. (Pitt B, Zannad F, Remme WJ, Cody R, Castaigne A, Perez A, Palensky J, Wittes J for the Randomized Aldactone Evaluation Study Investigators. The effect of spironolactone on morbidity and mortality in patients with severe heart failure. N Engl J Med 1999; 341(10):709-717). In the RALES trial, most patients in each treatment arm received standard therapy in addition to their study medication.

### Loss to follow-up

Sample size estimates have not been adjusted for loss-to-follow-up, because study management procedures are expected to keep the loss below 1%.

### Baseline and Demographic Analysis

Comparability of treatment groups with respect to baseline and demographic factors will be examined using t-tests for continuous variables and chi-square tests for categorical variables.

### Efficacy Analysis

### Primary and Secondary Endpoints

All statistical analyses of the primary and secondary endpoints will follow the intention-to-treat principle. Data from randomized patients will be analyzed according to the patients' original treatment assignment, and all patients will be followed for mortality and other major endpoints for the duration of the study, regardless of compliance with taking study medication.

For each endpoint, the time-to-event will be analyzed using the logrank test at the overall 0.05 level, accounting as needed for interim analyses. To be included in the statistical analysis, any adjudicatable endpoint event must be adjudicated by the Critical Events Committee. Kaplan-Meier curves will be used to summarize the various time-to-event distributions. Exploratory analyses of these endpoints using baseline characteristics as covariates may be performed using Cox proportional hazards regression to estimate relative hazard rates and 95% confidence intervals.

The logrank tests and Cox regression analyses will be stratified by geographic region. The regions will consist of the United States and Canada; Latin America; Eastern Europe; and Western Europe, which will also include Australia, New Zealand, Israel, and South Africa.

### Quality of Life

Appropriate statistical methods will be used to analyze QOL. Instruments to be analyzed include the KCCQ, SF-12, the EuroQoL Health Rating Scale, MOS-D depression scale, and BSI-A anxiety scale.

### Subgroup Analyses

Subgroup analyses of the primary and secondary endpoints will be performed. Subgroups will be based on baseline recordings of race (black, non-black), sex, age, presence of diabetes, ejection fraction, serum potassium, serum creatinine, use of β-blockers, use of digoxin, use of potassium supplements, first versus subsequent AMI, Killip class, reperfusion status, history of hypertension, history of HF, history of smoking, history of angina, time from index AMI to randomization, and geographic region. Subgroups based on continuous measures such as age, ejection fraction, serum potassium, and serum creatinine will be dichotomized at the median value.

### Safety Analysis

All patients who receive at least one dose of study medication will be included in the analysis of safety. Routine safety data will be collected only for patients still taking study medication.

### Symptoms and Adverse Events

All adverse events will be coded and summarized by treatment group. The incidence of treatment emergent adverse events will be tabulated by treatment group and body system as well as by severity and attribution. In addition, the incidence of adverse events causing discontinuation of study medication and serious adverse events will be summarized by treatment group. Only those adverse and serious adverse events that occur within 30 days after a patient's last dose of study medication will be included in the analysis of safety.

### Vital Signs

Vital signs will be listed and summarized by scheduled time and treatment. Changes from baseline in heart rate and BP will be analyzed by analysis of covariance, with baseline value as a covariate.

### Clinical Laboratory Tests

Clinical laboratory data will be summarized and treatment groups will be compared. Within treatment group changes from baseline to post-treatment will be analyzed using a paired t-test. Differences between treatment groups will be evaluated using analysis of covariance with baseline value as a covariate. Shift tables will be used to graphically depict the shift in laboratory values. These shift tables will capture those laboratory values that are clinically relevantly high or low at either baseline or post-treatment. The incidence of clinically relevant laboratory results will be tabulated by treatment group.

### Committees

In conjunction with the Sponsor, the Steering Committee, comprised of the lead Investigators from each participating country or region, will oversee the trial. There will also be an independent Data Safety Monitoring Board. All endpoints will be adjudicated by a Critical Events Committee.

### Steering Committee

The Steering Committee will be comprised of the lead Investigators of each participating country or region, the Sponsor, and an independent statistician. It will remain blinded to the trial results through the conduct of the trial. It will oversee the conduct and reporting of the trial, including developing the network of Investigators, assuring expert clinical guidance and a high standard of scientific quality and making any necessary modifications to the protocol. The Steering Committee Charter will define the responsibilities of the committee.

### Critical Events Committee (CEC)

The purpose of the independent Critical Events Committee (CEC) is to classify the cause of all endpoint events for each patient during this trial. This committee will review documentation from each suspected endpoint event and will remain blinded to treatment assignments. The CEC will assess the nature of each event and determine whether the pre-specified criteria for the endpoints were met. The CEC Charter will define the responsibilities of the committee.

### Data Safety Monitoring Board (DSMB) and Interim Analyses

An independent DSMB will be impaneled to monitor the safety and efficacy of the trial and to determine whether sufficient treatment differences exist to terminate the trial prematurely. The DSMB will consist of five members: four cardiologists, expert in the diagnosis of heart failure and its progression; and one medical statistician, expert in the analysis of clinical trial data. One member will serve as chairperson. No members of the DSMB will act as Investigators for the study. The DSMB Charter will define the responsibilities of the committee.

Decisions on early study closure for efficacy will guided by an O'Brien-Fleming-type boundary with a Lan-DeMets alpha spending function. Recommendations of the DSMB will be reported to the Chairman of the Steering Committee responsible for enacting the discontinuation.

To maintain the blind at Searle and among other study participants, an external statistical group will prepare all aspects of the interim reports and communicate directly with the DSMB.

### IVRS Center

An IVRS center will be used for treatment assignment and tracking of recruitment, withdrawal and medication supplies.

## Claims

1. A combination comprising a therapeutically-effective amount of an epoxy-steroidal aldosterone receptor antagonist and a therapeutically-effective amount of an angiotensin converting enzyme inhibitor, wherein said angiotensin converting enzyme inhibitor is selected from the group consisting of alacepril, benazepril, captopril, cilazapril, delapril, enalapril, enalaprilat, fosinopril, fosinoprilat, imidapril, lisinopril, perindopril, quinapril, ramipril, saralasin acetate, temocapril, trandolapril, ceranapril, moexipril, quinaprilat, spirapril, Bioproject BP1.137, Chiesi CHF 1514, Fisons FPL-66564, idrapril, Marion Merrell Dow MDL-100240, perindoprilat, and Servier S-5590.

2. The combination of claim 1 wherein said epoxy-steroidal aldosterone receptor antagonist is present in an amount which is therapeutically effective to antagonize aldosterone but which amount is not sufficient for said aldosterone receptor antagonist to induce a substantially adverse side effect.

3. The combination of claim 1 wherein said angiotensin converting enzyme inhibitor is selected from the group consisting of alacepril, benazepril, captopril, cilazapril, delapril, enalapril, enalaprilat, fosinopril, fosinoprilat, imidapril, lisinopril, perindopril, quinapril, ramipril, saralasin acetate, temocapril, trandolapril, ceranapril, moexipril, quinaprilat, and spirapril.

4. The combination of claim 1 wherein said aldosterone receptor antagonist is an epoxy-steroidal-type compound **characterized in** having a 9α-, 11α-substituted epoxy moiety.

5. The combination of claim 4 wherein said epoxy-steroidal-type compound is eplerenone.

6. The combination of claim 1 further **characterized by** said angiotensin converting enzyme inhibitor and said aldosterone receptor antagonist being present in said combination in a weight ratio range from 0.5-to-one to twenty-to-one of said angiotensin converting enzyme inhibitor to said aldosterone receptor antagonist.

7. The combination of claim 6 wherein said weight ratio range is from one-to-one to fifteen-to-one.

8. The combination of claim 7 wherein said weight ratio range is from one-to-one to five-to-one.

9. Use of a combination comprising a therapeutically-effective amount of a two-component combination consisting essentially of an epoxy-steroidal aldosterone receptor antagonist, as a first component, and an angiotensin converting enzyme inhibitor as a second component, wherein said angiotensin converting enzyme inhibitor is selected from the group consisting of alacepril, benazepril, captopril, cilazapril, delapril, enalapril, enalaprilat, fosinopril, fosinoprilat, imidapril, lisinopril, perindopril, quinapril, ramipril, saralasin acetate, temocapril, trandolapril, ceranapril, moexipril, quinaprilat, spirapril, Bioproject BP1.137, Chiesi CHF 1514, Fisons FPL-66564, idrapril, Marion Merrell Dow MDL-100240, perindoprilat, and Servier S-5590 for the preparation of a medicament for treating cardiovascular disorders in a subject afflicted with or susceptible to multiple cardiovascular disorders.

10. The use of the combination of claim 9 wherein said epoxy-steroidal aldosterone receptor antagonist is present in an amount therapeutically effective to antagonize aldosterone but insufficient to induce an adverse side effect.

11. 'The use of the combination of claim 9 wherein said angiotensin converting enzyme inhibitor is selected from the group consisting of alacepril, benazepril, captopril, cilazapril, delapril, enalapril, enalaprilat, fosinopril, fosinoprilat, imidapril, lisinopril, perindopril, quinapril, ramipril, saralasin acetate, temacapril, trandolapril, ceranapril, moexipril, quinaprilat, and spirapril.

12. The use of the combination of claim 9 wherein
said angiotensin converting enzyme inhibitor and said epoxy-steroidal aldosterone receptor antagonist are to be administered in a sequential manner.

13. The use of the combination of claim 9 wherein
said angiotensin converting enzyme inhibitor and said epoxy-steroidal aldosterone receptor antagonist are to be administered in a substantially simultaneous manner.

14. The use of the combination of claim 9 wherein said epoxy-steroidal aldosterone receptor antagonist is a compound **characterized in** having a 9α-,11α-substituted epoxy moiety.

15. The use of the combination of claim 14 wherein said epoxy-steroidal compound is eplerenone.

16. The use of the combination of claim 9 further **characterized in that** said angiotensin converting enzyme inhibitor and said epoxy-steroidal aldosterone receptor antagonist are to be used in said co-therapy in a weight ratio range from 0.5-to-one to twenty-to-one of said angiotensin converting enzyme inhibitor to said aldosterone receptor antagonist.

17. The use of the combination of claim 16 wherein said weight ratio range is from one-to-one to fifteen-to-one.

18. The use of the combination of claim 17 wherein said weight ratio range is from one-to-one to five-to-one.

19. The use of the combination of claim 9 wherein said angio-tensin converting enzyme inhibitor is captopril, in a dose range from 40 mg to 80 mg per dose, or is enalapril in a dose range from 5 mg to 25 mg per dose.

20. The use of the combination of claim 9 wherein said angiotensin converting enzyme inhibitor is ramipril, in a dose range from 2 mg to 20 mg per dose, or is lisinopril in a dose range from 5 mg to 25 mg per dose.

21. The use of the combination as in claims 9, 19 or 20 wherein said epoxy-steroidal aldosterone receptor antagonist is eplerenone in a dose range from 25 mg to 100 mg per dose.

## Patentansprüche

1. Kombination, umfassend eine therapeutisch wirksame Menge eines Epoxy-steroidalen Aldosteron-Rezeptorantagonisten und eine therapeutisch wirksame Menge eines Inhibitors für das Angiotensin-umwandelnde Enzym, wobei der genannte Inhibitor für das Angiotensin-umwandelnde Enzym aus der Gruppe, bestehend aus Alacepril, Benazepril, Captopril, Cilazapril, Delapril, Enalapril, Enalaprilat, Fosinopril, Fosinoprilat, Imidapril, Lisinopril, Perindopril, Chinapril, Ramipril, Saralasinacetat, Temocapril, Trandolapril, Ceranapril, Moexipril, Chinaprilat, Spirapril, Bioproject BP1.137, Chiesi CHF 1514, Fisons FPL-66564, Idrapril, Marion Merrell Dow MDL-100240, Perindoprilat und Servier S-5590, ausgewählt worden ist.

2. Kombination nach Anspruch 1, **dadurch gekennzeichnet, dass** der genannte Epoxy-steroidale Aldosteron-Rezeptorantagonist in einer Menge vorliegt, die therapeutisch wirksam ist um das Aldosteron zu antagonisieren, wobei diese Menge jedoch nicht ausreichend ist, dass der genannte Aldosteron-Rezeptorantagonist eine wesentliche nachteilige Nebenwirkung bewirkt.

3. Kombination nach Anspruch 1, **dadurch gekennzeichnet, dass** der Inhibitor für das Angiotensin-umwandelnde Enzym aus der Gruppe, bestehend aus Alacepril, Benazepril, Captopril, Cilazapril, Delapril, Enalapril, Enalaprilat, Fosinopril, Fosinoprilat, Imidapril, Lisinopril, Perindopril, Chinapril, Ramipril, Saralasinacetat, Temocapril, Trandolapril, Ceranapril, Moexipril, Chinaprilat und Spirapril, ausgewählt worden ist.

4. Kombination nach Anspruch 1, **dadurch gekennzeichnet, dass** der genannte Aldosteron-Rezeptorantagonist eine Verbindung vom Epoxy-steroidalen Typ ist, die dadurch charakterisiert ist, dass sie eine 9α-,11α-substituierte Epoxygruppierung hat.

5. Kombination nach Anspruch 4, **dadurch gekennzeichnet, dass** die genannte Verbindung vom Epoxy-steroidalen Typ Eplerenon ist.

6. Kombination nach Anspruch 1, weiterhin **dadurch gekennzeichnet, dass** der genannte Inhibitor für das Angiotensin-umwandelnde Enzym und der genannte Aldosteron-Rezeptorantagonist in der genannten Kombination in einem Gewichtsverhältnis von 0,5:1 bis 20:1 des genannten Inhibitors für das Angiotensin-umwandelnde Enzym zu dem genannten Aldosteron-Rezeptorantagonist vorhanden sind.

7. Kombination nach Anspruch 6, **dadurch gekennzeichnet, dass** das genannte Gewichtsverhältnis im Bereich von 1:1 bis 15:1 liegt.

8. Kombination nach Anspruch 7, **dadurch gekennzeichnet, dass** das genannte Gewichtsverhältnis im Bereich von 1:1 bis 5:1 liegt.

9. Verwendung einer Kombination, umfassend eine therapeutisch wirksame Menge einer Zweikomponentenkombination, bestehend im Wesentlichen aus einem Epoxy-steroidalen Aldosteron-Rezeptorantagonisten als erste Komponente und einem Inhibitor für das Angiotensin-umwandelnde Enzym als eine zweite Komponente, wobei der genannte Inhibitor für das Angiotensin-umwandelnde Enzym aus der Gruppe, bestehend aus Alacepril, Benazepril, Captopril, Cilazapril, Delapril, Enalapril, Enalaprilat, Fosinopril, Fosinoprilat, Imidapril, Lisinopril, Perindopril, Chinapril, Ramipril, Saralasinacetat, Temocapril, Trandolapril, Ceranapril, Moexipril, Chinaprilat, Spirapril, Bioproject BP1.137, Chiesi CHF 1514, Fisons FPL-66564, Idrapril, Marion Merrell Dow MDL-100240, Perindoprilat und Servier S-5590 ausgewählt worden ist, zur Herstellung eines Medikaments zur Behandlung von kardiovaskulären Störungen bei einem Patienten, der an mehrfachen kardiovaskulären Störungen leidet oder dafür empfindlich ist.

10. Verwendung der Kombination nach Anspruch 9, **dadurch gekennzeichnet, dass** der genannte Epoxy-steroidale Aldosteron-Rezeptorantagonist in einer therapeutisch wirksamen Menge vorhanden ist um das Aldosteron zu antagonisieren, jedoch nicht ausreichend ist, eine nachteilige Nebenwirkung zu bewirken.

11. Verwendung der Kombination nach Anspruch 9, **dadurch gekennzeichnet, dass** der genannte Inhibitor für das Angiotensin-umwandelnde Enzym aus der Gruppe Alacepril, Benazepril, Captopril, Cilazapril, Delapril, Enalapril, Enalaprilat, Fosinopril, Fosinoprilat, Imidapril, Lisinopril, Perindopril, Chinapril, Ramipril, Saralasinacetat, Temocapril, Trandolapril, Ceranapril, Moexipril, Chinaprilat und Spirapril ausgewählt wird.

12. Verwendung der Kombination nach Anspruch 9, **dadurch gekennzeichnet, dass** der Inhibitor für das Angiotensin-umwandelnde Enzym und der genannte Epoxy-steroidale Aldosteron-Rezeptorantagonist in sequentieller Art und Weise verabreicht werden sollen.

13. Verwendung der Kombination nach Anspruch 9, **dadurch gekennzeichnet, dass** der genannte Inhibitor für das Angiotensin-umwandelnde Enzym und der genannte Epoxy-steroidale Aldosteron-Rezeptorantagonist in im Wesentlichen gleichzeitiger Weise verabreicht werden sollen.

14. Verwendung der Kombination nach Anspruch 9, **dadurch gekennzeichnet, dass** der Epoxy-steroidale Aldosteron-Rezeptorantagonist eine Verbindung ist, die dadurch charakterisiert ist, dass sie eine 9α-,11α-substituierte Epoxygruppierung hat.

15. Verwendung der Kombination nach Anspruch 14, **dadurch gekennzeichnet, dass** die genannte Epoxy-steroidale Verbindung Eplerenon ist.

16. Verwendung der Kombination nach Anspruch 9, weiterhin **dadurch gekennzeichnet, dass** der Inhibitor für das genannte Angiotensin-umwandelnde Enzym und der genannte Epoxy-steroidale Aldosteron-Rezeptorantagonist bei der genannten Co-Therapie in einem Gewichtsverhältnis im Bereich von 0,5:1 bis 20:1 des genannten Inhibitors für das Angiotensin-umwandelnde Enzym zu dem genannten Aldosteron-Rezeptorantagonist verwendet werden sollen.

17. Verwendung der Kombination nach Anspruch 16, **dadurch gekennzeichnet, dass** das genannte Gewichtsverhältnis im Bereich von 1:1 bis 15:1 liegt.

18. Verwendung der Kombination nach Anspruch 17, **dadurch gekennzeichnet, dass** das genannte Gewichtsverhältnis im Bereich von 1:1 bis 5:1 liegt.

19. Verwendung der Kombination nach Anspruch 9, **dadurch gekennzeichnet, dass** der genannte Inhibitor für das Angiotensin-umwandelnde Enzym Captopril in einem Dosisbereich von 40 mg bis 80 mg pro Dosis oder Enalapril in einem Dosisbereich von 5 mg bis 25 mg pro Dosis ist.

20. Verwendung der Kombination nach Anspruch 9, **dadurch gekennzeichnet, dass** der Inhibitor für das genannte Angiotensin-umwandelnde Enzym Ramipril in einem Dosisbereich von 2 mg bis 20 mg pro Dosis oder Lisinopril in einem Dosisbereich von 5 mg bis 25 mg pro Dosis ist.

21. Verwendung der Kombination nach den Ansprüchen 9, 19 oder 20, **dadurch gekennzeichnet, dass** der genannte Epoxy-steroidale Aldosteron-Rezeptorantagonist Eplerenon in einem Dosisbereich von 25 mg bis 100 mg pro Dosis ist.

## Revendications

1. Combinaison comprenant une quantité thérapeutiquement efficace d'un antagoniste époxy stéroïdien du récepteur aldostérone et une quantité thérapeutiquement efficace d'un inhibiteur de l'enzyme convertissant l'angiotensine, dans laquelle ledit inhibiteur de l'enzyme convertissant l'angiotensine est choisi dans le groupe comprenant l'alacépril, le bénazopril, le captopril, le cilazapril, le délapril, l'énalapril, l'énalaprilat, le fosinopril, le fosinoprilat, l'imidapril, le lisinopril, le périndopril, le quinapril, le ramipril, l'acétate de saralasin, le tempocapril, le trandolapril, le céranapril, le moexipril, le quinaprilat, le spirapril, Bioproject BP1.137, Chiesi CHF 1514, Fisons FPL-66564, l'idapril, Marion Merrell Dow MDL-100240, le périndoprilat, et Servier S-5590.

2. Combinaison selon la revendication 1, dans laquelle ledit antagoniste époxy stéroïdien du récepteur aldostérone est présent en une quantité qui est thérapeutiquement efficace pour être un antagoniste de l'aldostérone mais ladite quantité n'est pas suffisante pour que ledit antagoniste du récepteur aldostérone induise un effet secondaire considérablement indésirable.

3. Combinaison selon la revendication 1, dans laquelle ledit antagoniste époxy stéroïdien du récepteur aldostérone est choisi dans le groupe comprenant l'alacépril, le bénazopril, le captopril, le cilazapril, le délapril, l'énalapril, l'énalaprilat, le fosinopril, le fosinoprilat, l'imidapril, le lisinopril, le périndopril, le quinapril, le ramipril, l'acétate de saralasin, le tempocapril, le trandolapril, le céranapril, le moexipril, le quinaprilat et le spirapril.

4. Combinaison selon la revendication 1, dans laquelle ledit antagoniste du récepteur aldostérone est un composé stéroïdien du type époxy **caractérisé en ce qu'**il a un fragment 9α-,11α-époxy.

5. Combinaison selon la revendication 4, dans laquelle ledit composé époxy stéroïdien est l'épélérone.

6. Combinaison selon la revendication 1, **caractérisée en outre en ce que** ledit inhibiteur de l'enzyme convertissant l'angiotensine et ledit antagoniste du récepteur aldostérone sont présents dans ladite combinaison selon une plage de rapport en poids allant de 0,5/1 à 20/1 dudit inhibiteur de l'enzyme convertissant l'angiotensine par rapport audit antagoniste du récepteur aldostérone.

7. Combinaison selon la revendication 6, dans laquelle ladite plage de rapport en poids va de 1/1 à 15/1.

8. Combinaison selon la revendication 7, dans laquelle ladite plage de rapport en poids va de 1/1 à 5/1.

9. Utilisation d'une combinaison d'une quantité thérapeutiquement efficace comprenant essentiellement un antagoniste époxy stéroïdien du récepteur aldostérone, comme premier composant, et un inhibiteur de l'enzyme convertissant l'angiotensine comme deuxième composant, dans laquelle ledit inhibiteur de l'enzyme convertissant l'angiotensine est choisi dans le groupe comprenant l'alacépril, le bénazopril, le captopril, le cilazapril, le délapril, l'énalapril, l'énalaprilat, le fosinopril, le fosinoprilat, l'imidapril, le lisinopril, le périndopril, le quinapril, le ramipril, l'acétate de saralasin, le tempocapril, le trandolapril, le céranapril, le moexipril, le quinaprilat, le spirapril, Bioproject BP1.137, Chiesi CHF 1514, Fisons FPL-66564, l'idapril, Marion Merrell Dow MDL-100240, le périndoprilat, et Servier S-5590 pour la préparation d'un médicament destiné au traitement de troubles cardiovasculaires chez un patient (sujet) ayant ou susceptible d'avoir de multiples troubles cardiovasculaires.

10. Utilisation de la combinaison selon la revendication 9, dans laquelle ledit antagoniste époxy stéroïdien du récepteur aldostérone est présent en une quantité thérapeutiquement efficace pour être un antagoniste de l'aldostérone mais insuffisante pour induire un effet secondaire indésirable.

11. Utilisation de la combinaison selon la revendication 9, dans laquelle ledit inhibiteur de l'enzyme convertissant l'angiotensine est choisi dans le groupe comprenant l'alacépril, le bénazopril, le captopril, le cilazapril, le délapril, l'énalapril, l'énalaprilat, le fosinopril, le fosinoprilat, l'imidapril, le lisinopril, le périndopril, le quinapril, le ramipril, l'acétate de saralasin, le tempocapril, le trandolapril, le céranapril, le moexipril, le quinaprilat et le spirapril.

12. Utilisation de la combinaison selon la revendication 9, dans laquelle ledit inhibiteur de l'enzyme convertissant l'angiotensine et ledit antagoniste époxy stéroïdien du récepteur aldostérone sont administrés de manière séquentielle.

13. Utilisation de la combinaison selon la revendication 9, dans laquelle ledit inhibiteur de l'enzyme conversant l'angiotensine et ledit antagoniste époxy stéroïdien du récepteur aldostérone sont administrés de manière considérablement séquentielle.

14. Combinaison selon la revendication 9, dans laquelle ledit antagoniste époxy stéroïdien du récepteur aldostérone est un composé **caractérisé en ce qu'**il a un fragment 9α-,11α-époxy.

15. Combinaison selon la revendication 14, dans laquelle ledit composé époxy stéroïdien est l'épélérone.

16. Utilisation d'une combinaison de la revendication 9, **caractérisée en outre en ce que** ledit inhibiteur de l'enzyme convertissant l'angiotensine et ledit antagoniste époxy stéroïdien du récepteur aldostérone sont utilisés dans ladite co-thérapie dans une plage de rapport en poids allant de 0,5/1 à 20/1 dudit inhibiteur de l'enzyme convertissant l'angiotensine par rapport audit antagoniste du récepteur aldostérone.

17. Utilisation de la combinaison selon la revendication 16, dans laquelle ladite plage de rapport en poids va de 1/1 à 15/1.

18. Utilisation de la combinaison selon la revendication 17, dans laquelle ladite plage de rapport en poids va de 1/1 à 5/1.

19. Utilisation de la combinaison selon la revendication 9, dans laquelle ledit inhibiteur de l'enzyme convertissant l'angiotensine est le captopril dans une plage de dosage allant de 40 mg à 80 mg par dose, ou est l'énalapril dans une plage de dosage allant de 5 mg à 25 mg par dose.

20. Utilisation de la combinaison selon la revendication 9, dans laquelle ledit inhibiteur de l'enzyme convertissant l'angiotensine est le ramipril dans une plage de dosage allant de 2 mg à 20 mg par dose, ou est le lisinopril dans une plage de dosage allant de 5 mg à 25 mg par dose.

21. Utilisation de la combinaison selon la revendication 9, 19, ou 20, dans laquelle ledit antagoniste époxy stéroïdien du récepteur aldostérone est l'épélérone dans une plage de dosage allant de 25 mg à 100 mg par dose.
